(19) Europäisches Patentamt
European Patent Office
Office européen des brevets

(11) EP 1 911 443 A1

(12) **EUROPEAN PATENT APPLICATION**

(43) Date of publication:
**16.04.2008 Bulletin 2008/16**

(51) Int Cl.:
***A61K 9/127*** *(2006.01)*

(21) Application number: **06255277.3**

(22) Date of filing: **13.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **novosom AG**
**06120 Halle (DE)**

(72) Inventor: **Panzner, Steffen**
**06114 Halle (DE)**

(74) Representative: **Crump, Julian Richard John et al**
**Mintz Levin Cohn Ferris Glovsky & Popeo Intellectual Property LLP**
**The Rectory**
**9 Ironmonger Lane**
**London EC2V 8EY (GB)**

(54) **Amphoteric liposomes, method of formulating an amphoteric liposome and a method of loading an amphoteric liposome**

(57) An amphoteric liposome composed of a mixture of lipids, said mixture comprising a cationic amphiphile, an anionic amphiphile and optionally one or more neutral amphiphiles, at least one of said cationic and anionic amphiphiles being chargeable and the respective amounts of said cationic and anionic amphiphiles being selected such there is a stoichiometric excess of positively charged cationic amphiphile at a first lower pH, a stoichiometric excess of negatively charged anionic amphiphile at a second higher pH and said mixture has an isoelectric point intermediate said first and second pHs; characterised in that said positively charged cationic and negatively charged anionic amphiphiles are adapted to form a lipid salt with one another at said isoelectric point. Also disclosed are methods of predicting the fusogenicity of an amphoteric liposome at a given pH, formulating an amphoteric liposome and loading an amphoteric liposome with a cargo moiety.

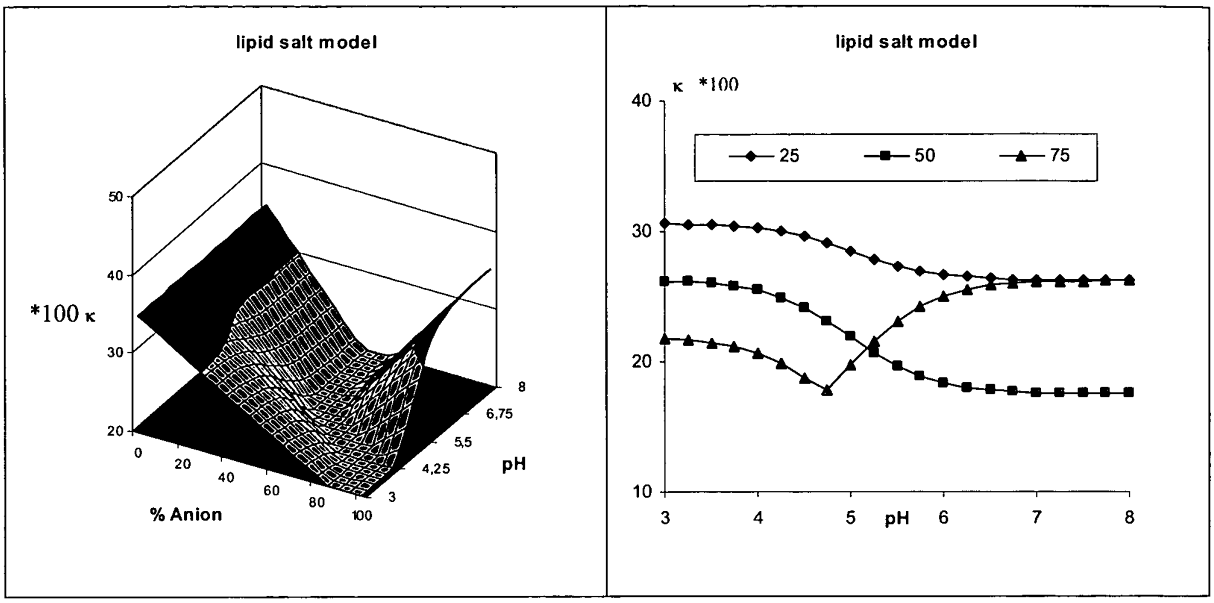


Fig. 1

EP 1 911 443 A1

## Description

Field of the invention

The present invention relates to improvements in or relating to amphoteric liposomes. In particular, the present invention provides a novel method for formulating such liposomes and a method for loading them, as well as liposomes produced by such methods.

Background to the invention

Amphoteric liposomes have been found to exhibit excellent biodistribution and to be well tolerated in animals. They can encapsulate active agents, including nucleic acid molecules, with high efficiency.

In contrast to zwitterionic structures, amphoteric liposomes advantageously have an isoelectric point and are negatively charged at higher pH values and positively charged at lower pH values. Amphoteric liposomes belong to the larger group of pH -sensitive liposomes that were introduced by Straubinger, et al. (FEBS Lett., 1985, 179(1), 148-154). Typical pH -responsive elements in pH -sensitive liposomes are cholesterol hemisuccinate (CHEMS), palmitoylhomocysteine, dioleoylglycerol hemisuccinate (DOG-Succ) and the like. CHEMS can stabilise dioleoylphosphatidylethanolamine (DOPE), a lipid which preferentially adopts the inverted hexagonal phase at temperatures above 10°C, into the lamellar phase at pH 7.4. Lamellar CHEMS/DOPE systems can be prepared at neutral or slightly alkaline pH but these systems become unstable and fuse at acidic pH (Hafez and Cullis, Biochim. Biophys. Acta, 2000, 1463, 107-114).

Hafez, et al. (Biophys. J. 2000, 79(3), 1438-1446) were unsatisfied with the limited control over the pH at which such fusion occurs and demonstrated a rational approach to fine-tune the fusion point by adding cationic lipids. Such mixtures have true amphoteric properties in that they exist in a cationic state at low pH and as anionic particles at higher pH, typically at physiological pH. According to Hafez, et al. fusion starts at pH values where the net charge of the particles is zero (their isoelectric point), and once such point is crossed (the pH is lower to any extent) fusion is a continuous process. This view is shared by Li and Schick (Biophys. J., 2001, 80, 1703-1711) who analysed the fusion tendency for amphoteric lipid mixtures using a mathematical model.

Israelachvili and Mitchell in 1975 (Biochim. Biophys. Acta, 1975, 389, 13-19) introduced the molecular shape concept which assumes that the overall form of lipid molecules determines the structure of the hydrated lipid membrane. This means that the lipid geometry and more specifically the size ratio between the polar head-group and the hydrophobic membrane anchor is the key parameter determining the lipid phase (Israelachvili, et al. Biochim Biophys Acta. 1977 17; 470(2):185-201). The original theory however did not consider counterions being a steric part of the polar head-group, but this was contributed by Li and Schick (Biophys. J., 2001, 80, 1703-1711). In their description of the DODAC/CHEMS system, the sodium ion enlarges the head-group of CHEMS at neutral pH, but dissociates as the pH drops, thus minimising the head-group volume and promoting a hexagonal phase; DODAC as a strong cation is assumed to be in constant association with its respective counterion, irrespective of the pH. The model predicts fusion at some pH and below.

Lipid phases according to the molecular shape concept (Israelachvili et al., 1980, Q. Rev. Biophys., 13(2), 121-200) :

| Shape | Organisation | Lipid phase | Examples |
|---|---|---|---|
| Inverted cone | Micelles | Isotropic Hexagonal I | Detergents Lysophopholipids |
| Cylinder | Bilayer | Lamellar (Cubic) | PC, PS, PI, SM |
| Cone | Reverse micelles | Hexagonal II | PE, PA at low pH or with Ca2+, Cholesterol, Cardiolipin |

The addition of neutral lipids to amphoteric lipid mixtures has been found to have little impact on the isoelectric point of amphoteric liposomes. WO 02/066012 (Panzner, et al.) discloses certain amphoteric liposomes comprising neutral lipids with a stable size at both low and neutral pHs. WO 02/066012 also describes a method of loading such particles with nucleic acids starting from a low pH.

Amphoteric liposomes are complex structures and comprise at least a complementary pair of charged lipids. The inclusion of one or more such neutral lipids significantly adds to the complexity of the mixture, especially since the individual amounts of the components may vary. Hafez, et al. (Biophys. J. 2000, 79(3), 1438-1446) and WO 02/066012 provide some guidance as to how to select lipid mixtures with truly amphoteric properties and more specifically how to determine their isoelectric point and onset of fusion. Nevertheless, the very high number of possible combinations of lipids represents a practical hurdle towards a more rapid optimisation of amphoteric liposomes, and there remains a need in the art for a method of predicting which mixtures of lipids form satisfactorily stable lamellar phases at high and

low pH, whilst forming a fusogenic, hexagonal phase at an intermediate pH.

Object of the invention

It is an object of the present invention therefore to provide an improved method for formulating such fusogenic amphoteric liposomes.

Summary of the invention

According to one aspect of the present invention therefore there is provided a method of formulating an amphoteric liposome comprising:

(i) selecting an anionic amphiphile, a cationic amphiphile, each of said anionic and cationic amphiphiles having respective polar head and apolar tail-groups, cationic and anionic counterions for said anionic and cationic amphiphiles respectively, and optionally one or more neutral amphiphiles, at least one of said anionic and cationic amphiphiles being chargeable;

(ii) calculating the $\kappa$ values for each of said anionic and cationic amphiphiles, when uncharged and when charged and associated respectively with said cationic and anionic counterions, and said one or more optional neutral amphiphiles and the $\kappa_{salt}$ value for a lipid salt comprising said anionic and cationic amphiphiles in charged form, $\kappa$ being the ratio of the molecular volume of the polar head-group $V_{head}$ to the molecular volume of the apolar tail-group $V_{apolar}$ of the respective species, the molecular volumes of the polar head-groups of the charged anionic and cationic amphiphiles including the respective counterions, $\kappa_{salt}$ being defined as:

$$\kappa_{salt} = \frac{V_{head}(cat) + V_{head}(an)}{V_{apolar}(cat) + V_{apolar}(an)}$$

wherein $V_{head}$*(cat)* is the molecular volume of the polar head-group of the cationic amphiphile without the respective counter-anion, $V_{head}$*(an)* is the molecular volume of the polar head-group of the anionic amphiphile without the respective countercation, $V_{apolar}$*(cat)* is the molecular volume of the apolar tail-group of the cationic amphiphile and $V_{apolar}$*(an)* is the molecular volume of the apolar tail-group of the anionic amphiphile;

(iii) modelling the function $\kappa_{total}$(pH) for a lipid mixture of said anionic and cationic amphiphiles and said one or more optional neutral amphiphiles, assuming said cationic and anionic amphiphiles form said lipid salt when charged, the respective amounts of said amphiphiles in said lipid mixture being chosen such that said mixture of lipids has an isoelectric point between a first lower pH and a second higher pH and has a stoichiometric excess of positively charged cationic amphiphile at said first pH and a stoichiometric excess of negatively charged anionic amphiphile at said second pH, $\kappa_{total}$(pH) being defined as:

$$\kappa_{total}(pH) = \kappa_{an} \cdot c_{an}(pH) + \kappa_{cat} \cdot c_{cat}(pH) + \kappa_{an-} \cdot c_{an-}(pH) + \kappa_{cat+} \cdot c_{cat+}(pH) + \kappa_{salt} \cdot c_{salt}(pH) + \sum \kappa_n \cdot c_n$$

wherein $c_{an}$*(pH)*, $c_{cat}$*(pH)*, $c_{an-}$*(pH)*, $c_{cat+}$*(pH)* and $c_{salt}$*(ph)* are the respective concentrations in the lipid mixture of the uncharged anionic, uncharged cationic, charged anionic and charged cationic amphiphiles and said lipid salt as a function of pH, $c_n$ is the concentration in the lipid mixture of the or each optional neutral amphiphile, and $\kappa_{an}$, $\kappa_{cat}$, $\kappa_{an-}$, $\kappa_{cat+}$, $\kappa_{salt}$ and $\kappa_n$ are the respective $\kappa$ values for the uncharged anionic, uncharged cationic, charged anionic and charged cationic amphiphiles, said lipid salt and the or each optional neutral amphiphile;

(iv) determining that $\kappa_{total}$(pH) exhibits a minimum at said isoelectric point;

(v) making liposomes composed of said lipid mixture and empirically confirming that said mixture exhibits stable

lamellar phases at said first and second pH 's and a fusogenic, hexagonal phase at said isoelectric point; and thereafter

(vi) manufacturing an amphoteric liposome composed of said lipid mixture.

Suitably. said molecular volumes may be calculated by molecular modelling.
Said mixture may have an isoelectric point in the range pH 4 to pH 8, preferably in the range pH 5 to pH 7.
Suitably, said first pH may be in the range pH 4 to pH 5. Said second pH may be in the range pH 7 to pH 8. Advantageously, said second pH is about physiological pH (about pH 7.4).
According to another aspect of the present invention there is provided an amphoteric liposome composed of a mixture of lipids, said mixture comprising a cationic amphiphile, an anionic amphiphile and optionally one or more neutral amphiphiles, at least one of said cationic and anionic amphiphiles being chargeable and the respective amounts of said cationic and anionic amphiphiles being selected such there is a stoichiometric excess of positively charged cationic amphiphile at a first lower pH, a stoichiometric excess of negatively charged anionic amphiphile at a second higher pH and said mixture has an isoelectric point intermediate said first and second pHs; characterised in that said positively charged cationic and negatively charged anionic amphiphiles are adapted to form a lipid salt with one another at said isoelectric point.
The fusion tendency of amphoteric liposomes has been analysed in more detail, and it has been demonstrated that stable liposomes can exist at low pHs, as well as under neutral conditions. The fusion of such liposomes is restricted to intermediate pHs. Such fusion at an intermediate pH in combination with stable lamellar phases at both low and high (neutral) pHs has been found in the presence, as well as in the absence, of neutral lipids.
Anionic and cationic amphiphiles have respective polar head and apolar tail-groups. In accordance with the invention, the polar head and apolar tail-groups of the anionic and cationic amphiphiles and said counterions may be selected such that $\kappa_{total}$(pH) for the mixture exhibits a minimum at said isoelectric point, whereby said mixture exhibits stable lamellar phases at said first and second pHs and a fusogenic, hexagonal phase at said isoelectric point.
Experimental evidence has now shown the existence of a stable lamellar phase at a pH below the fusion point.
It has been found therefore that the combination of the following assumptions allows a proper description of lipid phase behaviour:

1) shape theory as a basis for the description;

2) for polar head-groups in the charged state, counterions become part of the head-group volume; and

3) lipid-lipid salt formation occurs in the membrane

The method of the present invention therefore facilitates the identification of fusogenic amphoteric liposomes.
The amphoteric liposome of the present invention comprises a lipid pair that is capable of forming a lipid-lipid salt within a bilayer, rendering said liposome bistable.
In a particular aspect of the invention, the liposome may comprise a lipid mixture other than one having one of the following specific combinations of amphiphiles:

| Cationic amphiphile | Anionic amphiphile | Other | Ratio (mol.%) |
|---|---|---|---|
| DOTAP | Chems | | 30:40 |
| DOTAP | Chems | POPC | 10:40:50 |
| DOTAP | Chems | POPC | 25:25:50 |
| DOTAP | Chems | POPC | 20:30:50 |
| DOTAP | Chems | POPC | 20:20:60 |
| DOTAP | Chems | POPC | 10:30:60 |
| DOTAP | Chems | POPC | 15:25:60 |
| DOTAP | Chems | POPC: N-glutaryl-DPPE | 10:30:50:10 |
| DOTAP | Chems | DPPC:Chol | 10:30:50:10 |
| DOTAP | Chems | DPPC | 10:30:60 |

(continued)

| Cationic amphiphile | Anionic amphiphile | Other | Ratio (mol.%) |
|---|---|---|---|
| DOTAP | Chems | DPPC | 15:35:50 |
| DOTAP | Chems | POPC:Chol | 10:20:30:40 |
| DOTAP | Chems | DMPC:Chol | 10:30:20:40 |
| DOTAP | Chems | POPC | 15:45:40 |
| DOTAP | Chems | POPC | 20:60:20 |
| DOTAP | Chems | | 25:75 |
| DOTAP | Chems | POPC | 40:40:20 |
| DOTAP | Chems | POPC | 30:50:20 |
| DOTAP | Chems | POPC | 10:70:20 |
| DOTAP | Chems | POPC | 28:47:25 |
| DOTAP | Chems | DOPE | 40:40:20 |
| DOTAP | Chems | DOPE | 30:50:20 |
| DOTAP | Chems | DOPE | 20:60:20 |
| DOTAP | Chems | DOPE | 10:70:20 |

In another particular aspect of the invention, the liposome may comprise a lipid mixture other than one having one of the following specific combinations of amphiphiles

| Cationic amphiphile | Anionic amphiphile | Other | Ratio (mol.%) |
|---|---|---|---|
| CHIM | Chems | DPPC | 15:35:50 |
| CHIM | Chems | POPC | 15:35:50 |
| DC-Chol | DOPA | | 66:34 |
| DC-Chol | DOPA | Chol | 40:20:40 |
| DC-Chol | DOPA | DMPC | 27:13:60 |
| DC-Chol | DOPA | DMPC:Chol | 27:13:20:40 |
| DC-Chol | DOPA | DMPC:Chol | 20:10:30:40 |
| DC-Chol | DOPA | DMPC:Chol | 13:7:40:40 |
| HisChol | DG-Succ | DMPC:Chol | 10:10:40:40 |
| MoChol | DG-Succ | DMPC:Chol | 10:15:35:40 |
| MoChol | DG-Succ | DMPC:Chol | 10:10:40:40 |
| MoChol | DG-Succ | DMPC:Chol | 10:30:20:40 |
| MoChol | DG-Succ | DPPC:Chol | 10:30:20:40 |
| MoChol | DG-Succ | POPC:Chol | 10:15:35:40 |
| MoChol | DG-Succ | POPC:Chol | 10:30:20:40 |
| MoChol | DG-Succ | POPC:Chol | 20:10:30:40 |

In yet another particular aspect of the invention, the liposome may comprise a lipid mixture other than one having one of the following specific combinations of amphiphiles:

| Cationic amphiphile | Anionic amphiphile | Other | Ratio (mol.%) |
|---|---|---|---|
| CHIM | DMG-Succ | POPC:DOPE | 17:33:12.5:37.5 |
| CHIM | DMG-Succ | POPC:DOPE | 33:17:12.5:37.5 |
| CHIM | DMG-Succ | POPC:DOPE | 23:47:7.5:22.5 |
| CHIM | DMG-Succ | POPC:DOPE | 47:23:7.5:22.5 |
| CHIM | Chems | POPC:DOPE | 17:33:12.5:37.5 |
| CHIM | Chems | POPC:DOPE | 33:17:12.5:37.5 |
| CHIM | Chems | POPC:DOPE | 23:47:7.5:22.5 |
| CHIM | Chems | POPC:DOPE | 47:23:7.5:22.5 |
| MoChol | Cetyl-P | POPC:DOPE | 20:10:10:60 |
| MoChol | Cetyl-P | POPC:Chol | 20:10:35:35 |
| MoChol | DOG-Succ | POPC:DOPE | 17:33:12.5:37.5 |
| MoChol | DOG-Succ | POPC:DOPE | 33:17:12.5:37.5 |
| MoChol | DOG-Succ | POPC:DOPE | 23:47:7.5:22.5 |
| MoChol | DOG-Succ | POPC:DOPE | 47:23:7.5:22.5 |

According to yet another aspect of the present invention there is provided a method of loading an amphoteric liposome according to the present invention with a negatively charged cargo moiety, said method comprising generation of said liposome in the presence of said negatively charged cargo moiety at said first pH using a first solvent comprising anionic counterions, mixing said liposome with said negatively charged cargo moiety and thereafter exposing said liposome to said second pH using a second solvent comprising said cationic counterions.

Suitably, said solvent changes are performed by the one-step admixture of the respective second solvent, such that said liposome is rapidly brought to the desired pH.

Without being limited to such use, the amphoteric liposome according to the present invention is well suited for use as a carrier for nucleic acid-based drugs such, for example, as oligonucleotides and DNA plasmids. These drugs are classified into nucleic acids that encode one or more specific sequences for proteins, polypeptides or RNAs and into oligonucleotides that can specifically regulate protein expression levels or affect the protein structure through *inter alia* interference with splicing and artificial truncation.

In some embodiments of the present invention, therefore, the nucleic acid-based therapeutic may comprise a nucleic acid that is capable of being transcribed in a vertebrate cell into one or more RNAs, which RNAs may be mRNAs, shRNAs, miRNAs or ribozymes, wherein such mRNAs code for one or more proteins or polypeptides. Such nucleic acid therapeutics may be circular DNA plasmids, linear DNA constructs, like MIDGE vectors (Minimalistic Immunogenically Defined Gene Expression) as disclosed in WO 98/21322 or DE 19753182, or mRNAs ready for translation (e.g., EP 1392341).

In another embodiment of the invention, oligonucleotides may be used that can target existing intracellular nucleic acids or proteins. Said nucleic acids may code for a specific gene, such that said oligonucleotide is adapted to attenuate or modulate transcription, modify the processing of the transcript or otherwise interfere with the expression of the protein. The term "target nucleic acid" encompasses DNA encoding a specific gene, as well as all RNAs derived from such DNA, being pre-mRNA or mRNA. A specific hybridisation between the target nucleic acid and one or more oligonucleotides directed against such sequences may result in an inhibition or modulation of protein expression. To achieve such specific targeting, the oligonucleotide should suitably comprise a continuous stretch of nucleotides that is substantially complementary to the sequence of the target nucleic acid.

Oligonucleotides fulfilling the abovementioned criteria may be built with a number of different chemistries and topologies. Oligonucleotides may be single stranded or double stranded.

The mechanisms of action of oligonucleotides may vary and might comprise effects on inter alia splicing, transcription, nuclear-cytoplasmic transport and translation.

In a preferred embodiment of the invention single stranded oligonucleotides may be used, including, but not limited to, DNA-based oligonucleotides, locked nucleic acids, 2'-modified oligonucleotides and others, commonly known as antisense oligonucleotides. Backbone or base or sugar modifications may include, but are not limited to, Phosphothioate DNA (PTO), 2'O-methyl RNA (2'Ome), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), Morpholine phosphoamidate

(Morpholino), Cyclohexene nucleic acid (CeNA), tricyclo-DNA (tcDNA) and others. Moreover, mixed chemistries are known in the art, being constructed from more than a single nucleotide species as copolymers, block-copolymers or gapmers or in other arrangements. In addition to the aforementioned oligonucleotides, protein expression can also be inhibited using double stranded RNA molecules containing the complementary sequence motifs. Such RNA molecules are known as siRNA molecules in the art (e.g., WO 99/32619 or WO 02/055693). Again, various chemistries were adapted to this class of oligonucleotides. Also, DNA / RNA hybrid systems are known in the art.

In another embodiment of the present invention, decoy oligonucleotides can be used. These double stranded DNA molecules and chemical modifications thereof do not target nucleic acids but transcription factors. This means that decoy oligonucleotides bind sequence-specific DNA-binding proteins and interfere with the transcription (e.g., Cho-Chung, et al. in Curr. Opin. Mol. Ther., 1999).

In a further embodiment of the invention, oligonucleotides that may influence transcription by hybridizing under physiological conditions to the promoter region of a gene may be used. Again various chemistries may adapt to this class of oligonucleotides.

In a still further alternative of the invention, DNAzymes may be used. DNAzymes are single-stranded oligonucleotides and chemical modifications therof with enzymatic activity. Typical DNAzymes, known as the "10-23" model, are capable of cleaving single-stranded RNA at specific sites under physiological conditions. The 10-23 model of DNAzymes has a catalytic domain of 15 highly conserved deoxyribonucleotides, flanked by 2 substrate-recognition domains complementary to a target sequence on the RNA. Cleavage of the target mRNAs may result in their destruction and the DNAzymes recycle and cleave multiple substrates.

In yet another embodiment of the invention, ribozymes can be used. Ribozymes are single-stranded oligoribonucleotides and chemical modifications thereof with enzymatic activity. They can be operationally divided into two components, a conserved stem-loop structure forming the catalytic core and flanking sequences which are reverse complementary to sequences surrounding the target site in a given RNA transcript. Flanking sequences may confer specificity and may generally constitute 14-16 nt in total, extending on both sides of the target site selected.

In a still further embodiment of the invention, aptamers may be used to target proteins. Aptamers are macromolecules composed of nucleic acids, such as RNA or DNA, and chemical modifications thereof that bind tightly to a specific molecular target and are typically 15-60 nt long. The chain of nucleotides may form intramolecular interactions that fold the molecule into a complex three-dimensional shape. The shape of the aptamer allows it to bind tightly against the surface of its target molecule including but not limited to acidic proteins, basic proteins, membrane proteins, transcription factors and enzymes. Binding of aptamer molecules may influence the function of a target molecule.

All of the above-mentioned oligonucleotides may vary in length between as little as 10, preferably 15 and even more preferably 18, and 50, preferably 30 and more preferably 25, nucleotides. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one or more mismatches within the said continuous stretch of base pairs, although this is less preferred. In general, the type and chemical composition of such nucleic acids is of little impact for the performance of the inventive liposomes as vehicles be it *in vivo* or *in vitro*, and the skilled artisan may find other types of oligonucleotides or nucleic acids suitable for combination with the inventive liposomes.

## Brief description of the drawings

Figs. 1 and 2 are graphical representations of the calculation of κ for different ratios between anionic and cationic model lipids. Left panel: Surface plot for κ in response to pH and percentage of anionic lipid. Right panel: Detailed analysis of the pH response for selected amounts of anionic lipids.

Fig. 3 is a graphical representation of the calculation of κ for different ratios between anionic and cationic model lipids in amphoter III systems. Left panel: Surface plot for κ in response to pH and percentage of anionic lipid. Right panel: Detailed analysis of the pH response for selected amounts of anionic lipids.

Fig. 4: is a graphical representation of the calculation of κ for different ratios between anionic and cationic model lipids in amphoter III systems. Left panel: Salt bridge model; Right panel: independent ion model.

Fig. 5 shows the stabilisation of the anionic or cationic state of an amphoter II mixture through various counterion sizes. Left panel: Analysis for equal counterion sizes. Right panel: exclusive stabilisation of the anionic state through larger cationic counterions. CA - counter-anion; CC - counter-cation; the numbers in the legend indicate molecular volumes in $Å^3$

Fig. 6 illustrates the asymmetric stabilisation of a cationic amphoter II lipid phase through various counter-anions. During production, the cationic lipid phase is stabilised with larger anions (CA120). Liposomes are adjusted to a neutral pH and the buffer composition is changed for a smaller counter-anion (CA21). Liposomes that now encounter acidic pH are prone to fusion since the lipid phase has much lower values of κ. CA - counter-anion; CC - counter-cation; the numbers in the legend indicate molecular volumes in $Å^3$.

Fig. 7 illustrates the impact of absolute molecular volumes for given values of κ and counterion size. κ was adjusted to 0.175 for the cationic and anionic lipid in an amphoter II system, but the absolute lipid size was varied; the numbers in the legend indicate molecular head and tail volumes for both the cationic and anionic lipid in $Å^3$

Fig. 8 illustrates the impact of absolute molecular volumes for given values of κ and counterion size. κ was adjusted to 0.175 for the cationic and anionic lipid in an amphoter II system, but the anionic lipid had a smaller molecular volume than the cationic lipid.

Fig. 9 is a graphical representation of the calculation for κ in response to external pH in amphoter II systems further comprising neutral lipids. 50% of neutral lipids were added to the system with the κ values given in the figure legend.

Fig. 10 shows the size of DOTAP/CHEMS liposomes after pH - jump in CiP buffer. DOTAP liposomes containing 66 mol.% CHEMS (crosses), 75 mol.% CHEMS (asterisks) or 100 mol.% CHEMS (dots) were produced at pH 8, jumped to the indicated pH and neutralized after one hour incubation at the lower pH. Size was measured at the end of the cycle.

Fig. 11 shows the fusion behaviour of an amphoter II system comprising a MoCHol and CHEMS. Left - calculation of κ values for the system. Right - experimental fusion results after pH -jump of different mixtures of CHEMS and MoChol in CiP buffer. The percentage in the legend stands for the amount of CHEMS in the mixture.

Fig. 12 shows the fusion behaviour of an amphoter III system comprising a MoCHol and DOPA. Left - calculation of κ values for the system. Right - experimental fusion results after pH -jump of different mixtures of DOPA and MoChol in CiP buffer. The percentage in the legend stands for the amount of DOPA in the mixture.

Fig. 13 shows the fusion behaviour of an amphoter II system comprising a monoalkyl lipid. Left - calculation of κ values for the system. Right - experimental fusion results after pH -jump of different mixtures of oleic acid and MoChol in CiP buffer. The percentage in the legend stands for the amount of oleic acid in the mixture.

Fig. 14 illustrates fusion behaviour in the presence of POPC: Liposomes were produced at pH 7.5 and adjusted to acidic conditions to promote aggregation or fusion. Addition of 20 mol.% POPC greatly reduced the fusion tendency and liposomes were stable in size even at the lower pH. The composition in the legend represents the percentages for DOTAP and CHEMS; remainder is POPC. pI stands for the calculated isoelectric point of the mixture.

Fig. 15 illustrates fusion behaviour in the presence of DOPE: Liposomes were produced at pH 7.5 and adjusted to acidic conditions to promote aggregation or fusion. Addition of 20 mol.% DOPE maintains the fusion tendency of the amphoteric membrane. The composition in the legend represents the percentages for DOTAP and CHEMS; the remainder is DOPE. pI stands for the calculated isoelectric point of the mixture.

## <u>Detailed description of the invention</u>

By "chargeable" is meant that the amphiphile has a pK in the range pH 4 to pH 8. A chargeable amphiphile may therefore be a weak acid or base. A "stable" amphiphile is a strong acid or base, having a substantially stable charge on the range pH 4 to pH 8.

By "amphoteric" herein is meant a substance, a mixture of substances or a supra-molecular complex (e.g., a liposome) comprising charged groups of both anionic and cationic character wherein:

1) at least one, and optionally both, of the cation and anionic amphiphiles is chargeable, having at least one charged group with a pK between 4 and 8,

2) the cationic charge prevails at pH 4, and

3) the anionic charge prevails at pH 8.

As a result the substance or mixture of substances has an isoelectric point of neutral net charge between pH 4 and pH 8. Amphoteric character is by this definition different from zwitterionic character, as zwitterions do not have a pK in the range mentioned above. In consequence, zwitterions are essentially neutrally charged over a range of pH values; phosphatidylcholines and phosphatidylethanolamines are neutral lipids with zwitterionic character.

The following abbreviations for lipids are used herein, the majority of which abbreviations are in standard use in the literature:

| | |
|---|---|
| PC | Phosphatidylcholine (unspecified membrane anchor) |
| PE | Phosphatidylethanolamine (unspecified membrane anchor) |
| SM | Sphingomyelin |
| DMPC | Dimyristoylphosphatidylcholine |
| DPPC | Dipalmitoylphosphatidylcholine |
| DSPC | Distearoylphosphatidylcholine |

(continued)

| | |
|---|---|
| POPC | Palmitoyl-oleoylphosphatidylcholine |
| DOPC | Dioleoylphosphatidylcholine |
| DOPE | Dioleoylphosphatidylethanolamine |
| DMPE | Dimyristoylphosphatidylethanolamine |
| DPPE | Dipalmitoylphosphatidylethanolamine |
| CHEMS | Cholesterolhemisuccinate |
| DG-Succ | Diacylglycerolhemisuccinate (unspecified membrane anchor) |
| DOPS | Dioleoylphosphatidylserine |
| DPPS | Dipalmitoylphosphatidylserine |
| DOPG | Dioleoylphosphatidylglycerol |
| DPPG | Dipalmitoylphosphatidylglycerol |
| Chol-SO4 | Cholesterol sulphate |
| DOPA | Dioleoylphosphatidic acid |
| SDS | Sodium dodecyl sulphate |
| CHIM | Cholesterol-(3-imidazol-1-yl propyl)carbamate |
| DDAB | Dimethyldioctadecylammonium bromide |

DOTAP, DMTAP, DPTAP, DSTAP:

1,2-Diacyl-3-Trimethylammonium-Propane

DODAP, DMDAP, DPDAP, DSDAP:

1,2-Diacyl-3-Dimethylammonium-Propane

DOEPC, DMEPC, DPEPC, DSEPC:

1,2-Diacyl-sn-Glycero-3-Ethylphosphocholine

| | |
|---|---|
| DOTMA | N-[1-(2,3-dioleyloxy)propyl]-N,N,N-trimethyl ammonium chloride |
| DOTIM | 1-[2-(oleoyloxy)ethyl]-2-oleyl-3-(2-hydroxyethyl) imidazolinium chloride |
| TMAG | N-(a-trimethylammonioacetyl)-didodecyl-D-glutamate chloride |
| BCAT | O-(2R-1,2-di-O-(19Z,99Z-octadecadienyl)-glycerol)-N-(bis-2-aminoethyl) carbamate |
| DODAC | Dioleyldimethylammonium chloride |
| DORIE | 1,2-dioleoyl-3-dimethyl-hydroxyethyl ammonium bromide |
| DMRIE | 1,2-dimyristoyl-3-dimethyl-hydroxyethyl ammonium bromide |
| DOSC | 1,2-dioleoyl-3-succinyl-sn-glycerol choline ester |
| DORI | 1,2-dioleoyloxypropyl-3-dimethylhydroxyethylammonium chloride |
| DHMHAC | N,N-di-n-hexadecyl-N, Ndihydroxyethylammoniumbromide |
| DHDEAB | N,N-di-n-hexadecyl-N-methyl,N-(2-hydroxyethyl) ammonium chloride |
| DMHMAC | N,N-myristyl-N-(1-hydroxyprop-2-yl)-N-methylammoniumchloride |

(continued)

| | |
|---|---|
| DOTB | 1,2-dioleoyl-3-(4'-trimethylammonio)butanoyl-sn-glycerol |
| SAINT lipids | Synthetic Amphiphiles INTerdisciplinary |
| DPIM, DOIM | 4,(2,3-bis-acyloxy-propyl)-1-methyl-1H-imidazole (unspecified membrane anchor) |
| DPAPy | 2,3-bis-palmitoyl-propyl-pyridin-4-yl-amine |
| DC-Chol | 3b-[N-(N9,N9-dimethylaminoethane)carbamoyl] cholesterol |
| TC-Chol | 3b-[N-(N9,N9-trimethylaminoethane)carbamoyl] cholesterol |
| DAC-Chol | 3b(N-(N,N'-Dimethylaminoethan)-carbamoyl)cholesterol |
| CTAB | Cetyltrimethylammonium bromide |
| NeoPhectin™ | cationic cardiolipins (e.g. [1, 3-Bis-(1, 2-bis-tetradecyloxy-propyl-3-dimethylethoxyammoniumbromide)-propane-2-ol] |
| HistChol | Nα-Histidinyl-Cholesterol-hemisuccinate |

MoChol 4-(2-Aminoethyl)-Morpholino-Cholesterolhemisuccinate:

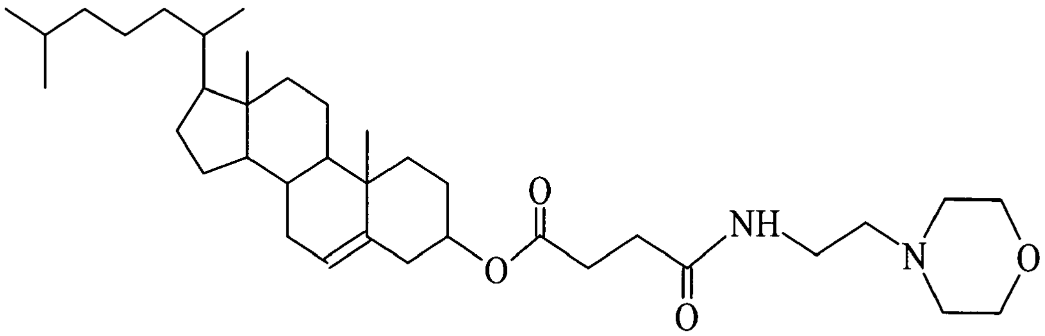

HisChol Histaminyl-Cholesterolhemisuccinate:

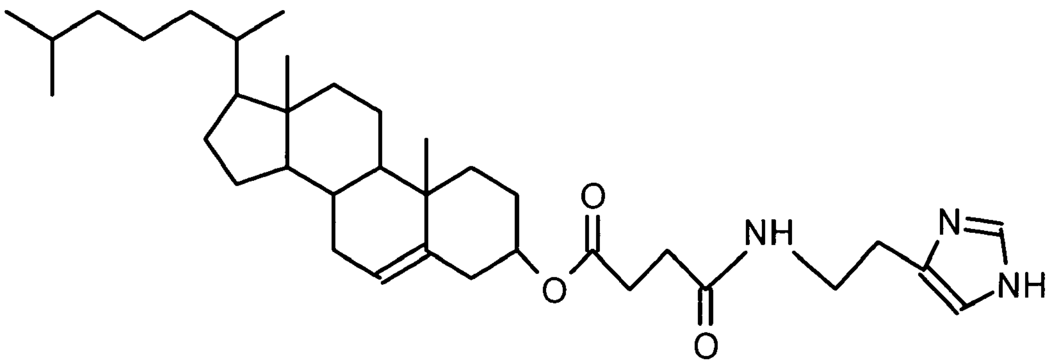

*Molecular volumes*

Lipid shape theory is built on a shape balance between the hydrophobic part and the polar head-group of a given amphiphile rather than on absolute values for the two molecular portions. In accordance with the present invention, κ is the volume ratio between the polar and apolar section of a lipid.

$$\kappa = \text{molecular volume (head)} / \text{molecular volume (tail)}$$

Various different ways are available to those skilled in the art to calculate molecular volumes and alternative methods and sources are given at:

http://www.ccl.net/cca/documents/molecular-modeling/node5.html

Molecular volume is commonly calculated by assigning a value called a van der Waals radius, $r^i_{vdW}$, to each atom type in such a way that the sum of these quantities for a given atom pair, i and j, is equal to their closest possible distance:

$$r^i_{vdW} + r^j_{vdW} \leq dij$$

Many different tables of "best" van der Waals radii exist, even though the values for corresponding atoms coming from different authors are similar. In geometric terms, the van der Waals radius may be imagined as a spherical "shield" surrounding the atom, and the closest distance between two non-bonded atoms is when their respective shields touch. However, the shields of covalently bonded atoms intersect since bond lengths are much shorter than the sum of the van der Waals radii partaking atoms. A molecular van der Waals surface, also called a van der Waals envelope, is composed of the spheres for individual atoms with their intersecting sections removed.

For a single molecule (i.e., molecule for which there is a path between any two atoms along covalent bonds), the van der Waals envelope is a closed surface, and hence, it contains volume. This volume is called the molecular volume, or van der Waals volume and is usually given in Å$^3$. The straightforward way of calculating molecular volume on a computer is by numerical integration.

In some embodiments, molecular volumes for lipid molecules and the respective head and tail fragments may be calculated using DS Viewer Pro 5.0 (Accelrys) and volumes within the respective van der Waals radii were calculated.

Typical membrane fragments are 1,2-diacyl-ethyleneglycols that represent the hydrophobic section for common phospholipids, leaving the 3' carbon atom of the original glycerol with the phosphocholine head-group. The same fragment is also found in the common cationic lipid DOTAP and its derivatives but also in diacylglycerols with other polar head-groups such as dimyristoylglycerol hemisuccinate and the like.

For the cholesterol derivatives, the entire sterol but not the 3' oxygene was defined as the hydrophobic section and the head-group being complementary to that.

Likewise, for cationic or anionic alkyl derivatives the polar head-group was defined as the polar fragment involving the C1 carbon of the alkyl chain. Consequently, the residual chain with n-1 carbon atoms represents the hydrophobic part.

Molecular volumes of course depend on the constants used for the calculations and are to some extent affected by the conformation of the molecule. Typical values obtained for the hydrophobic fragments are:

Table:1

| **Membrane fragment** | **Volume in Å$^3$** | |
|---|---|---|
| di-lauroylethyleneglycol | 356 | |
| di-myristoylethyleneglycol | 407 | |
| di-palmitoylethyleneglycol | 458 | |
| di-stearoylethyleneglycol | 509 | |
| di-oleoylethyleneglycol | 501 | |
| Palmitoyl-oleoylethyleneglycol | 478 | |
| di-phytanoylethylenglycol | 566 | |
| di-oleylethyleneglycol (e.g., in DOTMA) | 495 | |
| di-palmitylethylenglycol | 452 | |
| Didoceyl-D-glutamate (e.g., in TMAG) | 395 | |
| Cholesteryl | 334 | |
| C11 hydrophobic part in lauryl derivatives | 132 | |

(continued)

| Membrane fragment | Volume in Å$^3$ | |
|---|---|---|
| C13 hydrophobic part in myristyl derivatives | 158 | |
| C15 hydrophobic part in palmityl derivatives | 184 | |
| C17 hydrophobic part in stearyl derivatives | 210 | |
| C17 hydrophobic part in oleyl derivatives | 208 | |
| Sphingomyelin / Ceramide backbone | 467 | |

Molecular volumes for most counter-anions were derived the same way, but for Na+ or K+ the strongly bound hydration sphere is taken into account. The following values were used for further calculations:

Table 2:

| Counterion | Volume in Å$^3$ | |
|---|---|---|
| Acetate$^-$ | 40 | |
| Citrate$^-$ | 121 | |
| Phosphate$^{2-}$ | 49 | |
| Chloride$^-$ | 21 | |
| Formiate$^-$ | 29 | |
| $PF_6^-$ | 51 | |
| Methylsulfate$^-$ | 64 | |
| Trifluoroacetate$^-$ | 56 | |
| Barbituric acid | 79 | |
| Pyrophosphate$^{4-}$ | 88 | |
| Sodium$^+$ | 65 | |
| Potassium$^+$ | 111 | |
| Imidazolium$^+$ | 52 | |
| Morpholinium$^+$ | 69 | |
| Tris(hydroxymethyl)-aminoethan$^+$ | 91 | |
| Tris(hydroxyethyl)-aminoethan$^+$ | 130 | |
| Bis(hydroxymethyl)-aminoethan$^+$ | 74 | |
| Hydroxymethyl-aminoethan$^+$ | 50 | |
| Bis(hydroxymethyl)hydroxyethylaminoethan$^+$ | 107 | |
| Bis(hydroxyethyl)hydroxymethylaminoethan$^+$ | 123 | |
| Triethylamine$^+$ | 92 | |
| Diethyl-hydroxyethyl-amine$^+$ | 100 | |
| Glucoronic acid$^-$ | 129 | |
| Malonic acid$^-$ | 66 | |
| Tartaric acid$^-$ | 97 | |
| Glucosamine$^+$ | 129 | |

The charged polar head-groups have a great many of different representations and the molecular volumes are given below for some individual members of this group.

Table 3:

| Polar head-group (anions) | Volume in Å$^3$ | pK (calculated/ measured) |
|---|---|---|
| Hemisuccinate (e.g., in CHEMS) | 76 | |
| Hemisuccinate (e.g., in diacylglycerols) | 87 | |
| 2,3 dimethylhemisuccinate (e.g., esterified to diacylglycerols) | 117 | |
| Hemimalonate (e.g., esterified to cholesterol) | 78 | |
| Hemiadipate (e.g., esterified to diacylglycerols) | 115 | |
| Sulfate (e.g., in cholesteryl sulfate) | 50 | |
| Methylsulfate (e.g., SDS) | 64 | |
| Carboxyl (e.g., in alkyl carboxylic acids) | 42 | |
| Methylphosphate (e.g., in Cetylphosphate, phosphatidic acid) | 63 | |
| Phosphoglycerol | 115 | |
| Phosphoserin | 118 | |

| Polar head-groups (cations) | Volume in Å$^3$ | pK (calculated / measured) |
|---|---|---|
| Trimethylammoniummethyl (e.g., in cetyltrimethylammonium, DOTAP and others) | 67 | |
| Dimethylammonium-dimethyl (e.g., in DODAC) | 66 | |
| Trimethyl-hydroxyethyl ammonium (e.g., in DORIE) | 88 | |
| Dimethyl-di-hydroxyethyl ammonium (e.g., in DHDEAB) | 108 | |
| N-(1-hydroxyprop-2-yl)-N- trimethyl ammonium (e.g., in DMHMAC) | 102 | |
| 4-trimethylammonio-butenoic acid methylester (e.g., in DOTB) | 128 | |
| 1-Methyl-4-choline-succinic acid diester (e.g., in DOSC) | 157 | |
| Methylimidazol (e.g., in DOIM or DPIM) | 74 | |
| 3-imidazol-1-yl-propyl carbamate (e.g., , in CHIM) | 121 | |
| 2-(4-Imidazolyl)ethylamine hemisuccinate (e.g., in HisChol) | 148 | |
| N-Morpholino ethylamine hemisuccinate (e.g., in MoChol) | 166 | |
| N-Methylmorpholin | 81 | |
| Methylamine (e.g., in Stearylamine) | 30 | |
| Ethylphosphocholine (e.g., in DOEPC) | 161 | |
| 1-[2-Carboxyethyl]2-methyl-3-(2-hydroxyethyl)imidazolinium (e.g., in DOTIM) | 157 | |
| N-(a-trimethylammonioacetyl) (e.g., in TMAG) | 94 | |
| Pyridin-4-methylamine (e.g., in DPAPy) | 83 | |
| N-Methyl-pyridin (e.g., in SAINT-2) | 87 | |
| N-Methyl-4-carboxy-pyridine (e.g., in SAINT esters) | 110 | |
| N-Methyl-3,5-dicarboxy-pyridine (e.g., in SAINT diesters) | 145 | |
| Piperazine 4-N-aminoethyl carbamoyl | 130 | |
| (dimethyl)-aminoethyl carbamoyl (e.g., in DC-Chol) | 99 | |

(continued)

| Polar head-groups (cations) | Volume in Å$^3$ | pK (calculated / measured) |
|---|---|---|
| (trimethyl)-aminoethyl carbamoyl (e.g., in TC-Chol) | 113 | |
| N-Methyl-tris(hydroxymethyl)aminomethane | 104 | |
| N-Methyl-bis(hydroxymethyl)aminomethane | 83.5 | |
| N-Methyl-mono(hydroxymethyl)aminomethane | 61.8 | |

| Polar head-groups (neutral or zwitterionic) | Volume in Å$^3$ | |
|---|---|---|
| Phosphocholine | 133 | |
| Phosphoethanolamine | 97 | |

It is of course possible to use other methods to determine molecular volumes for the lipids. Also, some parameters such as the exact split-point between membrane tail and polar head; number of water molecules in the hydration cage or the van der Waals radii can be varied to some extent without affecting the general applicability of the model. With the same understanding more subtle changes in the molecular volumes were left unattended, in particular those arising from the dissociation of protons or from conformational changes.

The counterions fall into the same category of sizes than the actual polar head-groups. As such, it has been found that the addition or withdrawal of counterions from lipid polar regions has a substantial effect on the total head-group size and in consequence on the head/tail balance κ. As an example, the CHEMS sodium salt has a head-group size of 141 A$^3$ which is reduced to 76A$^3$ in the undissociated form at pH 4. κ varies between 0.42 and 0.23, respectively. CHEMS does form a lamellar phase at pH 7.5 and higher but adopts a hexagonal phase at low pH.

Other lipids with known phase behaviour can be used to select κ values for discrimination between the lamellar and hexagonal phase, an example is given in Table 4 below. PE head-groups can form an intramolecular ring structure with hydrogen bonding between the terminal amino group and the oxygen in the phosphoester group (betaine structure). PC head-groups are sterically hindered to do so and are recruit counterions to their respective charged groups.

Table 4:

| Lipid or mixture | κ. | Phase behaviour |
|---|---|---|
| POPC | 0.46 | Lamellar |
| DOPE | 0.19 | Hexagonal |

*pH induced changes of molecular volumes in amphoteric lipid mixtures*

In a first model, no lipid salt formation occurs between charged anionic and cationic lipids. This reflects the assumptions of Li and Schick (Biophys. J., 2001, 80, 1703-1711) and might be the case for lipids that are sterically hindered to form lipid salts (independent ion model).

The lipid species in the membrane comprise undissociated anions and cations as well as the dissociated anions and cations, the latter being complexed with their respective counterions. The κ value for such a mixture is assumed to be the weighted sum of its components:

$$\textbf{(1)}\quad \kappa = \kappa\,(anion^0)*c(anion^0) + \kappa\,(cation^0)*c(cation^0) + \kappa\,(anion^-)*c(anion^-) + \kappa(cation^+)*c(cation^+);$$

wherein anion° or cation° denotes the uncharged species and anion$^-$ or cation$^+$ denotes the respective charged species; and wherein c herein denotes concentration.

The amounts of the individual species present under such assumption can be calculated from known equilibrium constants K for the acid or base dissociation (the negative logarithm is more frequently used and known as pK):

$$(2)\quad c(anion^{-}) = c(anion^{tot}) / (c_{H+}/K + 1)$$

$$(3)\quad c(anion^{0}) = c(anion^{tot}) - c(anion^{-})$$

$$(4)\quad c(cation^{+}) = c(cation^{tot}) / (K/c_{H+} + 1)$$

$$(5)\quad c(cation^{0}) = c(cation^{tot}) - c(cation^{+}) \quad ;$$

wherein $anion^0$ is the undissociated anion, $anion^-$ the negatively charged molecule and $anion^{tot}$ the total concentration of the respective anion. Cations follow the same nomenclature and $c_{H+}$ and K describe the proton concentration and the equilibrium constant for the acid or base, respectively.

Taking possible interaction between a cationic and anionic amphiphile into account in accordance with the invention, the lipid salt occurs as a fifth species in the mixture:

$$(6)\quad \kappa = \kappa\ (anion^{0}) * c(anion^{0}) + \kappa\ (cation^{0}) * c(cation^{0}) + \kappa\ (anion^{-}) * c(anion^{-}) + \kappa\ (cation^{+}) * c(cation^{+}) + \kappa\ (salt) * c(salt)$$

In a lipid salt, the cationic amphiphile serves as a counterion to the anionic amphiphile and vice versa thus displacing the small counterions like sodium or phosphate from the head-group. The lipid salt is net uncharged and its geometry has to be assumed to be the sum of both parts without the small counterions. Therefore:

$$(7)\quad \kappa\ (salt) = (v_{head}(cation) + v_{head}(anion))/(v_{apolar}(cation) + v_{apolar}(anion))$$

Salt formation is limited by the charged amphiphile that is present in the lowest concentration:

$$(8)\quad c(salt) = MIN(c(cation^{+}); c(anion^{-}))$$

Salt formation between the two charged amphiphiles is assumed to be complete within that model, but of course, an incomplete salt formation may be assumed.

Model Calculations

To achieve amphoteric character of a lipid mix, at least one of the lipid ions needs to be a pH -sensitive, weak acid or base ("chargeable"). A detailed disclosure is found in WO 02/066012. Being different in character, three basic systems are possible and are analysed here:

| | |
|---|---|
| "Amphoter I" | strong cation and weak anion, |
| "Amphoter II" | weak cation and weak anion, |
| "Amphoter III" | weak cation and strong anion. |

Amphoter I systems

Amphoter I systems need an excess of the pH -sensitive anion to achieve amphoteric character. At pH 7 to 8 the anionic lipid is fully charged and salt formation occurs till all cationic lipids are consumed. In an example with 70 mol.% anionic lipid and 30 mol.% cationic lipid, all cationic lipid and a corresponding 30 mol.% of the anionic lipid would exist as lipid salt while 40 mol.% of the anionic lipid is unbound and recruits its counterion to the head-group.

Starting from neutral conditions, a reduction of the pH discharges the anionic lipid, the $\kappa$ value becomes smaller owing to loss of the counterion and reaches a minimum when the portion of still charged anionic lipid is equal to the amount of cationic lipid. Therefore, $\kappa$ is minimal at the isoelectric point of the amphoteric lipid mixture. If the pH is further lowered, an increasingly smaller portion of the anionic lipid remains charged. This means dissociation of the lipid salt and recruitment of counterions, now to the cationic lipid liberated from the lipid salt.

The left panel in Fig. 1 of the accompanying drawings illustrates the complex behaviour of $\kappa$ in dependence from pH and the amount of anionic lipid in the mixture. A valley of fusogenicity appears and any amphoteric mixture having more than 55 mol.% and less than 85 mol.% anionic lipid is expected to fuse under slightly acidic conditions but to be stable both at neutrality and under acidic conditions.

Amphoter I mixtures with less than 50 mol.% anionic lipid are no longer amphoteric since the anion can modulate, but not overcompensate, the charge on the cationic lipid. These mixtures might undergo a pH -dependent fusion, but do not provide a second stable phase at low pH. A 1:1 complex adopts a lamellar phase only at low pH and undergoes fusion at neutrality.

The parameters used for the calculations illustrated in Fig. 1 are given below; volumes in $Å^3$.

| | |
|---|---|
| Anion head volume | 70 |
| Anion tail volume | 400 |
| Anion pK | 5 |
| Cation head volume | 70 |
| Cation tail volume | 400 |
| Cation pK | 15 |
| Counterion+ | 70 |
| Counterion- | 70 |

Amphoter II systems

Amphoter II systems have the distinct advantage to be amphoteric over the entire range of anion: cation ratios and no charge overcompensation for the strong ion is needed as in Amphoter I or Amphoter III systems. A calculation for a model system is shown in Fig. 2.

The parameters used for the calculation are given below; all volumes in $Å^3$.

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ volume | 70 |

(continued)

| | |
|---|---|
| Counterion- volume | 70 |

Again, the lipid salt model predicts stable states at neutral to slightly alkaline pH but also at slightly acidic pH and a pronounced valley of instability or fusogenicity in between.

In contrast to amphoter I systems, fusogenic states can be reached across a wide range of different lipid ratios between the anionic and cationic components.

Amphoter III mixtures

Amphoter III mixtures comprising a stable anion and a pH - sensitive cation cannot form lipid salts at neutral pH, since little to no charged cationic lipid exists at this pH. It needs ongoing acidification to first create the cation which then may undergo salt formation. Calculation for a model system is shown in Fig. 3.

The parameters used for the calculation are given below; all volumes in $Å^3$.

| | |
|---|---|
| Anion head volume | 70 |
| Anion tail volume | 400 |
| Anion pK | 1 |
| Cation head volume | 70 |
| Cation tail volume | 400 |
| Cation pK | 6.5 |
| Counterion+ volume | 70 |
| Counterion- volume | 70 |

As can be seen from Figs. 1 and 3, amphoter III systems behave like the mirror image of amphoter I systems. They provide a valley of fusogenicity as long as the weak lipid ion is present in excess and over-compensates the constant charge on the opposite ion. In contrast to amphoter I systems the pH for fusion locates higher than the pK of the pH -sensitive lipid ion.

Experimental evidence for the fusion valley is given in the Examples 1 to 4 and provides confirmation for the central hypothesis of lipid salt formation in amphoteric liposomes. The examples also provide two closely related amphoter III systems (MoChol/POPG and MoChol/DOPA) where fusion is only observed in one of the two, namely MoChol/DOPA. This may be due to steric hindrance, as the protonated tertiary nitrogen in Mo-Chol is situated at the lower end of the morpholino ring and is therefore not easily accessible. In addition, the phosphate in POPG sits right at the lipid/water interface and is protected with a glycerol towards the water phase. In contrast, the phosphate group in DOPA is easily accessible. The model described above can be used to describe both situations, if lipid salt formation is optionally removed from the model building assumptions (equations (1) to (5) only). A comparison between the two scenarios is shown in Fig. 4. The parameters used for the calculation of Fig. 4 are given below, DOPA and MoChol in Na/ $H_2PO_4$ were used as model compounds; and volumes are expressed as $Å^3$.

| | |
|---|---|
| Anion head volume | 63 |
| Anion tail volume | 501 |
| Anion pK | 3 |
| Cation head volume | 166 |
| Cation tail volume | 371 |
| Cation pK | 6.5 |
| Counterion+ volume | 65 |
| Counterion- volume | 49 |

Whereas the lipid salt model predicts fusion with lower pH, a deletion of salt formation leads to a system where an

increase of κ is observed when the cationic lipid becomes protonated. If at all, such situation leads to an even more lamellar and eventually to a micellar state of the membrane. However, the limit for micelle formation has not been determined within the context of this model.

The monoanionic state of DOPA (pK 1=3, pK 2=8) was used for the model since DOPA exists as a mono-anion when MoChol becomes ionised (pK=6.5). The model predicts little or no fusogenicity at 33 mol.% anion, valley type fusion behaviour at 50 mol.% anion and monophasic fusion behaviour at 66 mol.% and more of the anion. The entire complexity of the model is reflected in the practical behaviour of the membrane as illustrated in Example 4.

The algorithm according to the present invention allows prediction of fusion behaviour of a wide range of amphoteric lipid mixtures. The prediction rules are derived from a simple geometrical description of the interacting lipids and are independent from the actual chemical representation of the molecules. As such, existing and novel lipid combinations can be easily tested by those skilled in the art, and the intended fusion behaviour can be predicted in a rational way. The following key parameters may illustrate such selection process, but other priorities might be set dependent on the respective goals of the application.

**1. κ of the lipid salt**

κ of the lipid salt is calculated in **(7)** and may suitably be lower than 0.35 to predict reasonably a fusogenic hexagonal phase. In preferred embodiments of the invention, κ may be lower than 0.3; more preferably lower than 0.25. κ (lipid salt) is low when the combined head-groups are small and the combined hydrophobic portions are large. The preferred sum of head-group volumes is about 300 $Å^3$ or smaller; in a more preferred embodiment this volume is smaller than 220 $Å^3$, and an even more preferred value is smaller than 170 $Å^3$.

**2. Amplitude of change (d(κ)/d(pH))**

In a preferred embodiment of the present invention, a lipid salt with a low value for κ is stabilised below or above its isoelectric point by recruitment of counterions. In a preferred embodiment of the invention larger counterions are used to stabilise either the cationic or the anionic state of the amphoteric lipid mixture. Fig. 5 illustrates such dependence from counterions size for an amphoter II system. The parameters used for the calculation of Fig. 5 are given below.

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ | See Fig. 5 |
| Counterion- | See Fig. 5 |

It becomes apparent from the right panel of Fig. 5 that such stabilisation may be asymmetric, e.g., providing rather limited stabilisation for the cationic phase and more stabilisation of the anionic phase of the amphoteric lipid mix; this may be advantageous for discharging the cargo from the liposomes *in vivo.* The selection of proper ion volumes for the individual or common stabilisation of a lipid phase is therefore a preferred application of the present invention. Such stabilisation is of particular use for the manufacturing and storage of amphoteric liposomes.

In some embodiments of the present invention larger counter-cations are used to stabilise the amphoteric liposomes at neutral conditions. In a preferred embodiment such counter-cations have a molecular volume of 50 $Å^3$ or more, in a more preferred embodiment this volume exceeds 75 $Å^3$ and said neutral pH is between pH 7 and pH 8, more preferred about the physiological pH of 7.4.

If amphoteric liposomes are produced for pharmaceutical purposes, compatibility of the used ions with the application route needs to be obeyed. It is known that, e.g., injection of larger amounts of potassium ions in the systemic circulation can be detrimental, especially in the absence of sodium ions.

Suitable counter-cations can be selected from Table 2 above describing the ion sizes. Preferred counter-cations for pharmaceutical compositions are sodium or tris(hydroxymethyl)aminomethan, tris-hydroxyethylaminomethan, triethylamine and the like.

In an embodiment of the invention the amphoteric liposomes may be manufactured at a low pH in their cationic state. Under these conditions, the liposomes can bind polyanions such as proteins, peptides or nucleic acids, whether its large plasmids or smaller oligonucleotides. Such binding is useful for improvement of the encapsulation efficacy of said materials into the amphoteric liposomes.

It is of specific advantage to use a lipid phase with a low κ at acidic pH. Selection of large counter-anions facilitates stabilisation of said lipid phase, e.g., for the production of such liposomes and the encapsulation of cargo under these conditions.

Suitable large counter-anions have a molecular volume larger than 50 $A^3$, preferred large counterions have a molecular volume larger than 75 $A^3$. Suitable counter-anions can be selected from Table 2 above. Preferred counter-anions are citrate, pyrophosphate, barbiturate, methyl sulphate and the like.

After having contacted the lipid phase with the cargo to be encapsulated under acidic conditions, the liposomes are than neutralized and non-encapsulated cargo can optionally be removed. Typically, non-encapsulated cargo detaches from the lipid membrane since both carry the same charge under neutral conditions. The amphoteric liposomes are negatively charged above their isoelectric point, e.g., at a pH between 7 and 8 and the cargo molecules exist as polyanions at such a pH. This is in particular the case with nucleic acids that carry one negative charge per nucleobase. Such liposomes can undergo effective destabilisation when exposed to the low pH in combination with a smaller counter-anion. This is for example the case after systemic administration and cellular uptake and endocytosis of such liposomes. Chloride or phosphate are the most common counter-anions in the body fluids of animals, be it any animal, a mammal or humans. Phosphate, but even more so chloride, are small counterions with little or no hydration shell and molecular volumes < 60 $A^3$.

Fig. 6 illustrates a cycle of liposome generation and use which illustrates selective stabilisation and destabilisation of the lipid phase under acidic conditions through asymmetric counterion use. The parameters used for the calculation of Fig. 6 are given below; volumes in $Å^3$.

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ | See Fig. 6 |
| Counterion- | See Fig. 6 |

### 3. Isoelectric point

A mathematical description for the isoelectric point of amphoteric liposomes has been given in the WO 02/066012. In accordance with the present invention, the isoelectric point of the amphoteric liposomes can be adjusted to a wide range of conditions, and there is sufficient chemical representation for individual lipids with different pK dissociation constants that allows the skilled artisan to select useful components and combinations for the making of amphoteric liposomes.

### 4. Relationship between κ (lipid salt) and counterions

Preferred values for κ and counterion size have been given above. In addition to these criteria, the absolute molecular volume of a lipid molecule is of importance. For large absolute volumes the relative impact of the counterion binding becomes smaller. This is illustrated in Fig. 7 for lipids with identical κ, but different absolute volumes. Other parameters used for the calculation of Fig. 7 are given below; volumes in $Å^3$.

| | |
|---|---|
| Anion head volume | see legend in Fig. 7 |
| Anion tail volume | see legend in Fig. 7 |
| Anion pK | 5 |
| Cation head volume | see legend in Fig. 7 |

(continued)

| | |
|---|---|
| Cation tail volume | see legend in Fig. 7 |
| Cation pK | 6.5 |
| Counterion+ volume | 70 |
| Counterion- volume | 70 |

In a preferred embodiment of the present invention the absolute molecular volumes for the lipids are small, e.g., <1000 $Å^3$ for the combined volumes of an anionic and cationic lipid. More preferred are lipid pairs with about 700 $Å^3$ molecular volume.

As was the case for counterions of different volumes, asymmetric stability of a cationic and anionic lipid phase can also be achieved by selection of lipids with different absolute molecular volumes, even if said lipids have equal κ values. The example in Fig. 8 illustrates such a design variant, where a smaller anionic lipid leads to selective destabilisation under acidic conditions. Parameters used for the calculation of Fig. 8 are given below; volumes in $Å^3$.

| | |
|---|---|
| Anion head volume | 50 |
| Anion tail volume | 286 |
| Anion pK | 5 |
| Cation head volume | 100 |
| Cation tail volume | 571 |
| Cation pK | 6.5 |
| Counterion+ volume | 70 |
| Counterion- volume | 70 |

Complex selection rules and preferred lipid systems

The present invention enables the formulation of amphoteric liposomes for a number of technical challenges. A more detailed analysis is given below for use of amphoteric liposomes in pharmaceutical applications. Amongst the pharmaceutical applications, systemic administration into the blood stream of an animal, preferably a mammal or human, is of particular importance. Amphoteric liposomes, amongst other use, have specific applicability in the intracellular delivery of cargo molecules. During uptake into the cells, said amphoteric liposomes are exposed to an acidified environment in the endosome or lysosome of cells. Destabilisation of the lipid phase, e.g., by enhanced fusogenicity is accepted to facilitate endosome escape and intracellular delivery.

The amphoteric liposomes of the invention with a preferred low value of κ(salt) respond advantageously to acidification with the intended destabilisation or formation of a fusogenic phase.

In order to be stable under storage conditions or while in the blood stream, a certain difference between κ(total) at neutral pH and κ(salt) is necessary. In a preferred embodiment, such difference is higher than 0.08 and in a more preferred case >0.12.

The difference between the κ(salt) and the κ(total) for acidic conditions is of less importance, since an unstable lipid phase under acidic conditions does not interfere with cellular uptake. In addition, methods to stabilise such lipid phase for production have been described above.

Amphoteric lipid systems with lipid head-group sizes between 40 and 190 $A^3$ and lipid hydrophobic tail sizes of 200, 350 or 500 $A^3$ have been analysed in the presence of large counter-cations, specifically sodium (65 $A^3$) and small counter-anions, specifically chloride (21 $A^3$).

For amphoter I systems, a mixture of about 1/3 cationic amphiphile and 2/3 anionic amphiphile is most typical and was used for the system analysis.

Besides the volume parameters, full dissociation of the cationic amphiphile was assumed throughout the range of pH values. For the anionic amphiphile, full dissociation at pH 8 and no dissociation at pH 4 was assumed. A library of 324 amphoter I lipid systems was constructed and preferred lipid systems having κ(salt) <0.3 and a difference in κ (total) and κ(salt) at pH 8 (called dκ(pH 8)) of more than 0.08, or preferably more than 0.12 are selected in the list below.

(The rest of this page is intentionally blank)

| System ID | Anion head | tail | Cation head | tail | k(salt) | Amphoter I 66 A k(pH 8) | 33 C k (pH4) | dk (pH8) | dk (pH 4) |
|---|---|---|---|---|---|---|---|---|---|
| 221 | 160 | 200 | 40 | 500 | 0,29 | 0,66 | 0,57 | 0,38 | 0,28 |
| 220 | 130 | 200 | 40 | 500 | 0,24 | 0,56 | 0,47 | 0,32 | 0,23 |
| 238 | 130 | 200 | 70 | 500 | 0,29 | 0,58 | 0,49 | 0,30 | 0,20 |
| 219 | 100 | 200 | 40 | 500 | 0,20 | 0,46 | 0,37 | 0,26 | 0,17 |
| 237 | 100 | 200 | 70 | 500 | 0,24 | 0,48 | 0,39 | 0,24 | 0,15 |
| 111 | 100 | 200 | 40 | 350 | 0,25 | 0,47 | 0,39 | 0,22 | 0,13 |
| 255 | 100 | 200 | 100 | 500 | 0,29 | 0,50 | 0,41 | 0,22 | 0,12 |
| 218 | 70 | 200 | 40 | 500 | 0,16 | 0,36 | 0,27 | 0,21 | 0,11 |
| 236 | 70 | 200 | 70 | 500 | 0,20 | 0,38 | 0,29 | 0,18 | 0,09 |
| 110 | 70 | 200 | 40 | 350 | 0,20 | 0,38 | 0,29 | 0,18 | 0,09 |
| 228 | 190 | 350 | 40 | 500 | 0,27 | 0,45 | 0,40 | 0,18 | 0,13 |
| 254 | 70 | 200 | 100 | 500 | 0,24 | 0,40 | 0,31 | 0,16 | 0,07 |
| 227 | 160 | 350 | 40 | 500 | 0,24 | 0,39 | 0,34 | 0,15 | 0,11 |
| 217 | 40 | 200 | 40 | 500 | 0,11 | 0,27 | 0,17 | 0,15 | 0,06 |
| 128 | 70 | 200 | 70 | 350 | 0,25 | 0,40 | 0,32 | 0,15 | 0,06 |
| 245 | 160 | 350 | 70 | 500 | 0,27 | 0,41 | 0,36 | 0,14 | 0,09 |
| 272 | 70 | 200 | 130 | 500 | 0,29 | 0,42 | 0,33 | 0,14 | 0,04 |
| 226 | 130 | 350 | 40 | 500 | 0,20 | 0,33 | 0,29 | 0,13 | 0,09 |
| 109 | 40 | 200 | 40 | 350 | 0,15 | 0,28 | 0,19 | 0,13 | 0,04 |
| 2 | 70 | 200 | 40 | 200 | 0,28 | 0,40 | 0,33 | 0,13 | 0,06 |
| 235 | 40 | 200 | 70 | 500 | 0,16 | 0,29 | 0,19 | 0,13 | 0,03 |
| 244 | 130 | 350 | 70 | 500 | 0,24 | 0,35 | 0,31 | 0,12 | 0,07 |
| 119 | 160 | 350 | 40 | 350 | 0,29 | 0,40 | 0,36 | 0,12 | 0,07 |
| 225 | 100 | 350 | 40 | 500 | 0,16 | 0,28 | 0,23 | 0,11 | 0,06 |
| 1 | 40 | 200 | 40 | 200 | 0,20 | 0.31 | 0,23 | 0,11 | 0,03 |
| 127 | 40 | 200 | 70 | 350 | 0.20 | 0,31 | 0,22 | 0,11 | 0,02 |
| 253 | 40 | 200 | 100 | 500 | 0,20 | 0,31 | 0,21 | 0,11 | 0,01 |
| 262 | 130 | 350 | 100 | 500 | 0,27 | 0,37 | 0,33 | 0,10 | 0,05 |
| 118 | 130 | 350 | 40 | 350 | 0,24 | 0,34 | 0,30 | 0,10 | 0,06 |
| 243 | 100 | 350 | 70 | 500 | 0,20 | 0,30 | 0,25 | 0,10 | 0,05 |
| 224 | 70 | 350 | 40 | 500 | 0,13 | 0,22 | 0,17 | 0,09 | 0,04 |
| 234 | 190 | 500 | 40 | 500 | 0,23 | 0,32 | 0,29 | 0,09 | 0,06 |
| 117 | 100 | 350 | 40 | 350 | 0,20 | 0,29 | 0,25 | 0,09 | 0,05 |
| 136 | 130 | 350 | 70 | 350 | 0,29 | 0,37 | 0,33 | 0,09 | 0,05 |
| 271 | 40 | 200 | 130 | 500 | 0,24 | 0,33 | 0,23 | 0,08 | -0,01 |
| 261 | 100 | 350 | 100 | 500 | 0,24 | 0,32 | 0,27 | 0,08 | 0,03 |
| 233 | 160 | 500 | 40 | 500 | 0,20 | 0,28 | 0,25 | 0,08 | 0,05 |

List of preferred amphoter I lipid systems ranked by dk(pH 8).

The most preferred amphoter I lipid systems are systems with following ID numbers:

| | | | | | | | | | | | |
|---|---|---|---|---|---|---|---|---|---|---|---|
| 2 | 109 | 110 | 111 | 119 | 128 | 217 | 218 | 219 | 220 | 221 | 226 |
| | 227 | 228 | 235 | 236 | 237 | 238 | 244 | 245 | 254 | 255 | 272 |

For the more preferred amphoter I systems with dκ(pH 8) >0.12 the following rules apply:

1) combined head-group size is about 160+/-80 $A^3$, preferably +/- 40 $A^3$

2) combined tail regions size is about 700+/-150 $A^3$; preferred are small anion tails having molecular volumes of

200...350 $A^3$ and large cation tails having molecular volumes of 350...500 $A^3$

3) the mixture has κ(salt)<0.3 and a difference between κ(salt) and κ(total) at pH 8 >0.12

For amphoter II systems, in a first analysis a mixture of about 1/3 cationic amphiphile and 2/3 anionic amphiphile was used.

Besides the volume parameters, for the cationic amphiphile no ionisation was assumed for pH 8 and full ionisation was assumed for pH 4. For the anionic amphiphile, full dissociation at pH 8 and no dissociation at pH 4 was assumed. A library of 324 amphoter II lipid systems with an excess of the anionic amphiphile was constructed and preferred lipid systems having κ(salt) <0.3 and a difference in κ(total) at pH 8 and κ(salt) (called "dκ(pH 8)") of more than 0.08, preferably 0.12, are selected in the list below.

(The rest of this page is intentionally blank)

| Anion head | tail | Cation head | tail | k(salt) | System ID | Amphoter II 33 A k(pH 8) | 66 C k (pH4) | dk (pH8) | dk (pH 4) |
|---|---|---|---|---|---|---|---|---|---|
| 40 | 500 | 160 | 200 | 0,29 | 584 | 0,60 | 0,62 | 0,31 | 0,34 |
| 40 | 500 | 130 | 200 | 0,24 | 566 | 0,50 | 0,52 | 0,26 | 0,28 |
| 70 | 500 | 130 | 200 | 0,29 | 567 | 0,52 | 0,54 | 0,23 | 0,26 |
| 40 | 500 | 100 | 200 | 0,20 | 548 | 0,40 | 0,43 | 0,20 | 0,23 |
| 70 | 500 | 100 | 200 | 0,24 | 549 | 0,42 | 0,45 | 0,18 | 0,20 |
| 40 | 350 | 100 | 200 | 0,25 | 542 | 0,43 | 0,44 | 0,17 | 0,18 |
| 40 | 500 | 190 | 350 | 0,27 | 710 | 0,43 | 0,42 | 0,16 | 0,15 |
| 100 | 500 | 100 | 200 | 0,29 | 550 | 0,44 | 0,47 | 0,15 | 0,18 |
| 40 | 500 | 70 | 200 | 0,16 | 530 | 0,30 | 0,33 | 0,14 | 0,17 |
| 160 | 200 | 40 | 500 | 0,29 | 720 | 0,42 | 0,34 | 0,14 | 0,06 |
| 40 | 500 | 160 | 350 | 0,24 | 692 | 0,37 | 0,37 | 0,14 | 0,13 |
| 130 | 200 | 40 | 500 | 0,24 | 719 | 0,37 | 0,30 | 0,13 | 0,05 |
| 40 | 350 | 70 | 200 | 0,20 | 524 | 0,33 | 0,34 | 0,13 | 0,14 |
| 40 | 200 | 70 | 200 | 0,28 | 518 | 0,40 | 0,37 | 0,13 | 0,09 |
| 130 | 200 | 70 | 500 | 0,29 | 737 | 0,41 | 0,33 | 0,13 | 0,05 |
| 100 | 200 | 40 | 500 | 0,20 | 718 | 0,33 | 0,25 | 0,13 | 0,05 |
| 100 | 200 | 70 | 500 | 0,24 | 736 | 0,36 | 0,29 | 0,12 | 0,04 |
| 70 | 500 | 160 | 350 | 0,27 | 693 | 0,39 | 0,39 | 0,12 | 0,12 |
| 70 | 500 | 70 | 200 | 0,20 | 531 | 0,32 | 0,35 | 0,12 | 0,15 |
| 100 | 200 | 100 | 500 | 0,29 | 754 | 0,40 | 0,32 | 0,12 | 0,04 |
| 70 | 200 | 40 | 500 | 0,16 | 717 | 0,28 | 0,20 | 0,12 | 0,04 |
| 70 | 200 | 70 | 500 | 0,20 | 735 | 0,32 | 0,24 | 0,12 | 0,04 |
| 40 | 350 | 160 | 350 | 0,29 | 686 | 0,40 | 0,38 | 0,12 | 0,09 |
| 40 | 500 | 130 | 350 | 0,20 | 674 | 0,31 | 0,31 | 0,11 | 0,11 |
| 70 | 200 | 100 | 500 | 0,24 | 753 | 0,35 | 0,28 | 0,11 | 0,03 |
| 40 | 200 | 40 | 500 | 0,11 | 716 | 0,23 | 0,15 | 0,11 | 0,03 |
| 70 | 200 | 130 | 500 | 0,29 | 771 | 0,39 | 0,31 | 0,11 | 0,03 |
| 40 | 200 | 70 | 500 | 0,16 | 734 | 0,27 | 0,19 | 0,11 | 0,03 |
| 40 | 200 | 100 | 350 | 0,25 | 644 | 0,36 | 0,29 | 0,11 | 0,04 |
| 40 | 200 | 40 | 200 | 0,20 | 500 | 0,31 | 0,27 | 0,11 | 0,07 |
| 40 | 200 | 70 | 350 | 0,20 | 626 | 0,31 | 0,24 | 0,11 | 0,04 |
| 40 | 200 | 100 | 500 | 0,20 | 752 | 0,31 | 0,23 | 0,11 | 0,03 |
| 70 | 350 | 70 | 200 | 0,25 | 525 | 0,36 | 0,37 | 0,10 | 0,11 |
| 40 | 200 | 40 | 350 | 0,15 | 608 | 0,25 | 0,18 | 0,10 | 0,04 |
| 40 | 200 | 130 | 500 | 0,24 | 770 | 0,34 | 0,27 | 0,10 | 0,02 |
| 40 | 350 | 130 | 350 | 0,24 | 668 | 0,34 | 0,32 | 0,10 | 0,08 |
| 70 | 200 | 70 | 350 | 0,25 | 627 | 0,35 | 0,29 | 0,10 | 0,03 |

(continued)

| Anion head | tail | Cation head | tail | k(salt) | System ID | Amphoter II 33 A k(pH 8) | 66 C k (pH4) | dk (pH8) | dk (pH 4) |
|---|---|---|---|---|---|---|---|---|---|
| 70 | 500 | 130 | 350 | 0,24 | 675 | 0,33 | 0,33 | 0,10 | 0,10 |
| 40 | 200 | 160 | 500 | 0,29 | 788 | 0,38 | 0,30 | 0,10 | 0,02 |
| 70 | 200 | 40 | 350 | 0,20 | 609 | 0,30 | 0,23 | 0,10 | 0,03 |
| 100 | 500 | 70 | 200 | 0,24 | 532 | 0,34 | 0,37 | 0,10 | 0,12 |
| 40 | 500 | 100 | 350 | 0,16 | 656 | 0,26 | 0,25 | 0,09 | 0,09 |
| 100 | 200 | 40 | 350 | 0,25 | 610 | 0,35 | 0,28 | 0,09 | 0,03 |
| 40 | 500 | 190 | 500 | 0,23 | 818 | 0,32 | 0,30 | 0,09 | 0,07 |
| 40 | 350 | 100 | 350 | 0,20 | 650 | 0,29 | 0,27 | 0,09 | 0,07 |
| 40 | 500 | 40 | 200 | 0,11 | 512 | 0,20 | 0,23 | 0,09 | 0,11 |
| 70 | 350 | 130 | 350 | 0,29 | 669 | 0,37 | 0,35 | 0,09 | 0,07 |
| 40 | 350 | 40 | 200 | 0,15 | 506 | 0,23 | 0,24 | 0,09 | 0,09 |
| 100 | 500 | 130 | 350 | 0,27 | 676 | 0,35 | 0,35 | 0,08 | 0,08 |
| 40 | 500 | 160 | 500 | 0,20 | 800 | 0,28 | 0,27 | 0,08 | 0,07 |

List of preferred amphoter II lipid systems having an excess of anionic amphiphile ranked by dκ(pH 8).

The most preferred amphoter II lipid systems having an excess of anionic amphiphile are systems with following ID numbers:

| | | | | | | | | | | | |
|---|---|---|---|---|---|---|---|---|---|---|---|
| 518 | 524 | 530 | 531 | 542 | 548 | 549 | 550 | 566 | 567 | 584 | 686 |
| | 692 | 693 | 710 | 717 | 718 | 719 | 720 | 735 | 736 | 737 | 754 |

For the more preferred amphoter II systems with excess of anionic amphiphile and with dκ(pH 8) >0.12 the following rules apply:

1) combined head-group size is about 160+/-80 $A^3$, preferably +/- 40 $A^3$

2) combined tail regions size is about 700+/-150 $A^3$; preferred are small anion tails having molecular volumes of 200...350 $A^3$ and large cation tails having molecular volumes of 350...500 $A^3$

3) the mix has κ(salt)<0.3 and a difference between κ(salt) and κ(total) at pH 8 >0.12.

Surprisingly, the preferred amphoter I and amphoter II systems with an excess of anionic amphiphile follow common selection rules in the presence of sodium chloride.

For amphoter II systems, in a another analysis, a mixture of about 2/3 cationic amphiphile and 1/3 anionic amphiphile was used.

Besides the volume parameters, for the cationic amphiphile no ionisation was assumed for pH 8 and full ionisation was assumed for pH 4. For the anionic amphiphile, full dissociation at pH 8 and no dissociation at pH 4 was assumed. A library of 324 amphoter II lipid systems with an excess of the cationic amphiphile was constructed and preferred lipid systems having κ(salt) <0.3 and a difference in κ(total) at pH 8 and κ(salt) (called "dκ(pH 8)") of more than 0.08 or preferred 0.12 are selected in the list below.

| Anion head | tail | Cation head | tail | k(salt) | | Amphoter II 33A k(pH 8) | 66C k (pH4) | dk (pH8) | dk (pH 4) |
|---|---|---|---|---|---|---|---|---|---|
| 160 | 200 | 40 | 500 | 0,29 | 1720 | 0,77 | 0,57 | 0,48 | 0,28 |
| 130 | 200 | 40 | 500 | 0,24 | 1719 | 0,67 | 0,47 | 0,43 | 0,23 |
| 130 | 200 | 70 | 500 | 0,29 | 1737 | 0,69 | 0,49 | 0,40 | 0,20 |
| 100 | 200 | 40 | 500 | 0,20 | 1718 | 0,57 | 0,37 | 0,37 | 0,17 |
| 100 | 200 | 70 | 500 | 0,24 | 1736 | 0,59 | 0,39 | 0,35 | 0,15 |
| 100 | 200 | 40 | 350 | 0,25 | 1610 | 0,58 | 0,39 | 0,33 | 0,13 |

(continued)

| | | | | | | Amphoter II | | | |
|---|---|---|---|---|---|---|---|---|---|
| Anion head | tail | Cation head | tail | k(salt) | | 33A k(pH 8) | 66C k (pH4) | dk (pH8) | dk (pH 4) |
| 100 | 200 | 100 | 500 | 0,29 | 1754 | 0,61 | 0,41 | 0,32 | 0,12 |
| 70 | 200 | 40 | 500 | 0,16 | 1717 | 0,47 | 0,27 | 0,31 | 0,11 |
| 70 | 200 | 70 | 500 | 0,20 | 1735 | 0,49 | 0,29 | 0,29 | 0,09 |
| 70 | 200 | 40 | 350 | 0,20 | 1609 | 0,48 | 0,29 | 0,28 | 0,09 |
| 70 | 200 | 100 | 500 | 0,24 | 1753 | 0,51 | 0,31 | 0,27 | 0,07 |
| 40 | 200 | 40 | 500 | 0,11 | 1716 | 0,37 | 0,17 | 0,26 | 0,06 |
| 70 | 200 | 70 | 350 | 0,25 | 1627 | 0,51 | 0,32 | 0,26 | 0,06 |
| 70 | 200 | 130 | 500 | 0,29 | 1771 | 0,53 | 0,33 | 0,25 | 0,04 |
| 40 | 200 | 40 | 350 | 0,15 | 1608 | 0,38 | 0,19 | 0,24 | 0,04 |
| 190 | 350 | 40 | 500 | 0,27 | 1727 | 0,51 | 0,40 | 0,24 | 0,13 |
| 70 | 200 | 40 | 200 | 0,28 | 1501 | 0,51 | 0,33 | 0,24 | 0,06 |
| 40 | 200 | 70 | 500 | 0,16 | 1734 | 0,39 | 0,19 | 0,24 | 0,03 |
| 160 | 350 | 40 | 500 | 0,24 | 1726 | 0,45 | 0,34 | 0,22 | 0,11 |
| 40 | 200 | 40 | 200 | 0,20 | 1500 | 0,41 | 0,23 | 0,21 | 0,03 |
| 40 | 200 | 70 | 350 | 0,20 | 1626 | 0,41 | 0,22 | 0,21 | 0,02 |
| 40 | 200 | 100 | 500 | 0,20 | 1752 | 0,41 | 0,21 | 0,21 | 0,01 |
| 160 | 350 | 70 | 500 | 0,27 | 1744 | 0,47 | 0,36 | 0,20 | 0,09 |
| 130 | 350 | 40 | 500 | 0,20 | 1725 | 0,39 | 0,29 | 0,19 | 0,09 |
| 40 | 200 | 130 | 500 | 0,24 | 1770 | 0,43 | 0,23 | 0,19 | -0,01 |
| 40 | 200 | 70 | 200 | 0,28 | 1518 | 0,46 | 0,28 | 0,19 | 0,01 |
| 40 | 200 | 100 | 350 | 0,25 | 1644 | 0,44 | 0,25 | 0,19 | -0,01 |
| 130 | 350 | 70 | 500 | 0,24 | 1743 | 0,41 | 0,31 | 0,18 | 0,07 |
| 160 | 350 | 40 | 350 | 0,29 | 1618 | 0,46 | 0,36 | 0,18 | 0,07 |
| 100 | 350 | 40 | 500 | 0,16 | 1724 | 0,34 | 0,23 | 0,17 | 0,06 |
| 40 | 200 | 160 | 500 | 0,29 | 1788 | 0,45 | 0,25 | 0,17 | -0,03 |
| 130 | 350 | 100 | 500 | 0,27 | 1761 | 0,43 | 0,33 | 0,16 | 0,05 |
| 130 | 350 | 40 | 350 | 0,24 | 1617 | 0,41 | 0,30 | 0,16 | 0,06 |
| 100 | 350 | 70 | 500 | 0,20 | 1742 | 0,36 | 0,25 | 0,16 | 0,05 |
| 70 | 350 | 40 | 500 | 0,13 | 1723 | 0,28 | 0,17 | 0,15 | 0,04 |
| 100 | 350 | 40 | 350 | 0,20 | 1616 | 0,35 | 0,25 | 0,15 | 0,05 |
| 130 | 350 | 70 | 350 | 0,29 | 1635 | 0,43 | 0,33 | 0,15 | 0,05 |
| 100 | 350 | 100 | 500 | 0,24 | 1760 | 0,38 | 0,27 | 0,14 | 0,03 |
| 70 | 350 | 70 | 500 | 0,16 | 1741 | 0,30 | 0,19 | 0,14 | 0,03 |
| 70 | 350 | 40 | 350 | 0,16 | 1615 | 0,29 | 0,19 | 0,14 | 0,03 |
| 100 | 350 | 70 | 350 | 0,24 | 1634 | 0,38 | 0,27 | 0,13 | 0,03 |
| 190 | 500 | 40 | 500 | 0,23 | 1733 | 0,36 | 0,29 | 0,13 | 0,06 |
| 40 | 350 | 40 | 500 | 0,09 | 1722 | 0,22 | 0,12 | 0,13 | 0,02 |
| 100 | 350 | 130 | 500 | 0,27 | 1778 | 0,40 | 0,29 | 0,13 | 0,02 |
| 160 | 500 | 40 | 500 | 0,20 | 1732 | 0,32 | 0,25 | 0,12 | 0,05 |
| 190 | 500 | 70 | 500 | 0,26 | 1751 | 0,38 | 0,31 | 0,12 | 0,05 |
| 100 | 350 | 40 | 200 | 0,25 | 1508 | 0,38 | 0,29 | 0,12 | 0,03 |
| 40 | 350 | 40 | 350 | 0,11 | 1614 | 0,24 | 0,13 | 0,12 | 0,02 |
| 70 | 350 | 40 | 200 | 0,20 | 1507 | 0,32 | 0,23 | 0,12 | 0,03 |
| 70 | 350 | 70 | 350 | 0,20 | 1633 | 0,32 | 0,22 | 0,12 | 0,02 |
| 70 | 350 | 100 | 500 | 0,20 | 1759 | 0,32 | 0,21 | 0,12 | 0,01 |
| 100 | 350 | 100 | 350 | 0,29 | 1652 | 0,41 | 0,30 | 0,12 | 0,02 |
| 40 | 350 | 40 | 200 | 0,15 | 1506 | 0,26 | 0,18 | 0,12 | 0,03 |
| 40 | 500 | 160 | 200 | 0,29 | 1584 | 0,40 | 0,35 | 0,12 | 0,07 |

(continued)

| Anion head | tail | Cation head | tail | k(salt) | | Amphoter II 33A k(pH 8) | 66C k (pH4) | dk (pH8) | dk (pH 4) |
|---|---|---|---|---|---|---|---|---|---|
| 70 | 350 | 70 | 200 | 0,25 | 1525 | 0,37 | 0,28 | 0,12 | 0,03 |
| 40 | 350 | 70 | 500 | 0,13 | 1740 | 0,24 | 0,14 | 0,11 | 0,01 |
| 130 | 500 | 40 | 500 | 0,17 | 1731 | 0,28 | 0,21 | 0,11 | 0,04 |
| 40 | 350 | 70 | 200 | 0,20 | 1524 | 0,31 | 0,23 | 0,11 | 0,03 |
| 160 | 500 | 70 | 500 | 0,23 | 1750 | 0,34 | 0,27 | 0,11 | 0,04 |
| 190 | 500 | 100 | 500 | 0,29 | 1769 | 0,40 | 0,33 | 0,11 | 0,04 |
| 40 | 500 | 130 | 200 | 0,24 | 1566 | 0,35 | 0,30 | 0,11 | 0,06 |
| 40 | 350 | 100 | 200 | 0,25 | 1542 | 0,36 | 0,28 | 0,11 | 0,02 |
| 70 | 500 | 130 | 200 | 0,29 | 1567 | 0,39 | 0,34 | 0,11 | 0,06 |
| 40 | 350 | 70 | 350 | 0,16 | 1632 | 0,26 | 0,16 | 0,11 | 0,00 |
| 70 | 350 | 100 | 350 | 0,24 | 1651 | 0,35 | 0,25 | 0,11 | 0,00 |
| 70 | 350 | 130 | 500 | 0,24 | 1777 | 0,34 | 0,23 | 0,11 | 0,00 |
| 100 | 500 | 40 | 500 | 0,14 | 1730 | 0,24 | 0,17 | 0,10 | 0,03 |
| 190 | 500 | 40 | 350 | 0,27 | 1625 | 0,37 | 0,31 | 0,10 | 0,04 |
| 130 | 500 | 70 | 500 | 0,20 | 1749 | 0,30 | 0,23 | 0,10 | 0,03 |
| 40 | 500 | 100 | 200 | 0,20 | 1548 | 0,30 | 0,25 | 0,10 | 0,05 |
| 160 | 500 | 100 | 500 | 0,26 | 1768 | 0,36 | 0,29 | 0,10 | 0,03 |

List of preferred amphoter II lipid systems having an excess of cationic amphiphile ranked by dκ(pH 8).

The most preferred amphoter II lipid systems having an excess of cationic amphiphile are systems with following ID numbers:

| | | | | | | | | | | | |
|---|---|---|---|---|---|---|---|---|---|---|---|
| 1500 | 1501 | 1506 | 1507 | 1508 | 1518 | 1525 | 1584 | 1608 | 1609 | 1610 | 1614 |
| | 1615 | 1616 | 1617 | 1618 | 1626 | 1627 | 1633 | 1634 | 1635 | 1644 | 1652 |
| | 1716 | 1717 | 1718 | 1719 | 1720 | 1722 | 1723 | 1724 | 1725 | 1726 | 1727 |
| | 1732 | 1733 | 1734 | 1735 | 1736 | 1737 | 1741 | 1742 | 1743 | 1744 | 1751 |
| | 1752 | 1753 | 1754 | 1759 | 1760 | 1761 | 1770 | 1771 | 1778 | 1788 | |

For the more preferred amphoter II systems with excess of the cationic amphiphile and with dκ(pH 8) >0.12 the following rules apply:

1) combined head-group size is about 160+/-80 $A^3$, preferably +/- 40 $A^3$

2) combined tail regions size is about 700+/-150 $A^3$,

3) the mix has κ(salt)<0,3 and a difference between κ(salt) and κ(total) at pH 8 >0.12

4) further preferred: the κ(anion) and κ(cation) are different from each other so that a more hexagonal and a more lamellar lipid complement each other.

For amphoter III systems, in a mixture of about 2/3 cationic amphiphile and 1/3 anionic amphiphile was used for the analysis.

Besides the volume parameters, for the cationic amphiphile no ionisation was assumed for pH 8 and full ionisation was assumed for pH 4. For the anionic amphiphile, full dissociation between pH 4 and pH 8 was assumed. A library of 324 amphoter III lipid systems with an excess of the cationic amphiphile was constructed and preferred lipid systems having κ(salt) <0.3 and a difference in κ(total) at pH 8 and κ(salt) called dκ(pH 8) of more than 0.08, preferably 0.12, are selected in the list below.

| Anion head | tail | Cation head | tail | k(salt) | System ID | Amphoter III 33 A k(pH 8) | 66C k (pH4) | dk (pH8) | dk (pH 4) |
|---|---|---|---|---|---|---|---|---|---|
| 40 | 500 | 160 | 200 | 0,29 | 2084 | 0,60 | 0,59 | 0,31 | 0,30 |
| 40 | 500 | 130 | 200 | 0,24 | 2066 | 0,50 | 0,49 | 0,26 | 0,25 |
| 70 | 500 | 130 | 200 | 0,29 | 2067 | 0,52 | 0,51 | 0,23 | 0,22 |
| 40 | 500 | 100 | 200 | 0,20 | 2048 | 0,40 | 0,39 | 0,20 | 0,19 |
| 70 | 500 | 100 | 200 | 0,24 | 2049 | 0,42 | 0,41 | 0,18 | 0,17 |
| 40 | 350 | 100 | 200 | 0,25 | 2042 | 0,43 | 0,40 | 0,17 | 0,15 |
| 40 | 500 | 190 | 350 | 0,27 | 2210 | 0,43 | 0,40 | 0,16 | 0,13 |
| 100 | 500 | 100 | 200 | 0,29 | 2050 | 0,44 | 0,43 | 0,15 | 0,14 |
| 40 | 500 | 70 | 200 | 0,16 | 2030 | 0,30 | 0,29 | 0,14 | 0,13 |
| 160 | 200 | 40 | 500 | 0,29 | 2220 | 0,42 | 0,33 | 0,14 | 0,04 |
| 40 | 500 | 160 | 350 | 0,24 | 2192 | 0,37 | 0,35 | 0,14 | 0,11 |
| 130 | 200 | 40 | 500 | 0,24 | 2219 | 0,37 | 0,28 | 0,13 | 0,04 |
| 40 | 350 | 70 | 200 | 0,20 | 2024 | 0,33 | 0,30 | 0,13 | 0,10 |
| 40 | 200 | 70 | 200 | 0,28 | 2018 | 0,40 | 0,33 | 0,13 | 0,06 |
| 130 | 200 | 70 | 500 | 0,29 | 2237 | 0,41 | 0,32 | 0,13 | 0,04 |
| 100 | 200 | 40 | 500 | 0,20 | 2218 | 0,33 | 0,23 | 0,13 | 0,03 |
| 100 | 200 | 70 | 500 | 0,24 | 2236 | 0,36 | 0,27 | 0,12 | 0,03 |
| 70 | 500 | 160 | 350 | 0,27 | 2193 | 0,39 | 0,37 | 0,12 | 0,10 |
| 70 | 500 | 70 | 200 | 0,20 | 2031 | 0,32 | 0,31 | 0,12 | 0,11 |
| 100 | 200 | 100 | 500 | 0,29 | 2254 | 0,40 | 0,31 | 0,12 | 0,03 |
| 70 | 200 | 40 | 500 | 0,16 | 2217 | 0,28 | 0,18 | 0,12 | 0,03 |
| 70 | 200 | 70 | 500 | 0,20 | 2235 | 0,32 | 0,22 | 0,12 | 0,02 |
| 40 | 350 | 160 | 350 | 0,29 | 2186 | 0,40 | 0,36 | 0,12 | 0,07 |
| 40 | 500 | 130 | 350 | 0,20 | 2174 | 0,31 | 0,29 | 0,11 | 0,09 |
| 70 | 200 | 100 | 500 | 0,24 | 2253 | 0,35 | 0,26 | 0,11 | 0,02 |
| 40 | 200 | 40 | 500 | 0,11 | 2216 | 0,23 | 0,13 | 0,11 | 0,02 |
| 70 | 200 | 130 | 500 | 0,29 | 2271 | 0,39 | 0,30 | 0,11 | 0,02 |
| 40 | 200 | 70 | 500 | 0,16 | 2234 | 0,27 | 0,17 | 0,11 | 0,02 |
| 40 | 200 | 100 | 350 | 0,25 | 2144 | 0,36 | 0,27 | 0,11 | 0,02 |
| 40 | 200 | 40 | 200 | 0,20 | 2000 | 0,31 | 0,23 | 0,11 | 0,03 |
| 40 | 200 | 70 | 350 | 0,20 | 2126 | 0,31 | 0,22 | 0,11 | 0,02 |
| 40 | 200 | 100 | 500 | 0,20 | 2252 | 0,31 | 0,21 | 0.11 | 0,01 |
| 70 | 350 | 70 | 200 | 0,25 | 2025 | 0,36 | 0,33 | 0,10 | 0,08 |
| 40 | 200 | 40 | 350 | 0,15 | 2108 | 0,25 | 0,16 | 0,10 | 0,02 |
| 40 | 200 | 130 | 500 | 0,24 | 2270 | 0,34 | 0,25 | 0,10 | 0,01 |
| 40 | 350 | 130 | 350 | 0,24 | 2168 | 0,34 | 0,30 | 0,10 | 0,06 |
| 70 | 200 | 70 | 350 | 0,25 | 2127 | 0,35 | 0,27 | 0,10 | 0,01 |
| 70 | 500 | 130 | 350 | 0,24 | 2175 | 0,33 | 0,31 | 0,10 | 0,08 |
| 40 | 200 | 160 | 500 | 0,29 | 2288 | 0,38 | 0,29 | 0,10 | 0,01 |
| 70 | 200 | 40 | 350 | 0,20 | 2109 | 0,30 | 0,21 | 0,10 | 0,01 |
| 100 | 500 | 70 | 200 | 0,24 | 2032 | 0,34 | 0,33 | 0,10 | 0,09 |
| 40 | 500 | 100 | 350 | 0,16 | 2156 | 0,26 | 0,23 | 0,09 | 0,07 |
| 100 | 200 | 40 | 350 | 0,25 | 2110 | 0,35 | 0,26 | 0,09 | 0,01 |
| 40 | 500 | 190 | 500 | 0,23 | 2318 | 0,32 | 0,29 | 0,09 | 0,06 |
| 40 | 350 | 100 | 350 | 0,20 | 2150 | 0,29 | 0,25 | 0,09 | 0,05 |
| 40 | 500 | 40 | 200 | 0,11 | 2012 | 0,20 | 0,19 | 0,09 | 0,08 |
| 70 | 350 | 130 | 350 | 0,29 | 2169 | 0,37 | 0,33 | 0,09 | 0,05 |
| 40 | 350 | 40 | 200 | 0,15 | 2006 | 0,23 | 0,20 | 0,09 | 0,06 |

(continued)

| Anion head | tail | Cation head | tail | k(salt) | System ID | Amphoter III 33 A k(pH 8) | 66C k (pH4) | dk (pH8) | dk (pH 4) |
|---|---|---|---|---|---|---|---|---|---|
| 100 | 500 | 130 | 350 | 0,27 | 2176 | 0,35 | 0,33 | 0,08 | 0,06 |
| 40 | 500 | 160 | 500 | 0,20 | 2300 | 0,28 | 0,25 | 0,08 | 0,05 |

List of preferred amphoter III lipid systems having an excess of cationic amphiphile ranked by dκ(pH 8).

The most preferred amphoter III lipid systems are systems with following ID numbers:

| | | | | | | | | | | | |
|---|---|---|---|---|---|---|---|---|---|---|---|
| 2018 | 2024 | 2030 | 2031 | 2042 | 2048 | 2049 | 2050 | 2066 | 2067 | 2084 | 2186 |
| | 2192 | 2193 | 2210 | 2217 | 2218 | 2219 | 2220 | 2235 | 2236 | 2237 | 2254 |

For the more preferred amphoter III systems with excess of cationic amphiphile and with dκ(pH 8) >0.12 the following rules apply:

1) combined head-group size is about 160+/-80 $A^3$, preferably +/- 40 $A^3$

2) combined tail regions size is about 700+/-150 $A^3$,

3) the mix has κ(salt) <0.3 and a difference between κ(salt) and κ(total) at pH 8 >0.12

4) further preferred: the κ(anion) and κ(cation) are different from each other so that a more hexagonal and a more lamellar lipid complement each other.

Interestingly, both systems with an excess of the cationic amphiphiles are commanded by common rules in the presence of sodium chloride as counterions.

Also, amphoter II systems in a mixture of about equal molar amounts of cationic amphiphile and anionic amphiphile were analysed.

Besides the volume parameters, for the cationic amphiphile no ionisation was assumed for pH 8 and full ionisation was assumed for pH 4. For the anionic amphiphile, full dissociation at pH 8 and no dissociation at pH 4 was assumed. A library of 324 amphoter II lipid systems with about equal amounts of the cationic and anionic amphiphile was constructed and preferred lipid systems having κ(salt) <0.3 and a difference in κ(total) at pH 8 and κ(salt) called dκ (pH 8) of more than 0.08, preferably 0.12, are selected in the list below.

(The rest of this page is intentionally blank)

| Anion head | tail | Cation head | tail | k(salt) | System ID | Amphoter II 50 A k(pH 8) | 50 C k (pH4) | dk (pH8) | dk (pH 4) |
|---|---|---|---|---|---|---|---|---|---|
| 160 | 200 | 40 | 500 | 0,29 | 1220 | 0,60 | 0,46 | 0,32 | 0,18 |
| 130 | 200 | 40 | 500 | 0,24 | 1219 | 0,53 | 0,39 | 0,28 | 0,14 |
| 130 | 200 | 70 | 500 | 0,29 | 1237 | 0,56 | 0,42 | 0,27 | 0,13 |
| 100 | 200 | 40 | 500 | 0,20 | 1218 | 0,45 | 0,31 | 0,25 | 0,11 |
| 100 | 200 | 70 | 500 | 0,24 | 1236 | 0,48 | 0,34 | 0,24 | 0,10 |
| 100 | 200 | 100 | 500 | 0,29 | 1254 | 0,51 | 0,37 | 0,23 | 0,09 |
| 70 | 200 | 40 | 500 | 0,16 | 1217 | 0,38 | 0,24 | 0,22 | 0,08 |
| 40 | 500 | 160 | 200 | 0,29 | 1084 | 0,51 | 0,49 | 0,22 | 0,21 |
| 100 | 200 | 40 | 350 | 0,25 | 1110 | 0,47 | 0,34 | 0,22 | 0,08 |
| 70 | 200 | 70 | 500 | 0,20 | 1235 | 0,41 | 0,27 | 0,21 | 0,07 |
| 70 | 200 | 40 | 350 | 0,20 | 1109 | 0,39 | 0,26 | 0,19 | 0,06 |
| 70 | 200 | 100 | 500 | 0,24 | 1253 | 0,44 | 0,30 | 0,19 | 0,05 |
| 40 | 200 | 40 | 500 | 0,11 | 1216 | 0,30 | 0,16 | 0,19 | 0,05 |
| 40 | 500 | 130 | 200 | 0,24 | 1066 | 0,43 | 0,42 | 0,19 | 0,17 |

(continued)

| Anion head | tail | Cation head | tail | k(salt) | System ID | Amphoter II 50 A k(pH 8) | 50 C k (pH4) | dk (pH8) | dk (pH 4) |
|---|---|---|---|---|---|---|---|---|---|
| 70 | 200 | 70 | 350 | 0,25 | 1127 | 0,44 | 0,31 | 0,18 | 0,05 |
| 70 | 200 | 130 | 500 | 0,29 | 1271 | 0,47 | 0,33 | 0,18 | 0,04 |
| 40 | 200 | 70 | 500 | 0,16 | 1234 | 0,33 | 0,19 | 0,18 | 0,03 |
| 70 | 500 | 130 | 200 | 0,29 | 1067 | 0,46 | 0,45 | 0,17 | 0,16 |
| 40 | 200 | 40 | 350 | 0,15 | 1108 | 0,32 | 0,19 | 0,17 | 0,04 |
| 40 | 200 | 40 | 200 | 0,20 | 1000 | 0,36 | 0,25 | 0,16 | 0,05 |
| 70 | 200 | 40 | 200 | 0,28 | 1001 | 0,44 | 0,33 | 0,16 | 0,05 |
| 40 | 200 | 70 | 200 | 0,28 | 1018 | 0,44 | 0,33 | 0,16 | 0,05 |
| 40 | 200 | 70 | 350 | 0,20 | 1126 | 0,36 | 0,23 | 0,16 | 0,03 |
| 40 | 200 | 100 | 500 | 0,20 | 1252 | 0,36 | 0,22 | 0,16 | 0,02 |
| 40 | 500 | 100 | 200 | 0,20 | 1048 | 0,36 | 0,34 | 0,16 | 0,14 |
| 40 | 200 | 100 | 350 | 0,25 | 1144 | 0,41 | 0,27 | 0,15 | 0,02 |
| 40 | 200 | 130 | 500 | 0,24 | 1270 | 0,39 | 0,25 | 0,15 | 0,01 |
| 40 | 350 | 100 | 200 | 0,25 | 1042 | 0,40 | 0,36 | 0,15 | 0,11 |
| 70 | 500 | 100 | 200 | 0,24 | 1049 | 0,39 | 0,37 | 0,14 | 0,13 |
| 40 | 200 | 160 | 500 | 0,29 | 1288 | 0,42 | 0,28 | 0,14 | 0,00 |
| 190 | 350 | 40 | 500 | 0,27 | 1227 | 0,40 | 0,33 | 0,13 | 0,06 |
| 100 | 500 | 100 | 200 | 0,29 | 1050 | 0,42 | 0,40 | 0,13 | 0,12 |
| 160 | 350 | 40 | 500 | 0,24 | 1226 | 0,36 | 0,29 | 0,13 | 0,05 |
| 40 | 350 | 70 | 200 | 0,20 | 1024 | 0,33 | 0,28 | 0,13 | 0,08 |
| 40 | 500 | 70 | 200 | 0,16 | 1030 | 0,28 | 0,27 | 0,12 | 0,11 |
| 160 | 350 | 70 | 500 | 0,27 | 1244 | 0,39 | 0,32 | 0,12 | 0,05 |
| 130 | 350 | 40 | 500 | 0,20 | 1225 | 0,32 | 0,25 | 0,12 | 0,05 |
| 70 | 350 | 70 | 200 | 0,25 | 1025 | 0,37 | 0,33 | 0,11 | 0,07 |
| 130 | 350 | 70 | 500 | 0,24 | 1243 | 0,35 | 0,28 | 0,11 | 0,04 |
| 100 | 350 | 40 | 500 | 0,16 | 1224 | 0,28 | 0,20 | 0,11 | 0,04 |
| 70 | 500 | 70 | 200 | 0,20 | 1031 | 0,31 | 0,30 | 0,11 | 0,10 |
| 130 | 350 | 100 | 500 | 0,27 | 1261 | 0,38 | 0,31 | 0,11 | 0,04 |
| 40 | 500 | 190 | 350 | 0,27 | 1210 | 0,38 | 0,34 | 0,11 | 0,07 |
| 100 | 350 | 70 | 500 | 0,20 | 1242 | 0,31 | 0,23 | 0,11 | 0,03 |
| 40 | 350 | 40 | 200 | 0,15 | 1006 | 0,25 | 0,21 | 0,10 | 0,06 |
| 70 | 350 | 40 | 500 | 0,13 | 1223 | 0,23 | 0,16 | 0,10 | 0,03 |
| 100 | 350 | 100 | 500 | 0,24 | 1260 | 0,34 | 0,26 | 0,10 | 0,03 |
| 40 | 500 | 160 | 350 | 0,24 | 1192 | 0,33 | 0,30 | 0,10 | 0,06 |
| 70 | 350 | 70 | 500 | 0,16 | 1241 | 0,26 | 0,19 | 0,10 | 0,03 |
| 100 | 500 | 70 | 200 | 0,24 | 1032 | 0,34 | 0,33 | 0,10 | 0,08 |
| 40 | 350 | 40 | 500 | 0,09 | 1222 | 0,19 | 0,12 | 0,10 | 0,02 |
| 100 | 350 | 130 | 500 | 0,27 | 1278 | 0,37 | 0,29 | 0,10 | 0,02 |
| 70 | 500 | 160 | 350 | 0,27 | 1193 | 0,36 | 0,33 | 0,09 | 0,06 |
| 160 | 350 | 40 | 350 | 0,29 | 1118 | 0,38 | 0,32 | 0,09 | 0,03 |
| 130 | 350 | 40 | 350 | 0,24 | 1117 | 0,34 | 0,27 | 0,09 | 0,03 |
| 100 | 350 | 70 | 350 | 0,24 | 1134 | 0,34 | 0,27 | 0,09 | 0,03 |
| 70 | 350 | 100 | 350 | 0,24 | 1151 | 0,34 | 0,27 | 0,09 | 0,03 |
| 40 | 350 | 130 | 350 | 0,24 | 1168 | 0,34 | 0,27 | 0,09 | 0,03 |
| 70 | 350 | 40 | 200 | 0,20 | 1007 | 0,29 | 0,25 | 0,09 | 0,05 |
| 70 | 350 | 40 | 350 | 0,16 | 1115 | 0,25 | 0,19 | 0,09 | 0,03 |
| 100 | 350 | 40 | 350 | 0,20 | 1116 | 0,29 | 0,23 | 0,09 | 0,03 |

(continued)

| Anion head | tail | Cation head | tail | k(salt) | System ID | Amphoter II 50 A k(pH 8) | 50 C k (pH4) | dk (pH8) | dk (pH 4) |
|---|---|---|---|---|---|---|---|---|---|
| 40 | 350 | 70 | 350 | 0,16 | 1132 | 0,25 | 0,19 | 0,09 | 0,03 |
| 70 | 350 | 70 | 350 | 0,20 | 1133 | 0,29 | 0,23 | 0,09 | 0,03 |
| 130 | 350 | 70 | 350 | 0,29 | 1135 | 0,38 | 0,32 | 0,09 | 0,03 |
| 100 | 350 | 100 | 350 | 0,29 | 1152 | 0,38 | 0,32 | 0,09 | 0,03 |
| 70 | 350 | 130 | 350 | 0,29 | 1169 | 0,38 | 0,32 | 0,09 | 0,03 |
| 40 | 350 | 160 | 350 | 0,29 | 1186 | 0,38 | 0,32 | 0,09 | 0,03 |
| 70 | 350 | 100 | 500 | 0,20 | 1259 | 0,29 | 0,22 | 0,09 | 0,02 |
| 40 | 350 | 40 | 350 | 0,11 | 1114 | 0,21 | 0,14 | 0,09 | 0,03 |
| 40 | 350 | 100 | 350 | 0,20 | 1150 | 0,29 | 0,23 | 0,09 | 0,03 |
| 40 | 500 | 40 | 200 | 0,11 | 1012 | 0,21 | 0,19 | 0,09 | 0,08 |

List of preferred amphoter II lipid systems having an equal amount of cationic and anionic amphiphile ranked by dκ(pH 8).

The most preferred amphoter II lipid systems having an equal amount of cationic and anionic amphiphile are systems with following ID numbers:

| | | | | | | | | | | | |
|---|---|---|---|---|---|---|---|---|---|---|---|
| 1000 | 1001 | 1018 | 1024 | 1030 | 1042 | 1048 | 1049 | 1050 | 1066 | 1067 | 1084 |
| | 1108 | 1109 | 1110 | 1126 | 1127 | 1144 | 1216 | 1217 | 1218 | 1219 | 1220 |
| | 1225 | 1226 | 1227 | 1234 | 1235 | 1236 | 1237 | 1244 | 1252 | 1253 | 1254 |
| | 1270 | 1271 | 1288 | | | | | | | | |

For the more preferred amphoter II systems with equal amounts of cationic and anionic amphiphile and with dκ(pH 8) >0.12 the following rules apply:

1) combined head-group size is about 160+/-80 $A^3$, preferably +/- 40 $A^3$

2) combined tail regions size is about 700+/-150 $A^3$,

3) the mix has κ(salt) <0.3 and a difference between κ(salt) and κ(total) at pH 8 >0.12.

The influence of neutral or zwitterionic lipids

Neutral lipids comprise structures such as phosphatidylcholine, phosphatidyl ethanolamine, sphingolipids or cholesterol and the like. As these lipids do not have pH responsive elements that would react between pH 3 and 8, no changes in the molecular geometry occur in this range. Depending on individual κ values of the neutral lipids, a dilution of the bistable behaviour of the amphoteric lipid pair occurs and the steepness of d(κ)/d(pH) becomes smaller, as shown in Fig. 9. In addition, the curve in the phase diagram is shifter towards lower or higher values of κ, depending on the neutral lipid used for dilution of the charged lipids. The parameters used for the calculation of Fig. 9 are given below; volumes in $Å^3$.

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ volume | 70 |

(continued)

| | |
|---|---|
| Counterion- volume | 70 |

Fig. 9 illustrates this behaviour for addition of different neutral lipids with κ values of 0.5, 0.3 or 0.19, respectively, in combination with the amphoter II model system described above. The amplitude of the system is reduced from Δκ = 0.089 to 0.044, while the minimum value follows the κ for the individual neutral components.

Thus, in a preferred embodiment of the invention, neutral lipids may be added to the salt forming charged lipids to 65 mol.% or less. More preferred are additions of 50 mol.% and even more preferred are additions of 35 mol.% or less neutral lipid. The addition of neutral lipids might be done to stabilise further the lipid bilayer and preferred lipids for such intent have higher κ values, e.g., κ >0.4 or even about 0.5. Typical examples for such lipids are the phosphatidylcholines with C14 to C18 alkyl chains in the hydrophobic region. As most polar regions of lipids, head-groups of phosphatidylcholines recruit counterions.

The addition of neutral lipids might also extend the zone of fusogenic behaviours and to this end neutral lipid with low values of κ may be employed. Such preferred lipids have κ values of 0.3 or less; more preferred lipids have κ values of about 0.2. Typical examples for such lipids are phosphatidylethanolamines. Phosphatidylethanolamines are assumed to form internal salt bridges (betaine structures) between the terminal amino group and the phosphate; therefore no counterions are recruited to the head-groups.

Phosphatidylethanolamines with C14 to C18 alkyl chains are preferred lipids to modulated fusogenicity of the amphoteric liposomes.

It is of course possible to use mixtures of different neutral lipids to optimize the balance between fusogenicity and stability of such systems.

In summary, the algorithm disclosed herein is suitable to describe the phase transitions in amphoteric liposomes. Essential building blocks of the algorithm are (i) the lipid shape theory, (ii) the notion that counterions are part of the head-group volume and (iii) that lipid salt formation may occur in bilayers, leading to dissociation of the counterions. The simplicity of the algorithm makes it easy for the person skilled in the art to reproduce the calculations and adopt the system to the individual goals. It is of course possible to use other tools than indicated to calculate molecular volumes. The qualitative prediction would not even change if molecular cross-sections are used instead of the volumes. Of course, one would have to recalibrate the results in such a case.

As mentioned above, steric hindrance might interfere with salt formation. In such case, the phase behaviour may differ substantially from what has been hereinbefore described. Most importantly, no dual stability at low and neutral pH is observed. Instead, a saddle of stability is observed for intermediate pH with more instable zones on either one or both ends, depending on the type of amphoter system. Typical examples are included into the examples below.

Also, the molecular volume calculations disclosed herein are silent towards chain saturation in the hydrophobic parts. Use of unsaturated lipids has specific advantages, since lipid membranes comprising such lipids have higher fluidity at ambient temperatures which might improve the fusion behaviour. It is also known that unsaturated lipids exert lateral pressure in the membrane, thus a correction factor can be inserted to reflect the apparent volume of these components. Such correction factor is higher than 1.

The algorithm described above assumes the formation of lipid salts with a 1:1 stoichiometry between the two charged partners. It is of course possible to extent this concept to more complex situations, e.g., binding of multiple, monocharged lipids to a single other lipid with a number of charged groups. Binding of CHEMS, DOGS or oleic acid to amphiphilic derivatives of spermin may provide an example for this group, but many other combinations do exist. In another embodiment, the charged groups on the lipids might be more complex and comprise different charged groups, e.g., as in HistChol.

In such a case, 1:1 complexes might be formed with either other lipid anions or cations. Applying the concept of this invention for this example, counterion displacement can occur between the imidazolium cation and a separate lipid anion, e.g., CHEMS or DOGS or oleic acid anions; in addition, the parallel counterion dissociation from the carboxyl of the histidine moiety further supports the formation of a hexagonal phase.

It is apparent for the skilled artisan that the more complex arrangements of lipids put an extra burden on the steric compatibility of the interaction partners which may lead to lack of experimental success or mixed forms of possible interactions where not every possible binding site is engaged in salt formation.

<u>Complex selection rules and preferred lipid systems further comprising neutral lipids</u>

The present invention allows the formulation of amphoteric liposomes for a number of technical challenges. A more detailed analysis is given below for use of amphoteric liposomes further comprising neutral lipids in pharmaceutical applications and the same considerations with respect to production, storage and use as above apply also for these

systems.

In addition to the preferred systems described above, neutral lipids may allow use of lipid salts with higher κ (salt). This is especially the case for neutral lipids with low κ(lipid).

The particular analysis given below therefore targets lipid salts with κ(salt) between 0.25 and 0.45 that benefit from the addition of a fusogenic neutral lipid, e.g., DOPE.

As described in detail above, the addition of any neutral lipid does not contribute to dκ/dpH and rather diminishes the pH induced amplitude in the lipid phase behaviour. Therefore, in a preferred embodiment, dκ(pH 8) may be higher than 0.12 and, in a more preferred case, >0.16 for systems comprising neutral lipids. The increased stringency for the selection of dκ(pH 8) allows for the presence of neutral lipids diluting the amplitude of the system.

Amphoteric lipid systems with lipid head-group sizes between 40 and 190 $A^3$ and lipid hydrophobic tail sizes of 200, 350 or 500 $A^3$ have been analysed in the presence of large counter-cations, specifically sodium (65 $A^3$) and small counter-anions, specifically chloride (21 $A^3$).

For amphoter I systems, a mixture of about 1/3 cationic amphiphile and 2/3 anionic amphiphile is most typical and was used for the system analysis.

Besides the volume parameters, full dissociation of the cationic amphiphile was assumed throughout the range of pH values. For the anionic amphiphile, full dissociation at pH 8 and no dissociation at pH 4 was assumed. A library of 324 amphoter I lipid systems was constructed and preferred lipid systems having κ(salt) between 0.25 and 0.45 and a difference in κ(total) at pH 8 and κ(salt) called dκ(pH 8) of more than 0.12, preferably more than 0.16, are selected in the list below.

| System ID | Anion head | tail | Cation head | tail | k(salt) | Amphoter I 66 A k(pH 8) | 33 C k (pH4) | dk (pH8) | dk (pH 4) |
|---|---|---|---|---|---|---|---|---|---|
| 222 | 190 | 200 | 40 | 500 | 0,33 | 0,76 | 0,67 | 0,43 | 0,34 |
| 240 | 190 | 200 | 70 | 500 | 0,37 | 0,78 | 0,69 | 0,41 | 0,32 |
| 258 | 190 | 200 | 100 | 500 | 0,41 | 0,80 | 0,71 | 0,39 | 0,29 |
| 221 | 160 | 200 | 40 | 500 | 0,29 | 0,66 | 0,57 | 0,38 | 0,28 |
| 114 | 190 | 200 | 40 | 350 | 0,42 | 0,77 | 0,68 | 0,35 | 0,27 |
| 239 | 160 | 200 | 70 | 500 | 0,33 | 0,68 | 0,59 | 0,35 | 0,26 |
| 257 | 160 | 200 | 100 | 500 | 0,37 | 0,70 | 0,61 | 0,33 | 0,24 |
| 113 | 160 | 200 | 40 | 350 | 0,36 | 0,67 | 0,59 | 0,31 | 0,22 |
| 275 | 160 | 200 | 130 | 500 | 0,41 | 0,72 | 0,63 | 0,31 | 0,21 |
| 238 | 130 | 200 | 70 | 500 | 0,29 | 0,58 | 0,49 | 0,30 | 0,20 |
| 131 | 160 | 200 | 70 | 350 | 0,42 | 0,70 | 0,61 | 0,28 | 0,20 |
| 256 | 130 | 200 | 100 | 500 | 0,33 | 0,60 | 0,51 | 0,27 | 0,18 |
| 112 | 130 | 200 | 40 | 350 | 0,31 | 0,57 | 0,49 | 0,26 | 0,18 |
| 274 | 130 | 200 | 130 | 500 | 0,37 | 0,62 | 0,53 | 0,25 | 0,16 |
| 130 | 130 | 200 | 70 | 350 | 0,36 | 0,60 | 0,51 | 0,24 | 0,15 |
| 292 | 130 | 200 | 160 | 500 | 0,41 | 0,64 | 0,55 | 0,23 | 0,13 |
| 111 | 100 | 200 | 40 | 350 | 0,25 | 0,47 | 0,39 | 0,22 | 0,13 |
| 255 | 100 | 200 | 100 | 500 | 0,29 | 0,50 | 0,41 | 0,22 | 0,12 |
| 148 | 130 | 200 | 100 | 350 | 0,42 | 0,63 | 0,54 | 0,21 | 0,12 |
| 273 | 100 | 200 | 130 | 500 | 0,33 | 0,52 | 0,43 | 0,19 | 0,10 |
| 129 | 100 | 200 | 70 | 350 | 0,31 | 0,50 | 0,42 | 0,19 | 0,11 |
| 4 | 130 | 200 | 40 | 200 | 0,43 | 0,60 | 0,53 | 0,18 | 0,10 |
| 228 | 190 | 350 | 40 | 500 | 0,27 | 0,45 | 0,40 | 0,18 | 0,13 |
| 291 | 100 | 200 | 160 | 500 | 0,37 | 0,54 | 0,45 | 0,17 | 0,08 |
| 147 | 100 | 200 | 100 | 350 | 0,36 | 0,53 | 0,44 | 0,17 | 0,08 |
| 246 | 190 | 350 | 70 | 500 | 0,31 | 0,47 | 0,42 | 0,16 | 0,11 |
| 3 | 100 | 200 | 40 | 200 | 0,35 | 0,50 | 0,43 | 0,15 | 0,08 |
| 128 | 70 | 200 | 70 | 350 | 0,25 | 0,40 | 0,32 | 0,15 | 0,06 |
| 309 | 100 | 200 | 190 | 500 | 0,41 | 0,56 | 0,47 | 0,15 | 0,05 |
| 264 | 190 | 350 | 100 | 500 | 0,34 | 0,49 | 0,44 | 0,14 | 0,10 |
| 165 | 100 | 200 | 130 | 350 | 0,42 | 0,56 | 0,47 | 0,14 | 0,05 |

(continued)

| System ID | Anion head | tail | Cation head | tail | k(salt) | Amphoter I 66 A k(pH 8) | 33 C k (pH4) | dk (pH8) | dk (pH 4) |
|---|---|---|---|---|---|---|---|---|---|
| 245 | 160 | 350 | 70 | 500 | 0,27 | 0,41 | 0,36 | 0,14 | 0,09 |
| 272 | 70 | 200 | 130 | 500 | 0,29 | 0,42 | 0,33 | 0,14 | 0,04 |
| 2 | 70 | 200 | 40 | 200 | 0,28 | 0,40 | 0,33 | 0,13 | 0,06 |
| 282 | 190 | 350 | 130 | 500 | 0,38 | 0,51 | 0,46 | 0,13 | 0,08 |
| 120 | 190 | 350 | 40 | 350 | 0,33 | 0,46 | 0,42 | 0,13 | 0,09 |
| 21 | 100 | 200 | 70 | 200 | 0,43 | 0,55 | 0,48 | 0,13 | 0,06 |
| 146 | 70 | 200 | 100 | 350 | 0,31 | 0,43 | 0,35 | 0,12 | 0,04 |
| 263 | 160 | 350 | 100 | 500 | 0,31 | 0,43 | 0,38 | 0,12 | 0,08 |

List of preferred amphoter I lipid systems suitable for combination with neutral lipid or neutral lipid mixtures with low κ(lipid) ranked by dκ(pH 8).

The most preferred amphoter I lipid systems suitable for combination with neutral lipid or neutral lipid mixtures with low κ(lipid) are systems with following ID numbers:

| | | | | | | | | | | | |
|---|---|---|---|---|---|---|---|---|---|---|---|
| 4 | 111 | 112 | 113 | 114 | 129 | 130 | 131 | 147 | 148 | 221 | 222 |
| | 228 | 238 | 239 | 240 | 246 | 255 | 256 | 257 | 258 | 273 | 274 |
| | 275 | 291 | 292 | | | | | | | | |

For the more preferred amphoter I systems further comprising neutral lipids or mixtures of neutral lipids with dκ(pH 8) >0.16 the following rules apply:

1) combined head-group size is about 220+/-80 $A^3$, preferred +/- 40 $A^3$

2) combined tail regions size is about 700+/-150 $A^3$, preferred are small anion tails having molecular volumes of 200...350 $A^3$ and large cation tails having molecular volumes of 350...500 $A^3$

3) the mix has κ(salt) between 0.25 and 0.45 and a difference between κ(salt) and κ(total) at pH 8 >0.16

For amphoter II systems further comprising neutral lipids or mixtures of neutral lipids, in a first analysis a mixture of about 1/3 cationic amphiphile and 2/3 anionic amphiphile was used.

Besides the volume parameters, for the cationic amphiphile no ionisation was assumed for pH 8 and full ionisation was assumed for pH 4. For the anionic amphiphile, full dissociation at pH 8 and no dissociation at pH 4 was assumed. A library of 324 amphoter II lipid systems with an excess of the anionic amphiphile was constructed and preferred lipid systems having κ(salt) between 0.25 and 0.45 and a difference in κ(total) at pH 8 and κ(salt) called dκ (pH 8) of more than 0.12, preferably more than 0.16, are selected in the list below:

(The rest of this page is intentionally blank)

| Anion head | tail | Cation head | tail | k(salt) | System ID | Amphoter II 33 A k(pH 8) | 66 C k (pH4) | dk (pH8) | dk (pH 4) |
|---|---|---|---|---|---|---|---|---|---|
| 40 | 500 | 190 | 200 | 0,33 | 602 | 0,70 | 0,72 | 0,37 | 0,39 |
| 70 | 500 | 190 | 200 | 0,37 | 603 | 0,72 | 0,74 | 0,34 | 0,37 |
| 100 | 500 | 190 | 200 | 0,41 | 604 | 0,74 | 0,76 | 0,32 | 0,35 |
| 40 | 500 | 160 | 200 | 0,29 | 584 | 0,60 | 0,62 | 0,31 | 0,34 |
| 40 | 350 | 190 | 200 | 0,42 | 596 | 0,73 | 0,73 | 0,31 | 0,32 |
| 70 | 500 | 160 | 200 | 0,33 | 585 | 0,62 | 0,64 | 0,29 | 0,31 |
| 100 | 500 | 160 | 200 | 0,37 | 586 | 0,64 | 0,66 | 0,27 | 0,29 |
| 40 | 350 | 160 | 200 | 0,36 | 578 | 0,63 | 0,64 | 0,26 | 0,27 |
| 130 | 500 | 160 | 200 | 0,41 | 587 | 0,66 | 0,68 | 0,24 | 0,27 |

(continued)

| Anion head | tail | Cation head | tail | k(salt) | System ID | Amphoter II 33 A k(pH 8) | 66 C k (pH4) | dk (pH8) | dk (pH 4) |
|---|---|---|---|---|---|---|---|---|---|
| 70 | 350 | 160 | 200 | 0,42 | 579 | 0,66 | 0,66 | 0,24 | 0,25 |
| 70 | 500 | 130 | 200 | 0,29 | 567 | 0,52 | 0,54 | 0,23 | 0,26 |
| 40 | 350 | 130 | 200 | 0,31 | 560 | 0,53 | 0,54 | 0,22 | 0,23 |
| 100 | 500 | 130 | 200 | 0,33 | 568 | 0,54 | 0,56 | 0,21 | 0,24 |
| 70 | 350 | 130 | 200 | 0,36 | 561 | 0,56 | 0,56 | 0,19 | 0,20 |
| 130 | 500 | 130 | 200 | 0,37 | 569 | 0,56 | 0,58 | 0,19 | 0,21 |
| 40 | 200 | 130 | 200 | 0,43 | 554 | 0,60 | 0,56 | 0,18 | 0,14 |
| 40 | 350 | 100 | 200 | 0,25 | 542 | 0,43 | 0,44 | 0,17 | 0,18 |
| 100 | 350 | 130 | 200 | 0,42 | 562 | 0,58 | 0,59 | 0,17 | 0,17 |
| 160 | 500 | 130 | 200 | 0,41 | 570 | 0,58 | 0,60 | 0,16 | 0,19 |
| 40 | 500 | 190 | 350 | 0,27 | 710 | 0,43 | 0,42 | 0,16 | 0,15 |
| 40 | 200 | 100 | 200 | 0,35 | 536 | 0,50 | 0,47 | 0,15 | 0,12 |
| 100 | 500 | 100 | 200 | 0,29 | 550 | 0,44 | 0,47 | 0.15 | 0,18 |
| 70 | 350 | 100 | 200 | 0,31 | 543 | 0,46 | 0,47 | 0,15 | 0,16 |
| 190 | 200 | 40 | 500 | 0,33 | 721 | 0,47 | 0,39 | 0,14 | 0,07 |
| 190 | 200 | 70 | 500 | 0,37 | 739 | 0,51 | 0,43 | 0,14 | 0,06 |
| 70 | 500 | 190 | 350 | 0,31 | 711 | 0,45 | 0,44 | 0,14 | 0,14 |
| 190 | 200 | 100 | 500 | 0,41 | 757 | 0,55 | 0,47 | 0,14 | 0,06 |
| 160 | 200 | 40 | 500 | 0,29 | 720 | 0,42 | 0,34 | 0,14 | 0,06 |
| 160 | 200 | 70 | 500 | 0,33 | 738 | 0,46 | 0,38 | 0,14 | 0,06 |
| 160 | 200 | 100 | 500 | 0,37 | 756 | 0,50 | 0,42 | 0,13 | 0,05 |
| 130 | 500 | 100 | 200 | 0,33 | 551 | 0,46 | 0,49 | 0,13 | 0,16 |
| 40 | 200 | 70 | 200 | 0,28 | 518 | 0,40 | 0,37 | 0,13 | 0,09 |
| 40 | 350 | 190 | 350 | 0,33 | 704 | 0,46 | 0,44 | 0,13 | 0,11 |
| 160 | 200 | 130 | 500 | 0,41 | 774 | 0,54 | 0,46 | 0,13 | 0,05 |
| 130 | 200 | 70 | 500 | 0,29 | 737 | 0,41 | 0,33 | 0,13 | 0,05 |
| 70 | 200 | 100 | 200 | 0,43 | 537 | 0,55 | 0,51 | 0,13 | 0,09 |
| 100 | 500 | 190 | 350 | 0,34 | 712 | 0,47 | 0,46 | 0,13 | 0,12 |
| 130 | 200 | 100 | 500 | 0,33 | 755 | 0,45 | 0,37 | 0,13 | 0,05 |
| 100 | 350 | 100 | 200 | 0,36 | 544 | 0,49 | 0,49 | 0,12 | 0,13 |
| 130 | 200 | 130 | 500 | 0,37 | 773 | 0,49 | 0,41 | 0,12 | 0,04 |
| 70 | 500 | 160 | 350 | 0,27 | 693 | 0,39 | 0,39 | 0,12 | 0,12 |

List of preferred amphoter II lipid systems, further comprising neutral lipids or lipid mixtures, having an excess of anionic amphiphile ranked by dκ(pH 8).

The most preferred amphoter II lipid systems, further comprising neutral lipids or lipid mixtures, having an excess of anionic amphiphile are systems with following ID numbers:

| | | | | | | | | | | | |
|---|---|---|---|---|---|---|---|---|---|---|---|
| 542 | 554 | 560 | 561 | 562 | 567 | 568 | 569 | 570 | 578 | 579 | 584 |
| | 585 | 586 | 587 | 596 | 602 | 603 | 604 | 710 | | | |

For the more preferred amphoter II systems comprising neutral lipids or mixtures of neutral lipids with excess of anionic amphiphile and with dκ(pH 8) >0.16 the following rules apply:

1) combined head-group size is about 230+/-80 $A^3$, preferred +/- 40 $A^3$

2) combined tail regions size is about 700+/-150 $A^3$, preferred are small anion tails having molecular volumes of 200...350 $A^3$ and large cation tails having molecular volumes of 350...500 $A^3$

3) the mix has κ(salt) is between 0.25 and 0.45 and a difference between κ(salt) and κ(total) at pH 8 >0.16.

Surprisingly, the preferred amphoter I and amphoter II systems with an excess of anionic amphiphile follow common selection rules in the presence of sodium chloride.

For amphoter II systems further comprising neutral lipids or lipid mixtures, in a another analysis a mixture of about 2/3 cationic amphiphile and 1/3 anionic amphiphile was used.

Besides the volume parameters, for the cationic amphiphile no ionisation was assumed for pH 8 and full ionisation was assumed for pH 4. For the anionic amphiphile, full dissociation at pH 8 and no dissociation at pH 4 was assumed. A library of 324 amphoter II lipid systems with an excess of the cationic amphiphile was constructed and preferred lipid systems having κ(salt) between 0.25 and 0.45 and a difference in k(total) at pH 8 and κ(salt) called dκ (pH 8) of more than 0.12, preferably > 0.16, are selected in the list below:

| | | | | | Amphoter II | | | | |
|---|---|---|---|---|---|---|---|---|---|
| Anion head | tail | Cation head | tail | k(salt) | | 33A k(pH 8) | 66C k (pH4) | dk (pH8) | dk (pH 4) |
| 190 | 200 | 40 | 500 | 0,33 | 1721 | 0,87 | 0.67 | 0,54 | 0,34 |
| 190 | 200 | 70 | 500 | 0,37 | 1739 | 0,89 | 0,69 | 0,52 | 0,32 |
| 190 | 200 | 100 | 500 | 0,41 | 1757 | 0,91 | 0,71 | 0,49 | 0,29 |
| 160 | 200 | 40 | 500 | 0,29 | 1720 | 0,77 | 0,57 | 0,48 | 0,28 |
| 190 | 200 | 40 | 350 | 0,42 | 1613 | 0,88 | 0,68 | 0,46 | 0,27 |
| 160 | 200 | 70 | 500 | 0,33 | 1738 | 0,79 | 0,59 | 0,46 | 0,26 |
| 160 | 200 | 100 | 500 | 0,37 | 1756 | 0,81 | 0,61 | 0,44 | 0,24 |
| 160 | 200 | 40 | 350 | 0,36 | 1612 | 0,78 | 0,59 | 0,42 | 0,22 |
| 160 | 200 | 130 | 500 | 0,41 | 1774 | 0,83 | 0,63 | 0,41 | 0,21 |
| 130 | 200 | 70 | 500 | 0,29 | 1737 | 0,69 | 0,49 | 0,40 | 0,20 |
| 160 | 200 | 70 | 350 | 0,42 | 1630 | 0,81 | 0,61 | 0,39 | 0,20 |
| 130 | 200 | 100 | 500 | 0,33 | 1755 | 0,71 | 0,51 | 0,38 | 0,18 |
| 130 | 200 | 40 | 350 | 0,31 | 1611 | 0,68 | 0,49 | 0,37 | 0,18 |
| 130 | 200 | 130 | 500 | 0,37 | 1773 | 0,73 | 0,53 | 0,36 | 0,16 |
| 130 | 200 | 70 | 350 | 0,36 | 1629 | 0,71 | 0,51 | 0,35 | 0,15 |
| 130 | 200 | 160 | 500 | 0,41 | 1791 | 0,75 | 0,55 | 0,33 | 0,13 |
| 100 | 200 | 40 | 350 | 0,25 | 1610 | 0,58 | 0,39 | 0,33 | 0,13 |
| 100 | 200 | 100 | 500 | 0,29 | 1754 | 0,61 | 0,41 | 0,32 | 0,12 |
| 130 | 200 | 100 | 350 | 0,42 | 1647 | 0,74 | 0,54 | 0,32 | 0,12 |
| 100 | 200 | 130 | 500 | 0,33 | 1772 | 0,63 | 0,43 | 0,30 | 0,10 |
| 100 | 200 | 70 | 350 | 0,31 | 1628 | 0,61 | 0,42 | 0,30 | 0,11 |
| 130 | 200 | 40 | 200 | 0,43 | 1503 | 0,71 | 0,53 | 0,28 | 0,10 |
| 100 | 200 | 160 | 500 | 0,37 | 1790 | 0,65 | 0,45 | 0,28 | 0,08 |
| 100 | 200 | 100 | 350 | 0,36 | 1646 | 0,64 | 0,44 | 0,28 | 0,08 |
| 100 | 200 | 40 | 200 | 0,35 | 1502 | 0,61 | 0,43 | 0,26 | 0,08 |
| 70 | 200 | 70 | 350 | 0,25 | 1627 | 0,51 | 0,32 | 0,26 | 0,06 |
| 100 | 200 | 190 | 500 | 0,41 | 1808 | 0,67 | 0,47 | 0,26 | 0,05 |
| 100 | 200 | 130 | 350 | 0,42 | 1664 | 0,67 | 0,47 | 0,25 | 0,05 |
| 70 | 200 | 130 | 500 | 0,29 | 1771 | 0,53 | 0,33 | 0,25 | 0,04 |
| 190 | 350 | 40 | 500 | 0,27 | 1727 | 0,51 | 0,40 | 0,24 | 0,13 |
| 70 | 200 | 40 | 200 | 0,28 | 1501 | 0,51 | 0,33 | 0,24 | 0,06 |
| 100 | 200 | 70 | 200 | 0,43 | 1520 | 0,66 | 0,48 | 0,24 | 0,06 |
| 70 | 200 | 100 | 350 | 0,31 | 1645 | 0,54 | 0,35 | 0,23 | 0,04 |
| 70 | 200 | 160 | 500 | 0,33 | 1789 | 0,55 | 0,35 | 0,22 | 0,02 |
| 190 | 350 | 70 | 500 | 0,31 | 1745 | 0,53 | 0,42 | 0,22 | 0,11 |
| 70 | 200 | 70 | 200 | 0,35 | 1519 | 0,56 | 0,38 | 0,21 | 0,03 |
| 190 | 350 | 100 | 500 | 0,34 | 1763 | 0,55 | 0,44 | 0,21 | 0,10 |
| 70 | 200 | 130 | 350 | 0,36 | 1663 | 0,57 | 0,37 | 0,20 | 0,01 |
| 160 | 350 | 70 | 500 | 0,27 | 1744 | 0,47 | 0,36 | 0,20 | 0,09 |

(continued)

| Anion head | tail | Cation head | tail | k(salt) | Amphoter II | 33A k(pH 8) | 66C k (pH4) | dk (pH8) | dk (pH 4) |
|---|---|---|---|---|---|---|---|---|---|
| 70 | 200 | 190 | 500 | 0,37 | 1807 | 0,57 | 0,37 | 0,20 | 0,00 |
| 190 | 350 | 130 | 500 | 0,38 | 1781 | 0,57 | 0,46 | 0,19 | 0,08 |
| 190 | 350 | 40 | 350 | 0,33 | 1619 | 0,52 | 0,42 | 0.19 | 0,09 |
| 40 | 200 | 70 | 200 | 0,28 | 1518 | 0,46 | 0,28 | 0.19 | 0,01 |
| 40 | 200 | 100 | 350 | 0,25 | 1644 | 0,44 | 0,25 | 0,19 | -0,01 |
| 70 | 200 | 100 | 200 | 0,43 | 1537 | 0,61 | 0,43 | 0,19 | 0,01 |
| 160 | 350 | 100 | 500 | 0,31 | 1762 | 0,49 | 0,38 | 0,18 | 0,08 |
| 70 | 200 | 160 | 350 | 0,42 | 1681 | 0,60 | 0,40 | 0,18 | -0,02 |
| 160 | 350 | 40 | 350 | 0,29 | 1618 | 0,46 | 0,36 | 0,18 | 0,07 |
| 190 | 350 | 70 | 350 | 0,37 | 1637 | 0,55 | 0,44 | 0,18 | 0,07 |
| 190 | 350 | 160 | 500 | 0,41 | 1799 | 0,59 | 0,48 | 0,17 | 0,07 |
| 160 | 350 | 130 | 500 | 0,34 | 1780 | 0,51 | 0,40 | 0,17 | 0,06 |
| 40 | 200 | 160 | 500 | 0,29 | 1788 | 0,45 | 0,25 | 0,17 | -0,03 |
| 130 | 350 | 100 | 500 | 0,27 | 1761 | 0,43 | 0,33 | 0,16 | 0,05 |
| 160 | 350 | 70 | 350 | 0,33 | 1636 | 0,49 | 0,39 | 0,16 | 0,06 |
| 40 | 200 | 100 | 200 | 0,35 | 1536 | 0,51 | 0,33 | 0,16 | -0,02 |
| 190 | 350 | 100 | 350 | 0,41 | 1655 | 0,58 | 0,47 | 0,16 | 0,06 |
| 40 | 200 | 130 | 350 | 0,31 | 1662 | 0,47 | 0,27 | 0,16 | -0,03 |
| 190 | 350 | 190 | 500 | 0,45 | 1817 | 0,61 | 0,50 | 0,16 | 0,05 |
| 160 | 350 | 160 | 500 | 0,38 | 1798 | 0,53 | 0,42 | 0,15 | 0,04 |
| 130 | 350 | 70 | 350 | 0,29 | 1635 | 0,43 | 0,33 | 0,15 | 0,05 |
| 130 | 350 | 130 | 500 | 0,31 | 1779 | 0,45 | 0,34 | 0,15 | 0,04 |
| 160 | 350 | 100 | 350 | 0,37 | 1654 | 0,52 | 0,42 | 0,15 | 0,04 |
| 40 | 200 | 190 | 500 | 0,33 | 1806 | 0,47 | 0,27 | 0,14 | -0,06 |
| 160 | 350 | 190 | 500 | 0,41 | 1816 | 0,55 | 0,44 | 0,14 | 0,03 |
| 40 | 200 | 130 | 200 | 0,43 | 1554 | 0,56 | 0,38 | 0,14 | -0,04 |
| 40 | 200 | 160 | 350 | 0,36 | 1680 | 0,50 | 0,30 | 0,13 | -0,06 |
| 130 | 350 | 100 | 350 | 0,33 | 1653 | 0,46 | 0,36 | 0,13 | 0,03 |
| 160 | 350 | 130 | 350 | 0,41 | 1672 | 0,55 | 0,44 | 0,13 | 0,03 |
| 130 | 350 | 160 | 500 | 0,34 | 1797 | 0,47 | 0,36 | 0,13 | 0,02 |
| 190 | 350 | 40 | 200 | 0,42 | 1511 | 0,55 | 0,46 | 0,13 | 0,04 |
| 160 | 350 | 40 | 200 | 0,36 | 1510 | 0,49 | 0,40 | 0,13 | 0,04 |

List of preferred amphoter II lipid systems having an excess of cationic amphiphile ranked by dκ(pH 8), further comprising neutral lipids or neutral lipid mixtures.

The most preferred amphoter II lipid systems, further comprising neutral lipids or lipid mixtures, having an excess of cationic amphiphile are systems with following ID numbers:

| | | | | | | | | | | | |
|---|---|---|---|---|---|---|---|---|---|---|---|
| 1501 | 1502 | 1503 | 1518 | 1519 | 1520 | 1536 | 1537 | 1610 | 1611 | 1612 | 1613 |
| | 1618 | 1619 | 1627 | 1628 | 1629 | 1630 | 1636 | 1637 | 1644 | 1645 | 1646 |
| | 1647 | 1655 | 1662 | 1663 | 1664 | 1681 | 1720 | 1721 | 1727 | 1737 | 1738 |
| | 1739 | 1744 | 1745 | 1754 | 1755 | 1756 | 1757 | 1761 | 1762 | 1763 | 1771 |
| | 1772 | 1773 | 1774 | 1780 | 1781 | 1788 | 1789 | 1790 | 1791 | 1799 | 1807 |
| | 1808 | 1817 | | | | | | | | | |

For the more preferred amphoter II systems with excess of the cationic amphiphile and with dκ(pH 8) >0.16, further comprising neutral lipids or lipid mixtures the following rules apply:

1) combined head-group size is about 220+/-80 $A^3$, preferred +/- 50 $A^3$

2) combined tail regions size is about 700+/-150 A3,

3) the mix has κ(salt) between 0.25 and 0.45 and a difference between κ(salt) and κ(total) at pH 8 >0.16

For amphoter III systems further comprising neutral lipids or mixtures of neutral lipids, a mixture of about 2/3 cationic amphiphile and 1/3 anionic amphiphile was used for the analysis.

Besides the volume parameters, for the cationic amphiphile no ionisation was assumed for pH 8 and full ionisation was assumed for pH 4. For the anionic amphiphile, full dissociation between pH 4 and pH 8 was assumed. A library of 324 amphoter III lipid systems with an excess of the cationic amphiphile was constructed and preferred lipid systems having κ(salt) between 0.25 and 0.45 and a difference in κ(total) at pH 8 and κ(salt) called dκ(pH 8) of more than 0.12, preferably >0.16, are selected in the list below.

| Anion head | tail | Cation head | tail | k(salt) | System ID | Amphoter III 33 A k(pH 8) | 66C k (pH4) | dk (pH8) | dk (pH 4) |
|---|---|---|---|---|---|---|---|---|---|
| 40 | 500 | 190 | 200 | 0,33 | 2102 | 0,70 | 0,69 | 0,37 | 0,36 |
| 70 | 500 | 190 | 200 | 0,37 | 2103 | 0,72 | 0,71 | 0,34 | 0,34 |
| 100 | 500 | 190 | 200 | 0,41 | 2104 | 0,74 | 0,73 | 0,32 | 0,31 |
| 40 | 500 | 160 | 200 | 0,29 | 2084 | 0,60 | 0,59 | 0,31 | 0,30 |
| 40 | 350 | 190 | 200 | 0,42 | 2096 | 0,73 | 0,70 | 0,31 | 0,28 |
| 70 | 500 | 160 | 200 | 0,33 | 2085 | 0,62 | 0,61 | 0,29 | 0,28 |
| 100 | 500 | 160 | 200 | 0,37 | 2086 | 0,64 | 0,63 | 0,27 | 0,26 |
| 40 | 350 | 160 | 200 | 0,36 | 2078 | 0,63 | 0,60 | 0,26 | 0,24 |
| 130 | 500 | 160 | 200 | 0,41 | 2087 | 0,66 | 0,65 | 0,24 | 0,23 |
| 70 | 350 | 160 | 200 | 0,42 | 2079 | 0,66 | 0,63 | 0,24 | 0,21 |
| 70 | 500 | 130 | 200 | 0,29 | 2067 | 0,52 | 0,51 | 0,23 | 0,22 |
| 40 | 350 | 130 | 200 | 0,31 | 2060 | 0,53 | 0,50 | 0,22 | 0,19 |
| 100 | 500 | 130 | 200 | 0,33 | 2068 | 0,54 | 0,53 | 0,21 | 0,20 |
| 70 | 350 | 130 | 200 | 0,36 | 2061 | 0,56 | 0,53 | 0,19 | 0,17 |
| 130 | 500 | 130 | 200 | 0,37 | 2069 | 0,56 | 0,55 | 0,19 | 0,18 |
| 40 | 200 | 130 | 200 | 0,43 | 2054 | 0,60 | 0,53 | 0,18 | 0,10 |
| 40 | 350 | 100 | 200 | 0,25 | 2042 | 0,43 | 0,40 | 0,17 | 0,15 |
| 100 | 350 | 130 | 200 | 0,42 | 2062 | 0,58 | 0,56 | 0,17 | 0,14 |
| 160 | 500 | 130 | 200 | 0,41 | 2070 | 0,58 | 0,57 | 0,16 | 0,15 |
| 40 | 500 | 190 | 350 | 0,27 | 2210 | 0,43 | 0,40 | 0,16 | 0,13 |
| 40 | 200 | 100 | 200 | 0,35 | 2036 | 0,50 | 0,43 | 0,15 | 0,08 |
| 100 | 500 | 100 | 200 | 0,29 | 2050 | 0,44 | 0,43 | 0,15 | 0,14 |
| 70 | 350 | 100 | 200 | 0,31 | 2043 | 0,46 | 0,43 | 0,15 | 0,12 |
| 190 | 200 | 40 | 500 | 0,33 | 2221 | 0,47 | 0,38 | 0,14 | 0,05 |
| 190 | 200 | 70 | 500 | 0,37 | 2239 | 0,51 | 0,42 | 0,14 | 0,05 |
| 70 | 500 | 190 | 350 | 0,31 | 2211 | 0,45 | 0,42 | 0,14 | 0,12 |
| 190 | 200 | 100 | 500 | 0,41 | 2257 | 0,55 | 0,46 | 0,14 | 0,05 |
| 160 | 200 | 40 | 500 | 0,29 | 2220 | 0,42 | 0,33 | 0,14 | 0,04 |
| 160 | 200 | 70 | 500 | 0,33 | 2238 | 0,46 | 0,37 | 0,14 | 0,04 |
| 160 | 200 | 100 | 500 | 0,37 | 2256 | 0,50 | 0,41 | 0,13 | 0,04 |
| 130 | 500 | 100 | 200 | 0,33 | 2051 | 0,46 | 0,45 | 0,13 | 0,12 |
| 40 | 200 | 70 | 200 | 0,28 | 2018 | 0,40 | 0,33 | 0,13 | 0,06 |
| 40 | 350 | 190 | 350 | 0,33 | 2204 | 0,46 | 0,42 | 0,13 | 0,09 |
| 160 | 200 | 130 | 500 | 0,41 | 2274 | 0,54 | 0,45 | 0,13 | 0,04 |
| 130 | 200 | 70 | 500 | 0,29 | 2237 | 0,41 | 0,32 | 0,13 | 0,04 |
| 70 | 200 | 100 | 200 | 0,43 | 2037 | 0,55 | 0,48 | 0,13 | 0,06 |
| 100 | 500 | 190 | 350 | 0,34 | 2212 | 0,47 | 0,44 | 0,13 | 0,10 |
| 130 | 200 | 100 | 500 | 0,33 | 2255 | 0,45 | 0,36 | 0,13 | 0,03 |

(continued)

| Anion head | tail | Cation head | tail | k(salt) | System ID | Amphoter III 33 A k(pH 8) | 66C k (pH4) | dk (pH8) | dk (pH 4) |
|---|---|---|---|---|---|---|---|---|---|
| 100 | 350 | 100 | 200 | 0,36 | 2044 | 0,49 | 0,46 | 0,12 | 0,10 |
| 130 | 200 | 130 | 500 | 0,37 | 2273 | 0,49 | 0,40 | 0,12 | 0,03 |
| 70 | 500 | 160 | 350 | 0,27 | 2193 | 0,39 | 0,37 | 0,12 | 0,10 |

List of preferred amphoter III lipid systems further comprising neutral lipids or lipid mixtures having an excess of cationic amphiphile ranked by dκ(pH 8).

The most preferred amphoter III lipid systems, further comprising neutral lipids or lipid mixtures, having an excess of cationic amphiphile are systems with following ID numbers:

| | | | | | | | | | | | |
|---|---|---|---|---|---|---|---|---|---|---|---|
| 2042 | 2054 | 2060 | 2061 | 2062 | 2067 | 2068 | 2069 | 2070 | 2078 | 2079 | 2084 |
| | 2085 | 2086 | 2087 | 2096 | 2102 | 2103 | 2104 | 2210 | | | |

For the more preferred amphoter III systems with excess of cationic amphiphile and with dκ(pH 8) >0.16 the following rules apply:

1) combined head-group size is about 230+/-80 $A^3$, preferred +/- 40 $A^3$

2) combined tail regions size is about 700+/-150 $A^3$,

3) the mix has κ(salt) between 0.25 and 0.45 and a difference between κ(salt) and κ(total) at pH 8 >0.16
Interestingly, both systems with an excess of the cationic amphiphiles are commanded by common rules in the presence of sodium chloride as counterions.

Also, amphoter II systems in a mixture of about equal molar amounts of cationic amphiphile and anionic amphiphile were analysed for combination with neutral lipids or mixtures of neutral lipids.

Besides the volume parameters, for the cationic amphiphile no ionisation was assumed for pH 8 and full ionisation was assumed for pH 4. For the anionic amphiphile, full dissociation at pH 8 and no dissociation at pH 4 was assumed. A library of 324 amphoter II lipid systems with about equal amounts of the cationic and anionic amphiphile was constructed and preferred lipid systems having κ(salt) between 0.25 and 0.45 and a difference in κ(total) at pH 8 and κ(salt) called dκ(pH 8) of more than 0.12 or preferred 0.16 are selected in the list below:

(The rest of this page is intentionally blank)

| Anion head | tail | Cation head | tail | k(salt) | System ID | Amphoter II 50 A k(pH 8) | 50 C k (pH4) | dk (pH8) | dk (pH 4) |
|---|---|---|---|---|---|---|---|---|---|
| 190 | 200 | 40 | 500 | 0,33 | 1221 | 0,68 | 0,54 | 0,35 | 0,21 |
| 190 | 200 | 70 | 500 | 0,37 | 1239 | 0,71 | 0,57 | 0,34 | 0,19 |
| 190 | 200 | 100 | 500 | 0,41 | 1257 | 0,74 | 0,60 | 0,32 | 0,18 |
| 160 | 200 | 40 | 500 | 0,29 | 1220 | 0,60 | 0,46 | 0,32 | 0,18 |
| 160 | 200 | 70 | 500 | 0,33 | 1238 | 0,63 | 0,49 | 0,30 | 0,16 |
| 160 | 200 | 100 | 500 | 0,37 | 1256 | 0,66 | 0,52 | 0,29 | 0,15 |
| 160 | 200 | 130 | 500 | 0,41 | 1274 | 0,69 | 0,55 | 0,28 | 0,14 |
| 190 | 200 | 40 | 350 | 0,42 | 1113 | 0,69 | 0,56 | 0,28 | 0,14 |
| 130 | 200 | 70 | 500 | 0,29 | 1237 | 0,56 | 0,42 | 0,27 | 0,13 |
| 130 | 200 | 100 | 500 | 0,33 | 1255 | 0,59 | 0,45 | 0,26 | 0,12 |
| 160 | 200 | 40 | 350 | 0,36 | 1112 | 0,62 | 0,49 | 0,26 | 0,12 |
| 40 | 500 | 190 | 200 | 0,33 | 1102 | 0,58 | 0,57 | 0,25 | 0,24 |
| 130 | 200 | 130 | 500 | 0,37 | 1273 | 0,62 | 0,48 | 0,25 | 0,10 |
| 160 | 200 | 70 | 350 | 0,42 | 1130 | 0,66 | 0,53 | 0,24 | 0,11 |

(continued)

| Anion head | tail | Cation head | tail | k(salt) | System ID | Amphoter II 50 A k(pH 8) | 50 C k (pH4) | dk (pH8) | dk (pH 4) |
|---|---|---|---|---|---|---|---|---|---|
| 70 | 500 | 190 | 200 | 0,37 | 1103 | 0,61 | 0,60 | 0,24 | 0,23 |
| 130 | 200 | 40 | 350 | 0,31 | 1111 | 0,54 | 0,41 | 0,24 | 0,10 |
| 130 | 200 | 160 | 500 | 0,41 | 1291 | 0,65 | 0,51 | 0,23 | 0,09 |
| 100 | 200 | 100 | 500 | 0,29 | 1254 | 0,51 | 0,37 | 0,23 | 0,09 |
| 100 | 500 | 190 | 200 | 0,41 | 1104 | 0,64 | 0,63 | 0,23 | 0,21 |
| 130 | 200 | 70 | 350 | 0,36 | 1129 | 0,59 | 0,46 | 0,22 | 0,09 |
| 40 | 500 | 160 | 200 | 0,29 | 1084 | 0,51 | 0,49 | 0,22 | 0,21 |
| 100 | 200 | 40 | 350 | 0,25 | 1110 | 0,47 | 0,34 | 0,22 | 0,08 |
| 100 | 200 | 130 | 500 | 0,33 | 1272 | 0,54 | 0,40 | 0,21 | 0,07 |
| 130 | 200 | 100 | 350 | 0,42 | 1147 | 0,63 | 0,50 | 0,21 | 0,08 |
| 40 | 350 | 190 | 200 | 0,42 | 1096 | 0,63 | 0,58 | 0,21 | 0,17 |
| 70 | 500 | 160 | 200 | 0,33 | 1085 | 0,54 | 0,52 | 0,21 | 0,19 |
| 100 | 200 | 70 | 350 | 0,31 | 1128 | 0,51 | 0,38 | 0,20 | 0,07 |
| 100 | 200 | 160 | 500 | 0,37 | 1290 | 0,57 | 0,43 | 0,20 | 0,06 |
| 100 | 500 | 160 | 200 | 0,37 | 1086 | 0,57 | 0,55 | 0,19 | 0,18 |
| 100 | 200 | 100 | 350 | 0,36 | 1146 | 0,56 | 0,42 | 0,19 | 0,06 |
| 100 | 200 | 190 | 500 | 0,41 | 1308 | 0,60 | 0,46 | 0,19 | 0,05 |
| 40 | 350 | 160 | 200 | 0,36 | 1078 | 0,55 | 0,51 | 0,19 | 0,15 |
| 70 | 200 | 70 | 350 | 0,25 | 1127 | 0,44 | 0,31 | 0,18 | 0,05 |
| 70 | 200 | 130 | 500 | 0,29 | 1271 | 0,47 | 0,33 | 0,18 | 0,04 |
| 130 | 500 | 160 | 200 | 0,41 | 1087 | 0,60 | 0,58 | 0,18 | 0,17 |
| 100 | 200 | 130 | 350 | 0,42 | 1164 | 0,60 | 0,47 | 0,18 | 0,05 |
| 70 | 350 | 160 | 200 | 0,42 | 1079 | 0,59 | 0,55 | 0,17 | 0,13 |
| 70 | 500 | 130 | 200 | 0,29 | 1067 | 0,46 | 0,45 | 0,17 | 0,16 |
| 70 | 200 | 100 | 350 | 0,31 | 1145 | 0,48 | 0,35 | 0,17 | 0,04 |
| 70 | 200 | 160 | 500 | 0,33 | 1289 | 0,50 | 0,36 | 0,17 | 0,03 |
| 40 | 350 | 130 | 200 | 0,31 | 1060 | 0,48 | 0,43 | 0,17 | 0,13 |
| 100 | 200 | 40 | 200 | 0,35 | 1002 | 0,51 | 0,40 | 0,16 | 0,05 |
| 130 | 200 | 40 | 200 | 0,43 | 1003 | 0,59 | 0,48 | 0,16 | 0,05 |
| 70 | 200 | 70 | 200 | 0,35 | 1019 | 0,51 | 0,40 | 0,16 | 0,05 |
| 100 | 200 | 70 | 200 | 0,43 | 1020 | 0,59 | 0,48 | 0,16 | 0,05 |
| 40 | 200 | 100 | 200 | 0,35 | 1036 | 0,51 | 0,40 | 0,16 | 0,05 |
| 70 | 200 | 100 | 200 | 0,43 | 1037 | 0,59 | 0,48 | 0,16 | 0,05 |
| 40 | 200 | 130 | 200 | 0,43 | 1054 | 0,59 | 0,48 | 0,16 | 0,05 |
| 70 | 200 | 40 | 200 | 0,28 | 1001 | 0,44 | 0,33 | 0,16 | 0,05 |
| 40 | 200 | 70 | 200 | 0,28 | 1018 | 0,44 | 0,33 | 0,16 | 0,05 |
| 100 | 500 | 130 | 200 | 0,33 | 1068 | 0,49 | 0,48 | 0,16 | 0,15 |
| 70 | 200 | 130 | 350 | 0,36 | 1163 | 0,52 | 0,39 | 0,16 | 0,03 |
| 70 | 200 | 190 | 500 | 0,37 | 1307 | 0,53 | 0,39 | 0,16 | 0,01 |
| 70 | 350 | 130 | 200 | 0,36 | 1061 | 0,52 | 0,48 | 0,15 | 0,11 |
| 40 | 200 | 100 | 350 | 0,25 | 1144 | 0,41 | 0,27 | 0,15 | 0,02 |
| 130 | 500 | 130 | 200 | 0,37 | 1069 | 0,52 | 0,51 | 0,15 | 0,14 |
| 70 | 200 | 160 | 350 | 0,42 | 1181 | 0,57 | 0,43 | 0,15 | 0,02 |
| 40 | 350 | 100 | 200 | 0,25 | 1042 | 0,40 | 0,36 | 0,15 | 0,11 |
| 100 | 350 | 130 | 200 | 0,42 | 1062 | 0,56 | 0,52 | 0,14 | 0,10 |
| 40 | 200 | 130 | 350 | 0,31 | 1162 | 0,45 | 0,32 | 0,14 | 0,01 |
| 40 | 200 | 160 | 500 | 0,29 | 1288 | 0,42 | 0,28 | 0,14 | 0,00 |

(continued)

| Anion head | tail | Cation head | tail | k(salt) | System ID | Amphoter II 50 A k(pH 8) | 50 C k (pH4) | dk (pH8) | dk (pH 4) |
|---|---|---|---|---|---|---|---|---|---|
| 160 | 500 | 130 | 200 | 0,41 | 1070 | 0,55 | 0,54 | 0,14 | 0,12 |
| 70 | 350 | 100 | 200 | 0,31 | 1043 | 0,44 | 0,40 | 0,13 | 0,09 |
| 190 | 350 | 40 | 500 | 0,27 | 1227 | 0,40 | 0,33 | 0,13 | 0,06 |
| 100 | 500 | 100 | 200 | 0,29 | 1050 | 0,42 | 0,40 | 0,13 | 0,12 |
| 190 | 350 | 70 | 500 | 0,31 | 1245 | 0,43 | 0,36 | 0,13 | 0,06 |
| 40 | 200 | 160 | 350 | 0,36 | 1180 | 0,49 | 0,36 | 0,13 | -0,01 |
| 40 | 200 | 190 | 500 | 0,33 | 1306 | 0,45 | 0,31 | 0,12 | -0,02 |
| 190 | 350 | 100 | 500 | 0,34 | 1263 | 0,46 | 0,39 | 0,12 | 0,05 |
| 100 | 350 | 100 | 200 | 0,36 | 1044 | 0,49 | 0,45 | 0,12 | 0,08 |
| 160 | 350 | 70 | 500 | 0,27 | 1244 | 0,39 | 0,32 | 0,12 | 0,05 |

List of preferred amphoter II lipid systems having an equal amount of cationic and anionic amphiphile further being suitable for combination with neutral lipids or mixtures of neutral lipids, ranked by dκ(pH 8).

The most preferred amphoter II lipid systems, further comprising neutral lipids or lipid mixtures, having an equal amount of cationic and anionic amphiphile are systems with following ID numbers:

| | | | | | | | | | | | |
|---|---|---|---|---|---|---|---|---|---|---|---|
| 1001 | 1002 | 1003 | 1018 | 1019 | 1020 | 1036 | 1037 | 1054 | 1060 | 1067 | 1068 |
| | 1078 | 1079 | 1084 | 1085 | 1086 | 1087 | 1096 | 1102 | 1103 | 1104 | 1110 |
| | 1111 | 1112 | 1113 | 1127 | 1128 | 1129 | 1130 | 1145 | 1146 | 1147 | 1163 |
| | 1164 | 1220 | 1221 | 1237 | 1238 | 1239 | 1254 | 1255 | 1256 | 1257 | 1271 |
| | 1272 | 1273 | 1274 | 1289 | 1290 | 1291 | 1307 | 1308 | | | |

For the more preferred amphoter II systems with equal amounts of cationic and anionic amphiphile, neutral lipids or mixtures thereof and with dκ(pH 8) >0.16 the following rules apply:

1) combined head-group size is about 210+/-80 $A^3$, preferred +/- 50 $A^3$

2) combined tail regions size is about 700+/-150 $A^3$,

3) the mix has κ(salt) between 0.25 and 0.45 and a difference between κ(salt) and κ(total) at pH 8 >0,16.

The methods disclosed herein substantially reduce the number of variables involved with the optimisation of the system, thus creating important economic benefit.

The present invention has been exemplified with various calculations in the detailed description. Further experimental work is described in the following examples. Examples are given with the understanding of further detailing certain aspects of practising the current invention. Examples by no means should limit the scope of this disclosure.

**Example 1 - Preparation of liposomes and pH -dependent fusion experiment**

*Buffer system*

100 mM sodium citrate and 200 mM sodium hydrogen phosphate were prepared as stock solutions and variable amounts of both solutions were mixed to adjust for the pH needed. CiP 7.0 as an example specifies a buffer from that series having a pH of 7.0 and is made from citrate and phosphate.

*Liposome production*

Liposomes were formed from a dried lipid film. In brief, 20 μmol of the respective lipid composition was dissolved in 1mL chloroform/methanol 3:1 and dried in vacuum using a rotary evaporator. The resulting film was hydrated for 45 min in 1 mL of CiP 8.0 with gentle agitation. The resulting liposome suspension was frozen, sonicated after thawing and

eventually extruded through 200 nm polycarbonate filters.

*pH -jump experiment*

10 μl liposomes in CiP 8.0 were placed into a glass tube and mixed rapidly with 1 mL of CiP buffer of the pH needed. Samples were allowed to stand for 1 h at room temperature and 3 mL of 200 mM sodium hydrogen phosphate were rapidly mixed with the sample. Liposomes were analyzed for size using a MALVERN Zetasizer 3000HS and sizes were recorded as Z-average.

**Example 2 - Fusion of amphoter I lipid mixtures**

Liposomes were prepared from DOTAP and CHEMS in sodium citrate/ sodium phosphate pH 8.0 (CiP 8.0) and small amounts were injected into a CiP buffer with a lower pH (see Example 1 for details). Any larger structures observed at the lower pH might be either due to aggregate formations and generation of multicentric honeycomb structures or such structures might result from genuine fusion. To separate between these two outcomes we readjusted the pH to neutrality using 200mM sodium hydrogen phosphate. Electrostatic repulsion dissociates multicentric vesicles but not fusion products. The results are illustrated in Fig. 10.

As predicted in the mathematical salt bridge model, a valley of instability exists at slightly acidic conditions and fusion to larger particles was observed starting from pH 6.5. However, fast addition of the liposomes into low pH resulted in stabilisation of the particles as long as some DOTAP was present in the mixture. Liposomes from 100 mol.% CHEMS enter a fusogenic state below pH 4.5 and do not get stabilised at the lower pH.

Noteworthy, a 1:1 mixture of DOTAP/CHEMS cannot form liposomes in CiP 8.0 which is in good agreement with the mathematical model that predicts a non-lamellar phase for these parameters.

**Example 3 - fusion of amphoter II systems**

Liposomes were prepared from MoChol and CHEMS in sodium citrate/ sodium phosphate pH 8.0 (CiP 8.0) and small amounts were injected into a CiP buffer with a lower pH (see Example 1 for details). Any larger structures observed at the lower pH might be either due to aggregate formations and generation of multicentric honeycomb structures or such structures might result from genuine fusion. To separate between these two outcomes we readjusted the pH to neutrality using 200 mM sodium hydrogen phosphate. Electrostatic repulsion dissociates multicentric vesicles but not fusion products.

Experimental evidence supports the salt bridge model. (See Fig. 11). The fusion zone is inclined towards high anion content due to the large head-group size of MoCHol Consequently, no fusion occurs with 33 mol.% or 50 mol.% CHEMS in the mixture, whereas mixtures containing 66 mol.% or 75 mol.% CHEMS undergo fusion when exposed to a pH between 4 and 6. As predicted, the onset of fusion is shifted to lower pH values with higher amounts of CHEMS. Again, 100 mol.% CHEMS is fusogenic with low pH but has no stable state at low pH.

The parameters used for the calculation are given below; CHEMS and MoChol in Na/ $H_2PO_4$ were used as model compounds; all volumes in $Å^3$.

| | |
|---|---|
| Anion head volume | 76 |
| Anion tail volume | 334 |
| Anion pK | 5.8 |
| Cation head volume | 166 |
| Cation tail volume | 371 |
| Cation pK | 6.5 |
| Counterion+ volume | 65 |
| Counterion- volume | 49 |

**Example 4 - fusion in amphoter III systems with steric hindrance**

Liposomes were prepared from POPG and MoChol in sodium citrate/ sodium phosphate pH 8.0 (CiP 8.0) and small amounts were injected into a CiP buffer with a lower pH (see Example 1 for details). Any larger structures observed at the lower pH might be either due to aggregate formations and generation of multicentric honeycomb structures or

such structures might result from genuine fusion. To separate between these two outcomes we readjusted the pH to neutrality using 200mM sodium hydrogen phosphate. Electrostatic repulsion dissociates multicentric vesicles but not fusion products.

Experimental evidence supports only a situation where no salt bridge formation occurs. A mixture between MoChol and POPG does not undergo fusion in the pH -jump experiment (data not shown). This is quite possibly due to steric hindrance, as the protonated nitrogen in Mo-Chol is situated at the lower end of the morpholino ring and is therefore not easily accessible. In addition, the phosphate in POPG sits right at the lipid/water interface and is protected with a glycerol towards the water phase.

## Example 5 - fusion in amphoter III systems, no steric hindrance

Amphoter III systems from POPG / MoChol do not undergo fusion (see Example 4 above). It was therefore questioned whether the removal of the protecting glycerol and exchange of POPG with DOPA would avoid such steric hindrance. In fact, such system undergoes fusion, as illustrated in the Fig. 12.

Details as per Example 1 above. The parameters used for the calculation are given below; DOPA and MoChol in Na/ $H_2PO_4$ were used as model compounds, and volumes are expressed as $Å^3$.

| | |
|---|---|
| Anion head volume | 63 |
| Anion tail volume | 501 |
| Anion pK | 3 |
| Cation head volume | 166 |
| Cation tail volume | 371 |
| Cation pK | 6.5 |
| Counterion+ volume | 65 |
| Counterion- volume | 49 |

The model calculation reflects the full complexity of the experimental fusion behaviour: no fusion for mixtures with less than 50% MoCHol, strong fusion for MoChol=DOPA and ongoing fusion with no stable phase under acidic conditions for mixtures with excess DOPA.

## Example 6 - lipid salt formation with monoalkyl lipids

Oleic acid was chosen as a known and popular pH -sensitive membrane component. As the lipid tail is relatively small in volume, any change in the head-group has more pronounced consequences for the membrane stability. As shown in Fig. 13, modelling predicts oleic acid to be a strong driver for fusion in an amphoter II system with MoChol. This is confirmed experimentally. Mixtures of oleic acid do form liposomes with Mo-Chol and particles rapidly undergo fusion when exposed to different conditions. As expected from the algorithm, the extent of fusion is limited for smaller amounts of OA in the mixture, but 50 mol.% of the anion results in the classic valley type fusion pattern. Since the fusion tendency is much stronger with OA, a bigger portion of that anion in the mix results in extensive fusion over a wide range of pH values. Still, mixtures can always be stabilised at low pH. Details as per Example 1.

| | |
|---|---|
| MoChol head-group volume | 166 |
| MoChol tail volume | 371 |
| MoChol pK | 6.5 |
| Oleic acid head volume | 42 |
| Oleic acid tail volume | 208 |
| Oleic acid pK | 4.5 |
| Counterion citrate volume | 121 |
| Counterion sodium volume | 65 |

**Example 7 - influence of neutral lipids**

DOTAP and CHEMS were chosen as an amphoter I charged lipid pair and POPC ($\kappa$ ~ 0,5) was added as a neutral lipid. As expected, the pure mixture of the charged components undergoes aggregation or fusion at and below the isoelectric point. However, as shown in Fig. 14, the addition of only 20 mol.% POPC ameliorated such aggregation tendency to a great extent for all cation: anion ratios ranging from pure anion through to the 1:1 mixture that is no longer amphoteric.

As shown in Fig. 15, the addition of the same amount of DOPE ($\kappa$~0.19) to the amphoter I mixture from DOTAP and CHEMS maintains the fusion behaviour independent of the ratio between DOTAP and CHEMS.

## Claims

1. An amphoteric liposome composed of a mixture of lipids, said mixture comprising a cationic amphiphile, an anionic amphiphile and optionally one or more neutral amphiphiles, at least one of said cationic and anionic amphiphiles being chargeable and the respective amounts of said cationic and anionic amphiphiles being selected such there is a stoichiometric excess of positively charged cationic amphiphile at a first lower pH, a stoichiometric excess of negatively charged anionic amphiphile at a second higher pH and said mixture has an isoelectric point intermediate said first and second pHs; **characterised in that** said positively charged cationic and negatively charged anionic amphiphiles are adapted to form a lipid salt with one another at said isoelectric point.

2. An amphoteric liposome as claimed in claim 1, wherein each of said anionic and cationic amphiphiles has respective polar head and apolar tail groups, the polar head and apolar tail groups of the anionic and cationic amphiphiles being selected such that $\kappa_{total}$(pH) for the mixture in the presence of predetermined cationic and anionic counterions for said anionic and cationic amphiphiles respectively exhibits a minimum at said isoelectric point, whereby said mixture exhibits stable lamellar phases at said first and second pH's and a fusogenic, hexagonal phase at said isoelectric point; $\kappa_{total}$(pH) being defined as:

$$\kappa_{total}(pH) = \kappa_{an} \cdot c_{an}(pH) + \kappa_{cat} \cdot c_{cat}(pH) + \kappa_{an^-} \cdot c_{an^-}(pH) + \kappa_{cat^+} \cdot c_{cat^+}(pH) + \kappa_{salt} \cdot c_{salt}(pH) + \sum \kappa_n \cdot c_n$$

wherein $c_{an}(pH)$, $c_{cat}(pH)$, $c_{an-}(pH)$, $c_{cat+}(pH)$ and $c_{salt}(pH)$ are the respective concentrations in the lipid mixture of the uncharged anionic, uncharged cationic, charged anionic and charged cationic amphiphiles and said lipid salt as a function of pH,
$c_n$ is the concentration in the lipid mixture of the or each optional neutral amphiphile, and
$\kappa_{an}$, $\kappa_{cat}$, $\kappa_{an-}$, $\kappa_{cat+}$, $\kappa_{salt}$ and $\kappa_n$ are the respective $\kappa$ values for the uncharged anionic, uncharged cationic, charged anionic and charged cationic amphiphiles, said lipid salt and the or each optional neutral amphiphile,
$\kappa$ being the ratio of the molecular volume of the polar head group $V_{head}$ to the molecular volume of the apolar tail group $V_{apolar}$ of the respective species, the molecular volumes of the polar head groups of the charged anionic and cationic amphiphiles including the respective predetermined counterions, and $\kappa_{salt}$ being defined as:

$$\kappa_{salt} = \frac{V_{head}(cat) + V_{head}(an)}{V_{apolar}(cat) + V_{apolar}(an)}$$

wherein $V_{head}(cat)$ is the molecular volume of the polar head group of the cationic amphiphile without the respective counter-anion, $V_{head}(an)$ is the molecular volume of the polar head group of the anionic amphiphile without the respective counter-cation, $V_{apolar}(cat)$ is the molecular volume of the apolar tail group of the cationic amphiphile and $V_{apolar}(an)$ is the molecular volume of the apolar tail group of the anionic amphiphile.

3. An amphoteric liposome as claimed in claim 1 or claim 2, wherein said mixture has an isoelectric point in the range

pH 4 to pH 8.

4. An amphoteric liposome as claimed in claim 1 or 2, wherein said mixture has an isoelectric point in the range pH 5 to pH 7.

5. An amphoteric liposome as claimed in any preceding claim, wherein said first pH is in the range pH 4 to pH 5, and said second pH is in the range pH 7 to pH 8.

6. An amphoteric liposome as claimed in any preceding claim, wherein said second pH is about 7.4.

7. An amphoteric liposome as claimed in claim 2, wherein said lipid salt has a $\kappa_{salt}$ value of less than about 0.35.

8. An amphoteric liposome as claimed in any preceding claim, wherein $V_{head}(cat) + V_{head}(an)$ is less than or equal to about 300 Å$^3$.

9. An amphoteric liposome as claimed in any preceding claim, wherein $V_{apolar}(cat) + V_{apolar}(an)$ is greater than or equal to about 600 Å$_3$.

10. An amphoteric liposome as claimed in claim 2 or claim 7, wherein said counter-cations have a molecular volume of at least 50 A$^3$.

11. An amphoteric liposome as claimed in claim 10, wherein said counter-cations are selected from sodium or tris (hydroxymethyl)aminomethane, tris-hydroxyethylaminomethane and triethylamine.

12. An amphoteric liposome as claimed in any of claim 11, wherein said counter-cations are sodium.

13. An amphoteric liposome as claimed in any of claims 2, 7 or 10 to 12, wherein said anionic amphiphile is chargeable and is present in molar excess of said cationic amphiphile, said counterions are sodium and chloride or phosphate, and:

(i) $V_{head}(cat) + V_{head}(an)$ is about 160+/-80 A$^3$ ;
(ii) $V_{apolar}(cat) + V_{apolar}(an)$ is about 700+/-150 A$^3$; and
(iii) $\kappa_{salt} < 0.3$, and the difference between $\kappa_{salt}$ and $\kappa_{total}$(pH) at pH 8 > 0.12.

14. An amphoteric liposome as claimed in any of claims 2, 7 or 10 to 12, wherein said cationic amphiphile is weak and is present in molar excess of said anionic amphiphile, said counterions are sodium and chloride or phosphate, and:

(i) $V_{head}(cat) + V_{head}(an)$ is about 160+/-80 A$^3$;
(ii) $V_{apolar}(cat) + V_{apolar}(an)$ is about 700+/-150 A$^3$; and
(iii) $\kappa_{salt} < 0.3$, and the difference between $\kappa_{salt}$ and $\kappa_{total}$(pH) at pH 8 > 0.12.

15. An amphoteric liposome as claimed in any of claims 2, 7 or 10 to 12, wherein said cationic and anionic amphiphiles are both weak and are present in substantially equal molar amounts to one another, the counterions are sodium and chloride or phosphate, and:

(i) $V_{head}(cat) + V_{head}(an)$ is about 160+/-80 A$^3$ ;
(ii) $V_{apolar}(cat) + V_{apolar}(an)$ is about 700+/-150 A$^3$; and
(iii) $\kappa_{salt} < 0.3$ and the difference between $\kappa_{salt}$ and $\kappa_{total}$(pH) at pH 8 > 0.12.

16. An amphoteric liposome as claimed in any preceding claim, wherein said cationic and anionic amphiphiles each carry a single charge.

17. An amphoteric liposome as claimed in any of claims 2, 7 or 10 to 15, wherein said mixture comprises up to about 65 mol.% of one or more neutral amphiphiles.

18. An amphoteric liposome as claimed in claim 17, wherein said one or more neutral amphiphiles comprise a neutral amphiphile having a $\kappa_n$ value greater than or equal to about 0.4.

**19.** An amphoteric liposome as claimed in claim 17 or claim 18, wherein said one or more neutral amphiphiles comprise a neutral amphiphile having a $\kappa_n$ value less than or equal to about 0.3.

**20.** An amphoteric liposome as claimed in claim 17, claim 18 or claim 19, wherein said anionic amphiphile is weak and is present in molar excess of said cationic amphiphile, the counterions are sodium and chloride or phosphate, and:

(i) $V_{head}(cat) + V_{head}(an)$ is about 220+/-80 $A^3$;
(ii) $V_{apolar}(cat) + V_{apolar}(an)$ is about 700+/-150 $A^3$; and
(iii) $\kappa_{salt}$ is in the range 025 to 0.45; and the difference between $V_{head}(cat) + V_{head}(an)$ and $\kappa_{total}$(pH) at pH 8 > 0.16.

**21.** An amphoteric liposome as claimed in claim 17, claim 18 or claim 19, wherein said cationic amphiphile is weak and is present in molar excess of said anionic amphiphile, the counterions are sodium and chloride or phosphate, and:

(i) $V_{head}(cat) + V_{head}(an)$ is about 220+/-80 $A^3$;
(ii) $V_{apolar}(cat) + V_{apolar}(an)$ is about 700+/-150 $A^3$; and
(iii) $\kappa_{salt}$ is in the range 0.25 to 0.45, and the difference between $\kappa_{salt}$ and $\kappa_{total}$(pH) at pH 8 > 0.16.

**22.** An amphoteric liposome as claimed in claim 17, claim 18 or claim 20, wherein said cationic and anionic amphiphiles are both weak and are present in substantially equal molar amounts to one another, the counterions are sodium and chloride or phosphate and:

(i) $V_{head}(cat) + V_{head}(an)$ is about 210+/-80 $A^3$;
(ii) $V_{apolar}(cat) + V_{apolar}(an)$ is about 700+/-150 $A^3$; and
(iii) $\kappa_{salt}$ is in the range 0.25 to 0.45, and the difference between $\kappa_{salt}$ and $\kappa_{total}$(pH) at pH 8 > 0.16.

**23.** An amphoteric liposome as claimed in any preceding claim, wherein said liposome comprises a lipid mixture other than one having one of the following specific combinations of amphiphiles:

| Cationic amphiphile | Anionic amphiphile | Other | Ratio (mol.%) |
|---|---|---|---|
| DOTAP | Chems | | 30:40 |
| DOTAP | Chems | POPC | 10:40:50 |
| DOTAP | Chems | POPC | 25:25:50 |
| DOTAP | Chems | POPC | 20:30:50 |
| DOTAP | Chems | POPC | 20:20:60 |
| DOTAP | Chems | POPC | 10:30:60 |
| DOTAP | Chems | POPC | 15:25:60 |
| DOTAP | Chems | POPC: N-glutaryl-DPPE | 10:30:50:10 |
| DOTAP | Chems | DPPC:Chol | 10:30:50:10 |
| DOTAP | Chems | DPPC | 10:30:60 |
| DOTAP | Chems | DPPC | 15:35:50 |
| DOTAP | Chems | POPC:Chol | 10:20:30:40 |
| DOTAP | Chems | DMPC:Chol | 10:30:20:40 |
| DOTAP | Chems | POPC | 15:45:40 |
| DOTAP | Chems | POPC | 20:60:20 |
| DOTAP | Chems | | 25:75 |
| DOTAP | Chems | POPC | 40:40:20 |
| DOTAP | Chems | POPC | 30:50:20 |
| DOTAP | Chems | POPC | 10:70:20 |

(continued)

| Cationic amphiphile | Anionic amphiphile | Other | Ratio (mol.%) |
|---|---|---|---|
| DOTAP | Chems | POPC | 28:47:25 |
| DOTAP | Chems | DOPE | 40:40:20 |
| DOTAP | Chems | DOPE | 30:50:20 |
| DOTAP | Chems | DOPE | 20:60:20 |
| DOTAP | Chems | DOPE | 10:70:20 |
| CHIM | Chems | DPPC | 15:35:50 |
| CHIM | Chems | POPC | 15:35:50 |
| DC-Chol | DOPA | | 66:34 |
| DC-Chol | DOPA | Chol | 40:20:40 |
| DC-Chol | DOPA | DMPC | 27:13:60 |
| DC-Chol | DOPA | DMPC:Chol | 27:13:20:40 |
| DC-Chol | DOPA | DMPC:Chol | 20:10:30:40 |
| DC-Chol | DOPA | DMPC:Chol | 13:7:40:40 |
| HisChol | DG-Succ | DMPC:Chol | 10:10:40:40 |
| MoChol | DG-Succ | DMPC:Chol | 10:15:35:40 |
| MoChol | DG-Succ | DMPC:Chol | 10:10:40:40 |
| MoChol | DG-Succ | DMPC:Chol | 10:30:20:40 |
| MoChol | DG-Succ | DPPC:Chol | 10:30:20:40 |
| MoChol | DG-Succ | POPC:Chol | 10:15:35:40 |
| MoChol | DG-Succ | POPC:Chol | 10:30:20:40 |
| MoChol | DG-Succ | POPC:Chol | 20:10:30:40 |
| CHIM | DMG-Succ | POPC:DOPE | 17:33:12.5:37.5 |
| CHIM | DMG-Succ | POPC:DOPE | 33:17:12.5:37.5 |
| CHIM | DMG-Succ | POPC:DOPE | 23:47:7.5:22.5 |
| CHIM | DMG-Succ | POPC:DOPE | 47:23:7.5:22.5 |
| CHIM | Chems | POPC:DOPE | 17:33:12.5:37.5 |
| CHIM | Chems | POPC:DOPE | 33:17:12.5:37.5 |
| CHIM | Chems | POPC:DOPE | 23:47:7.5:22.5 |
| CHIM | Chems | POPC:DOPE | 47:23:7.5:22.5 |
| MoChol | Cetyl-P | POPC:DOPE | 20:10:10:60 |
| MoChol | Cetyl-P | POPC:Chol | 20:10:35:35 |
| MoChol | DOG-Succ | POPC:DOPE | 17:33:12.5:37.5 |
| MoChol | DOG-Succ | POPC:DOPE | 33:17:12.5:37.5 |
| MoChol | DOG-Succ | POPC:DOPE | 23:47:7.5:22.5 |
| MoChol | DOG-Succ | POPC:DOPE | 47:23:7.5:22.5 |

**24.** A method of loading an amphoteric liposome as claimed in any preceding claim with a negatively charged cargo moiety, said method comprising acidifying said liposome to said first pH with a first solvent comprising anionic counterions, mixing said liposome with said negatively charged cargo moiety and thereafter elevating the pH of said

liposome to said second pH using a second solvent comprising said cationic counterions.

**25.** A method as claimed in claim 24, wherein said acidifying and pH elevating steps are performed by the one-step admixture of the respective solvents, such that said liposome is rapidly brought to the desired respective pH.

**26.** A method as claimed in claim 24 or claim 25, wherein said second pH is about pH 7.4.

**27.** A method as claimed in claim 24 to 26, wherein said first solvent comprises a counter-anion having a molecular volume of > 50 $A^3$ for the encapsulation of said cargo moiety at said first pH.

**28.** A method as claimed in claim 27, wherein said counter-anion is selected from citrate, pyrophosphate, barbituric acid and methyl sulphate.

**29.** A method as claimed in any of claims 24 to 28, wherein said cargo moiety comprises a nucleic acid.

**30.** A method of formulating an amphoteric liposome comprising:

(i) selecting an anionic amphiphile, a cationic amphiphile, each of said anionic and cationic amphiphiles having respective polar head and apolar tail groups, and optionally one or more neutral amphiphiles, at least one of said anionic and cationic amphiphiles being chargeable;
(ii) calculating the $\kappa$ values for each of said anionic and cationic amphiphiles, when uncharged and when charged and associated respectively with predetermined cationic and anionic counterions, and said one or more optional neutral amphiphiles and the $\kappa_{salt}$ value for a lipid salt comprising said anionic and cationic amphiphiles in charged form, $\kappa$ being the ratio of the molecular volume of the polar head group $V_{head}$ to the molecular volume of the apolar tail group $V_{apolar}$ of the respective species, the molecular volumes of the polar head groups of the charged anionic and cationic amphiphiles including the respective counterions, and $\kappa_{salt}$ being defined as:

$$\kappa_{salt} = \frac{V_{head}(cat) + V_{head}(an)}{V_{apolar}(cat) + V_{apolar}(an)}$$

wherein $V_{head}(cat)$ is the molecular volume of the polar head group of the cationic amphiphile without the respective counter-anion, $V_{head}(an)$ is the molecular volume of the polar head group of the anionic amphiphile without the respective counter-cation, $V_{apolar}(cat)$ is the molecular volume of the apolar tail group of the cationic amphiphile and $V_{apolar}(an)$ is the molecular volume of the apolar tail group of the anionic amphiphile ;
(iii) modelling the function $\kappa_{total}$(pH) for a lipid mixture of said anionic and cationic amphiphiles and said one or more optional neutral amphiphiles, assuming said cationic and anionic amphiphiles form said lipid salt when charged, the respective amounts

$$\kappa_{total}(pH) = \kappa_{an} . c_{an}(pH) + \kappa_{cat} . c_{cat}(pH) + \kappa_{an} - .c_{an} - (pH)$$

of said amphiphiles in said lipid mixture being chosen such that said mixture of lipids has an isoelectric point between a first lower pH and a second higher pH and has a stoichiometric excess of positively charged cationic amphiphile at said first pH and a stoichiometric excess of negatively charged anionic amphiphile at said second pH, $\kappa_{total}$(pH) being defined as:
wherein *can (pH)*, $c_{cat}(pH)$, $c_{an-}(pH)$, $c_{cat+}(pH)$ and $c_{salt}(pH)$ are the respective concentrations in the lipid mixture of the uncharged anionic, uncharged cationic, charged anionic and charged cationic amphiphiles and said lipid salt as a function of pH, $c_n$ is the concentration in the lipid mixture of the or each optional neutral amphiphile, and $\kappa_{an}$, $\kappa_{cat}$, $\kappa_{an-}$, $\kappa_{cat+}$, $\kappa_{salt}$ and $\kappa_n$ are the respective $\kappa$ values for the uncharged anionic, uncharged cationic, charged anionic and charged cationic amphiphiles, said lipid salt and the or each optional neutral amphiphile;
(iv) determining that $\kappa_{total}$(pH) exhibits a minimum at said isoelectric point;
(v) making liposomes composed of said lipid mixture and empirically confirming that said mixture exhibits stable

lamellar phases at said first and second pH's and a fusogenic, hexagonal phase at said isoelectric point; and thereafter
(vi) manufacturing an amphoteric liposome composed of said lipid mixture.

**31.** A method as claimed in claim 30, wherein said molecular volumes are calculated by molecular modelling.

**32.** A method as claimed in claim 30 or claim 31, wherein said anionic and cationic amphiphiles and their respective amounts are selected such that said lipid mixture exhibits an isoelectric point in the range pH 4 to pH 8.

**33.** A method as claimed in claim 30, claim 31 or claim 32, wherein said anionic and cationic amphiphiles are selected such that said lipid salt has a $\kappa_{salt}$ value of less than about 0.35.

**34.** A method as claimed in any of claims 30 to 33, wherein said anionic and cationic amphiphiles are selected such that $V_{head}(cat) + V_{head}(an)$ is less than or equal to about 300 Å$^3$.

**35.** A method as claimed in any of claims 30 to 34, wherein said anionic and cationic amphiphiles are selected such that the combined molecular volumes of the cationic and anionic amphiphiles is less than about 1000 Å$^3$.

**36.** A method as claimed in any of claims 30 to 35, wherein said counter-cations have a molecular volume of at least 50 A$^3$.

**37.** A method as claimed in claim36, wherein said counter-cations are selected from are selected from sodium or tris (hydroxymethyl)aminomethane, tris-hydroxyethylaminomethane and triethylamine.

**38.** A method as claimed in claimed in claim 37, wherein said counter-cations are sodium.

**39.** A method as claimed in any of claims 30 to 38, wherein said anionic amphiphile is chargeable and is present in molar excess of said cationic amphiphile, said counterions are sodium and chloride or phosphate, and:

(i) $V_{head}(cat) + V_{head}(an)$ is about 160+/-80 A$^3$;
(ii) $V_{apolar}(cat) + V_{apolar}(an)$ is about 700+/-150 A$^3$; and
(iii) $\kappa_{salt} < 0.3$, and the difference between $\kappa_{salt}$ and $\kappa_{total}$(pH) at pH 8 > 0.12.

**40.** A method as claimed in any of claims 30 to 38, wherein said cationic amphiphile is weak and is present in molar excess of said anionic amphiphile, said counterions are sodium and chloride or phosphate, and:

(i) $V_{head}(cat) + V_{head}(an)$ is about 160+/-80 A$^3$;
(ii) $V_{apolar}(cat) + V_{apolar}(an)$ is about 700+/-150 A$^3$; and
(iii) $\kappa_{salt} < 0.3$, and the difference between $\kappa_{salt}$ and $\kappa_{total}$(pH) at pH 8 > 0.12.

**41.** A method as claimed in any of claims 30 to 38, wherein said cationic and anionic amphiphiles are both weak and are present in substantially equal molar amounts to one another, the counterions are sodium and chloride or phosphate, and:

(i) $V_{head}(cat) + V_{head}(an)$ is about 160+/-80 A$^3$;
(ii) $V_{apolar}(cat) + V_{apolar}(an)$ is about 700+/-150 A$^3$; and
(iii) $\kappa_{salt} < 0.3$ and the difference between $\kappa_{salt}$ and $\kappa_{total}$(pH) at pH 8 > 0.12.

**42.** A method as claimed in any of claims 30 to 41, wherein said selected cationic and anionic amphiphiles each carry a single charge.

**43.** A method as claimed in any of claims 30 to 42, wherein said mixture comprises up to about 65 mol.% of one or more neutral amphiphiles.

**44.** A method as claimed in claim 43, wherein said one or more neutral amphiphiles comprise a neutral amphiphile having a $\kappa_n$ value greater than or equal to about 0.4.

**45.** A method as claimed in claim 43 or claim 44, wherein said one or more neutral amphiphiles comprise a neutral amphiphile having a $\kappa_n$ value less than or equal to about 0.3.

**46.** A method as claimed in claim 43, claim 44 or claim 45, wherein said anionic amphiphile is weak and is present in molar excess of said cationic amphiphile, the counterions are sodium and chloride or phosphate, and:

(i) $V_{head}(cat) + V_{head}(an)$ is about 220+/-80 $A^3$;
(ii) $V_{apolar}(cat) + V_{apolar}(an)$ is about 700+/-150 $A^3$; and
(iii) $\kappa_{salt}$ is in the range 025 to 0.45, and the difference between $V_{head}(cat) + V_{head}(an)$ and $\kappa_{total}$(pH) at pH 8 > 0.16.

**47.** A method as claimed in claim 43, claim 44 or claim 45, wherein said cationic amphiphile is weak and is present in molar excess of said anionic amphiphile, the counterions are sodium and chloride or phosphate, and:

(i) $V_{head}(cat) + V_{head}(an)$ is about 220+/-80 $A^3$;
(ii) $V_{apolar}(cat) + V_{apolar}(an)$ is about 700+/-150 $A^3$; and
(iii) $\kappa_{salt}$ is in the range 0.25 to 0.45, and the difference between $\kappa_{salt}$ and $\kappa_{total}$(pH) at pH 8 > 0.16.

**48.** A method as claimed in claim 43, claim 44 or claim 45, wherein said cationic and anionic amphiphiles are both weak and are present in substantially equal molar amounts to one another, the counterions are sodium and chloride or phosphate and:

(i) $V_{head}(cat) + V_{head}(an)$ is about 210+/-80 $A^3$;
(ii) $V_{apolar}(cat) + V_{apolar}(an)$ is about 700+/-150 $A^3$; and
(iii) $\kappa_{salt}$ is in the range 0.25 to 0.45, and the difference between $\kappa_{salt}$ and $\kappa_{total}$(pH) at pH 8 > 0.16.

**49.** An amphoteric liposome formulated in accordance with any of claims 30 to 48.

**50.** A method of predicting the fusogenicity of an amphoteric liposome at a given pH, said liposome being composed of a lipid mixture comprising an anionic amphiphile, a cationic amphiphile, each of said anionic and cationic amphiphiles having respective polar head and apolar tail groups, and optionally one or more neutral amphiphiles, at least one of said anionic and cationic amphiphiles being chargeable; said method comprising:

calculating the $\kappa$ values for each of said anionic and cationic amphiphiles, when uncharged and when charged and associated respectively with predetermined cationic and anionic counterions for said anionic and cationic amphiphiles respectively, and said one or more optional neutral amphiphiles and the $\kappa_{salt}$ value for a lipid salt comprising said anionic and cationic amphiphiles in charged form, $\kappa$ being the ratio of the molecular volume of the polar head group $V_{head}$ to the molecular volume of the apolar tail group $V_{apolar}$ of the respective species, the molecular volumes of the polar head groups of the charged anionic and cationic amphiphiles including the respective counterions, $\kappa_{salt}$ being defined as:

$$\kappa_{salt} = \frac{V_{head}(cat) + V_{head}(an)}{V_{apolar}(cat) + V_{apolar}(an)}$$

wherein $V_{head}(cat)$ is the molecular volume of the polar head group of the cationic amphiphile without the respective counter-anion, $V_{head}(an)$ is the molecular volume of the polar head group of the anionic amphiphile without the respective counter-cation, $V_{apolar}(cat)$ is the molecular volume of the apolar tail group of the cationic amphiphile and $V_{apolar}(an)$ is the molecular volume of the apolar tail group of the anionic amphiphile; and
modelling the function $\kappa_{total}$(pH) for a lipid mixture of said anionic and cationic amphiphiles and said one or more optional neutral amphiphiles, assuming said cationic and anionic amphiphiles form said lipid salt when charged, $\kappa_{total}$(pH) being defined as:

$$\kappa_{total}(pH) = \kappa_{an} \cdot c_{an}(pH) + \kappa_{cat} \cdot c_{cat}(pH) + \kappa_{an^-} \cdot c_{an^-}(pH) + \kappa_{cat^+} \cdot c_{cat^+}(pH) + \kappa_{salt} \cdot c_{salt}(pH) + \sum \kappa_n \cdot c_n$$

wherein $c_{an}(pH)$, $c_{cat}(pH)$, $c_{an-}(pH)$, $c_{cat+}(PH)$ and $c_{salt}(PH)$ are the respective concentrations in the lipid mixture of

the uncharged anionic, uncharged cationic, charged anionic and charged cationic amphiphiles and said lipid salt as a function of pH, $c_n$ is the concentration in the lipid mixture of the or each optional neutral amphiphile, and $\kappa_{an}$, $\kappa_{cat}$, $\kappa_{an-}$, $\kappa_{cat+}$, $\kappa_{salt}$ and $\kappa_n$ are the respective $\kappa$ values for the uncharged anionic, uncharged cationic, charged anionic and charged cationic amphiphiles, said lipid salt and the or each optional neutral amphiphile, $\kappa_{total}$(pH) being an indicator of the fusogenicity of said liposome.

**51.** A method as claimed in claim 50, wherein said molecular volumes are calculated by molecular modelling.

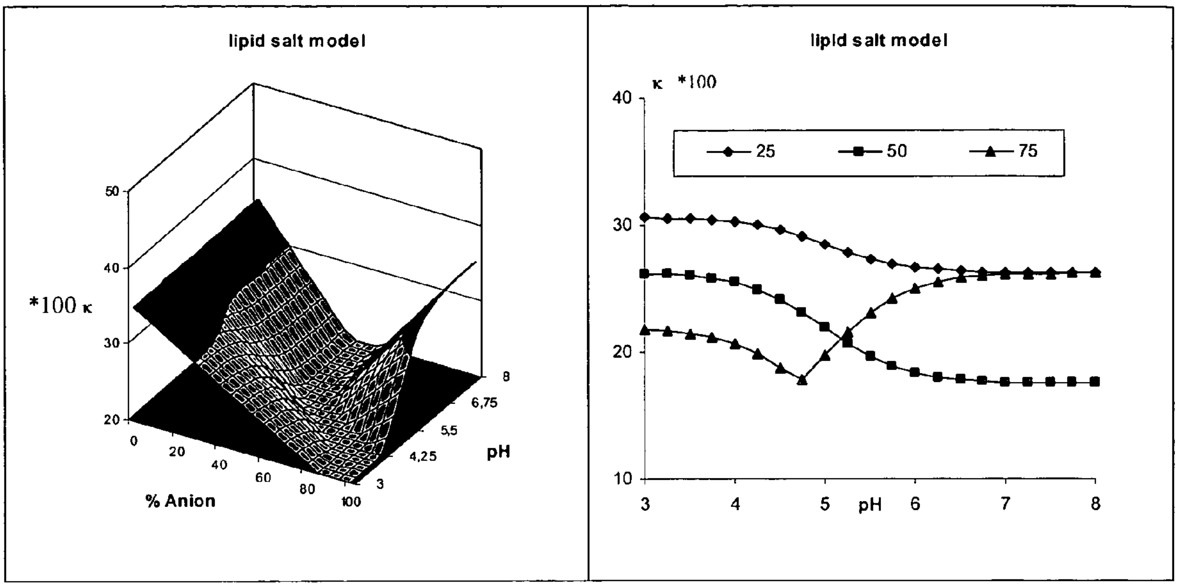
lipid salt model
50
40
*100 κ
30
20
0
20
40
60
80
100
% Anion
3
4,25
5,5
6,75
8
pH
lipid salt model
κ *100
40
30
20
10
25
50
75
3
4
5
pH
6
7
8

Fig. 1

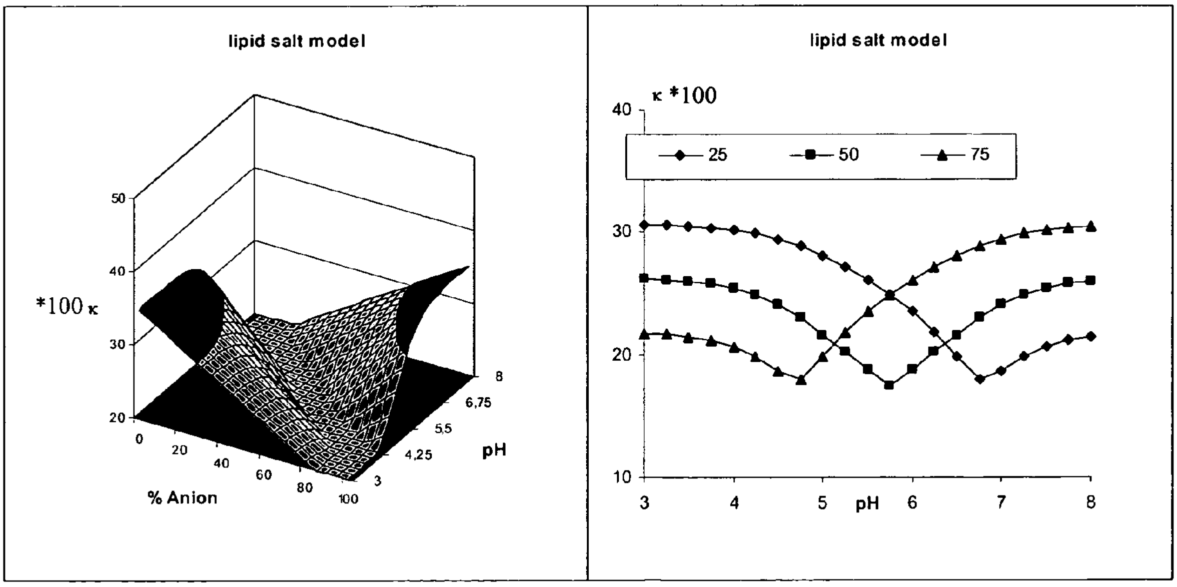
lipid salt model
50
40
*100 κ
30
20
0
20
40
60
80
100
% Anion
3
4,25
5,5
6,75
8
pH
lipid salt model
κ *100
40
30
20
10
25
50
75
3
4
5
pH
6
7
8

Fig. 2

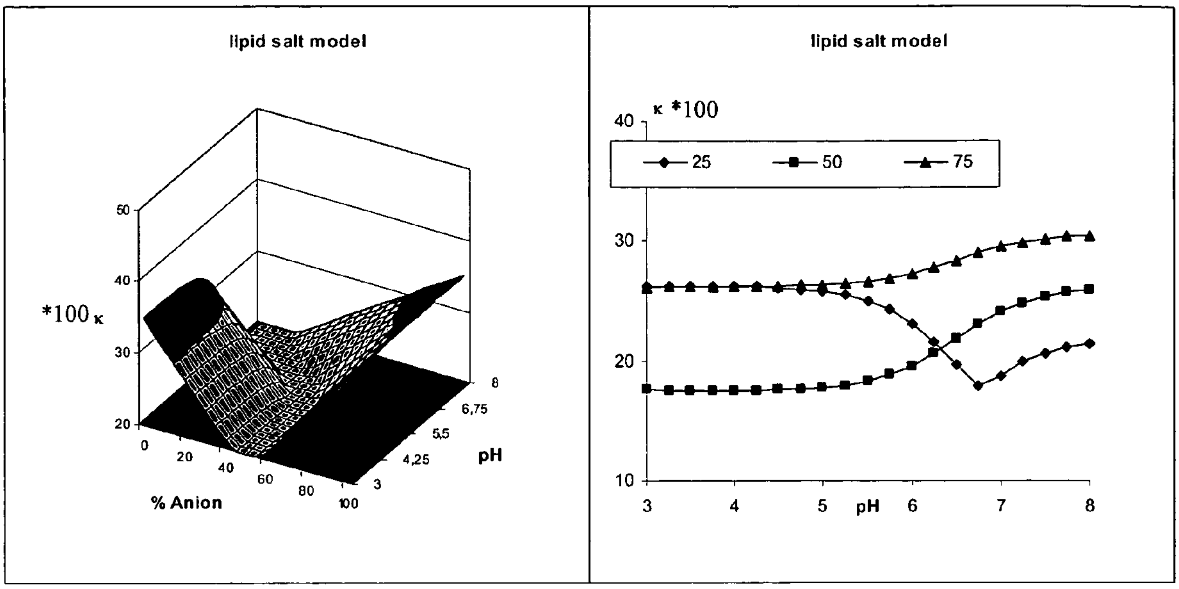
lipid salt model
*100 κ
50
40
30
20
0
20
40
60
80
100
% Anion
3
4,25
5,5
6,75
8
pH
lipid salt model
κ *100
40
30
20
10
25
50
75
3
4
5
pH
6
7
8

Fig. 3

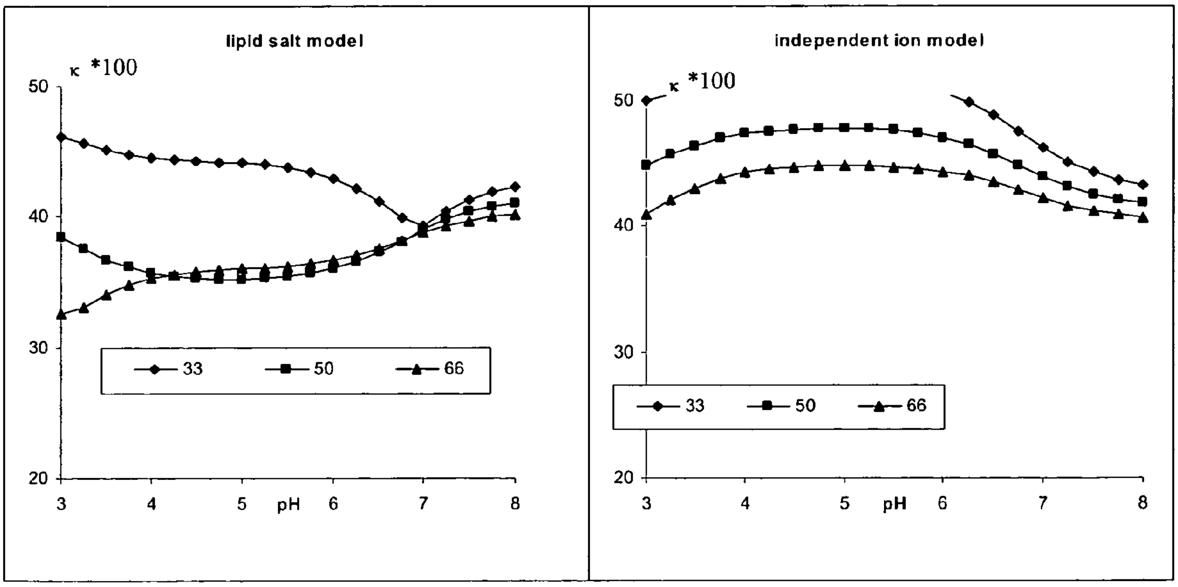
lipid salt model
κ *100
50
40
30
20
33
50
66
3
4
5
pH
6
7
8
independent ion model
κ *100
50
40
30
20
33
50
66
3
4
5
pH
6
7
8

Fig. 4

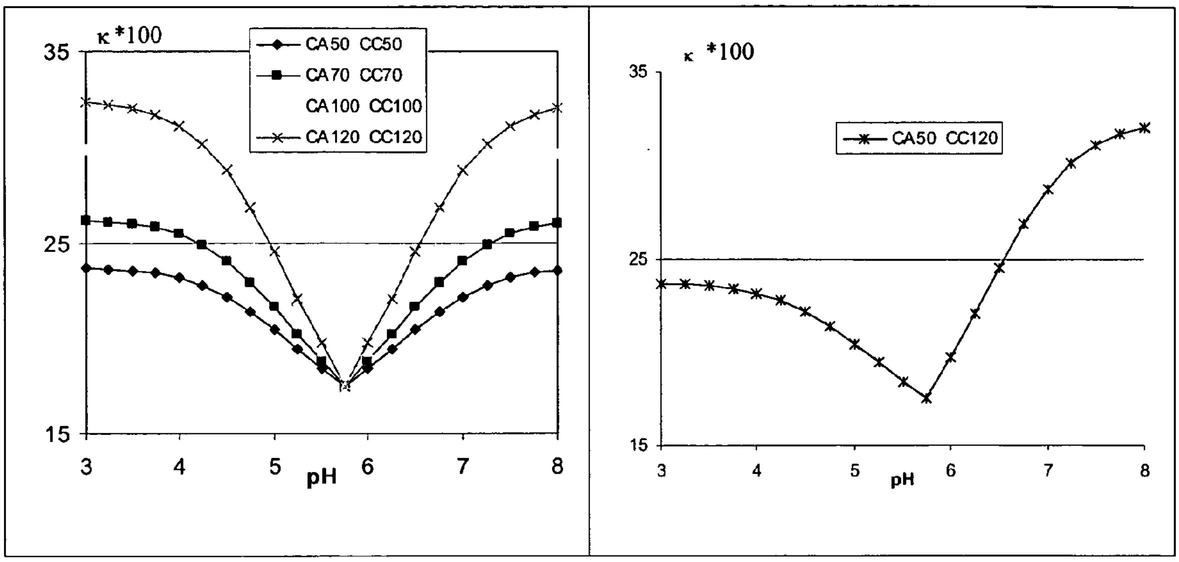
κ *100
35
25
15
3
4
5
pH
6
7
8
CA50 CC50
CA70 CC70
CA100 CC100
CA120 CC120
κ *100
35
25
15
CA50 CC120
3
4
5
pH
6
7
8

Fig. 5

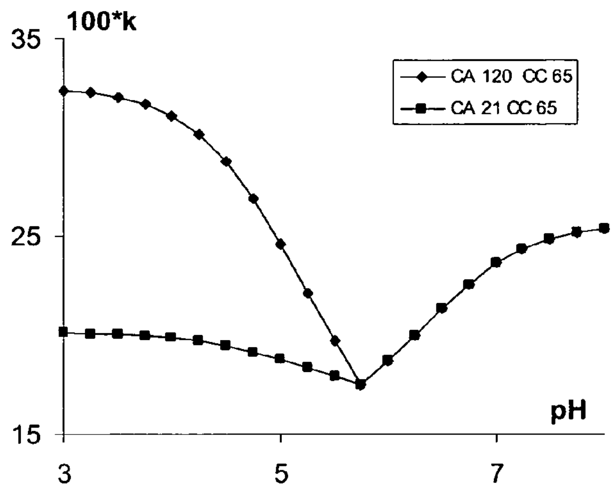
100*k
35
25
15
CA 120 CC 65
CA 21 CC 65
pH
3
5
7

Fig. 6

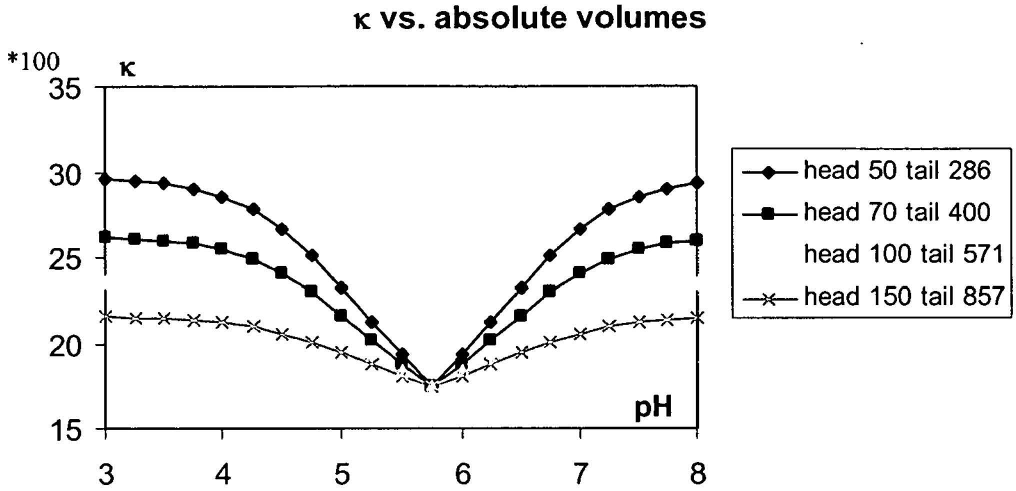
κ vs. absolute volumes
*100
κ
35
30
25
20
15
3
4
5
6
7
8
pH
head 50 tail 286
head 70 tail 400
head 100 tail 571
head 150 tail 857

Fig. 7

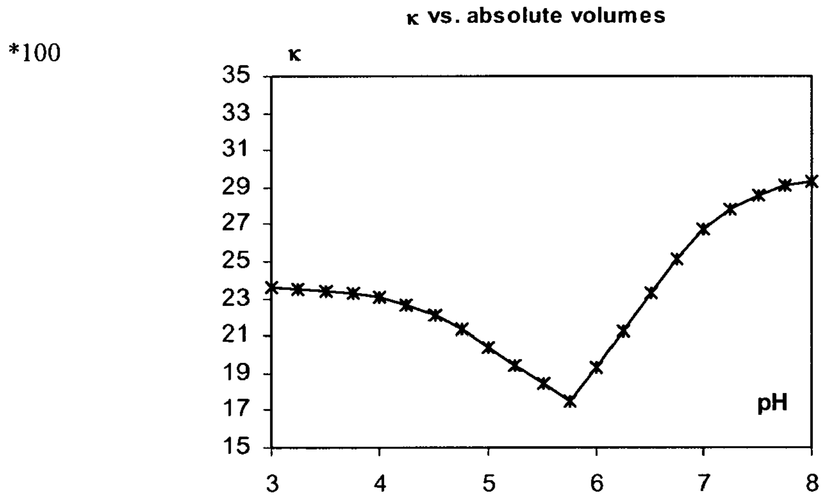
κ vs. absolute volumes
*100
κ
35
33
31
29
27
25
23
21
19
17
15
3
4
5
6
7
8
pH

Fig. 8

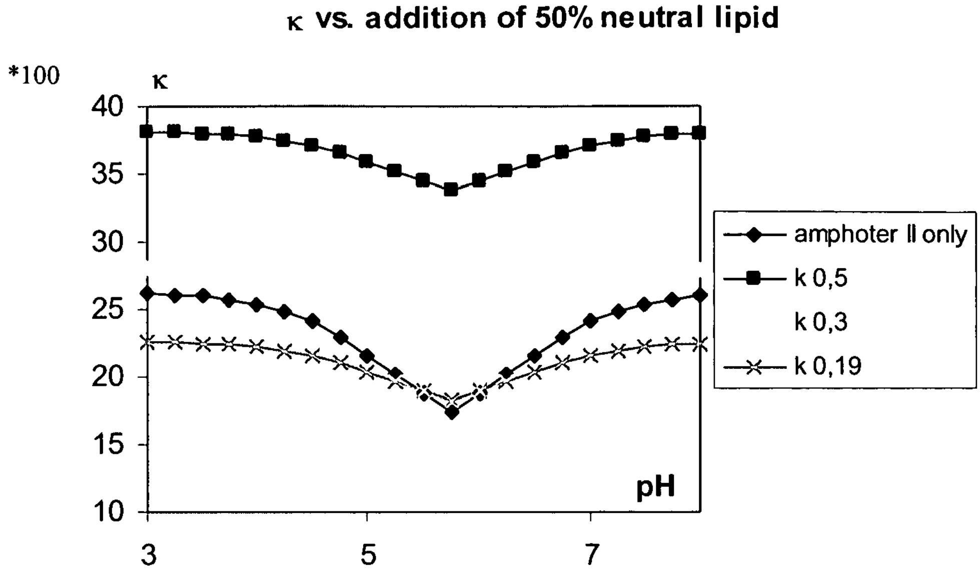
κ vs. addition of 50% neutral lipid
*100
κ
40
35
30
25
20
15
10
3
5
7
pH
amphoter II only
k 0,5
k 0,3
k 0,19

Fig. 9

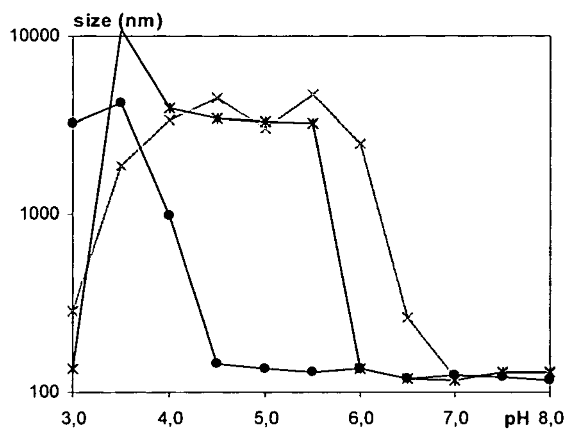
size (nm)
10000
1000
100
3,0
4,0
5,0
6,0
7,0
pH 8,0

Fig. 10

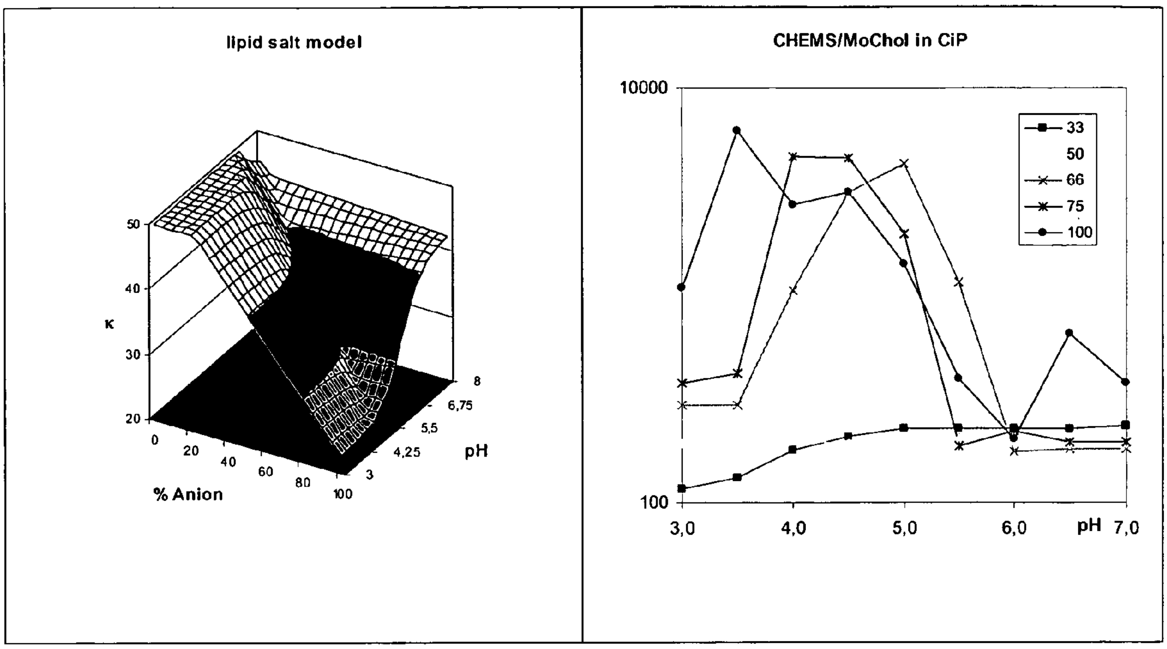
lipid salt model
κ
50
40
30
20
0
20
40
60
80
100
% Anion
3
4,25
5,5
6,75
8
pH
CHEMS/MoChol in CiP
10000
100
3,0
4,0
5,0
6,0
pH
7,0
33
50
66
75
100

Fig. 11

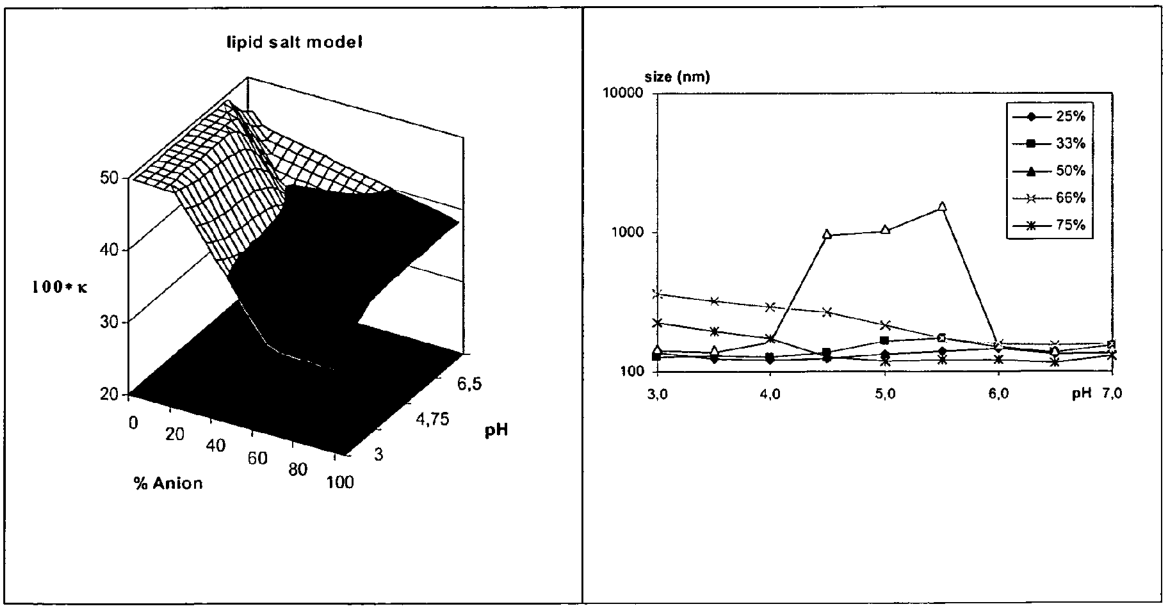
lipid salt model
100*κ
50
40
30
20
0
20
40
60
80
100
% Anion
3
4,75
6,5
pH
size (nm)
10000
1000
100
3,0
4,0
5,0
6,0
pH
7,0
25%
33%
50%
66%
75%

Fig. 12

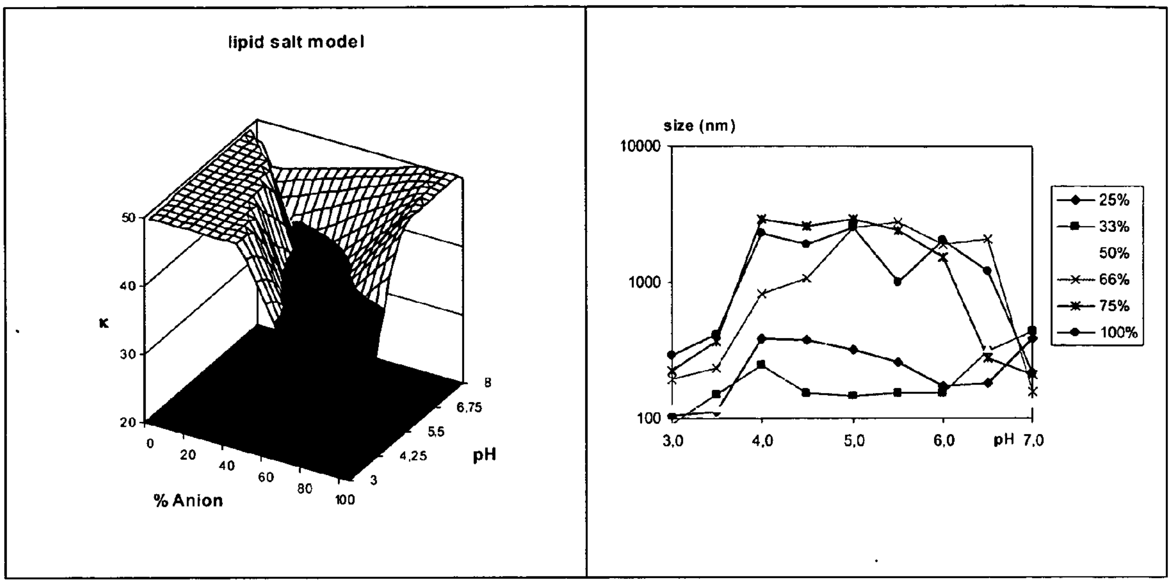
lipid salt model
50
40
30
20
κ
0
20
40
60
80
100
% Anion
3
4,25
5,5
6,75
8
pH
size (nm)
10000
1000
100
3,0
4,0
5,0
6,0
pH 7,0
25%
33%
50%
66%
75%
100%

Fig. 13

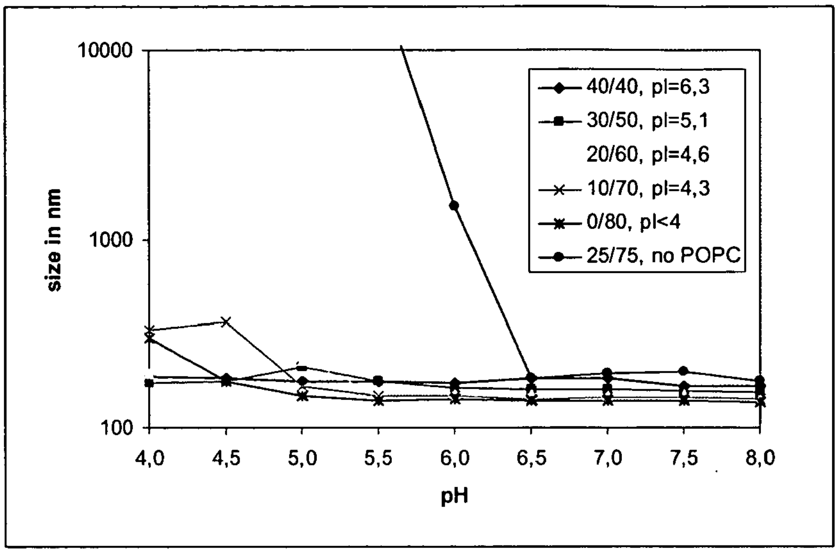
10000
1000
100
size in nm
4,0
4,5
5,0
5,5
6,0
6,5
7,0
7,5
8,0
pH
40/40, pI=6,3
30/50, pI=5,1
20/60, pI=4,6
10/70, pI=4,3
0/80, pI<4
25/75, no POPC

Fig. 14

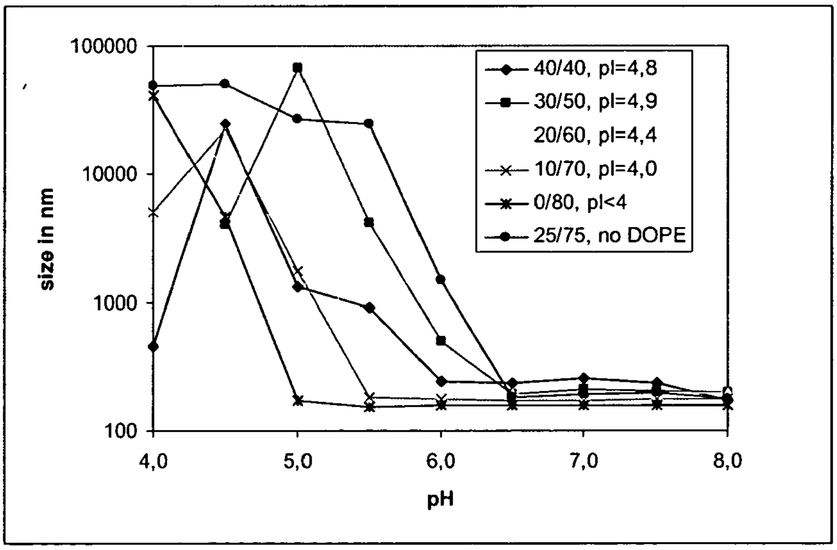
100000
10000
1000
100
size in nm
4,0
5,0
6,0
7,0
8,0
pH
40/40, pI=4,8
30/50, pI=4,9
20/60, pI=4,4
10/70, pI=4,0
0/80, pI<4
25/75, no DOPE

Fig. 15

European Patent Office

# PARTIAL EUROPEAN SEARCH REPORT

which under Rule 45 of the European Patent Convention shall be considered, for the purposes of subsequent proceedings, as the European search report

Application Number
EP 06 25 5277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 02/066012 A (NOVOSOM AG [DE]; PANZNER STEFFEN [DE]; FANKHAENEL STEFAN [DE]; ESSLER) 29 August 2002 (2002-08-29)<br>* examples * | 1-49 | INV.<br>A61K9/127 |
| X | WO 2006/047792 A (HEWLETT PACKARD DEVELOPMENT CO [US]; SHEN BO [US]; TROTT MITCHELL [US]) 4 May 2006 (2006-05-04)<br>* examples 6,7 * | 1-49 | |
| X | WO 2006/048329 A (NOVOSOM AG [DE]; PANZNER STEFFEN [DE]; RAUCHHAUS UNA [DE]; ENDERT GERO) 11 May 2006 (2006-05-11)<br>* examples 1,7 * | 1-49 | |
| X | WO 2006/053646 A (NOVOSOM AG [DE]; PANZNER STEFFEN [DE]; PANZNER CORNELIA [DE]; LUTZ SIL) 26 May 2006 (2006-05-26)<br>* page 9, line 8 - page 10, line 17 *<br>* example 1 * | 1-49 | |
| X | WO 02/066489 A (NOVOSOM AG [DE]; PANZNER STEFFEN [DE]; ENDERT GEROLD [DE]; FANKHAENEL) 29 August 2002 (2002-08-29)<br>* examples 2-4 * | 1-49 | TECHNICAL FIELDS SEARCHED (IPC)<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| The Hague | 14 March 2007 | Boulois, Denis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 06 25 5277

Claim(s) searched completely:
1-49

Claim(s) not searched:
50,51

Reason for the limitation of the search (non-patentable invention(s)):

The subject-matter of claims 50, 51 relate to a method excluded by patentability under Article 52(2) EPC, namely method for performing mental acts and mathematical methods. The subject-matter of these claims was not searched for this reason.

## ANNEX TO THE EUROPEAN SEARCH REPORT ON EUROPEAN PATENT APPLICATION NO. EP 06 25 5277

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2007

| Patent document cited in search report | | Publication date | Patent family member(s) | | | Publication date |
|---|---|---|---|---|---|---|
| WO 02066012 | A | 29-08-2002 | BR | 0207775 | A | 30-03-2004 |
| | | | CA | 2438116 | A1 | 29-08-2002 |
| | | | CN | 1492756 | A | 28-04-2004 |
| | | | DE | 10109897 | A1 | 07-11-2002 |
| | | | EP | 1363601 | A2 | 26-11-2003 |
| | | | JP | 2004525898 | T | 26-08-2004 |
| | | | US | 2003099697 | A1 | 29-05-2003 |
| WO 2006047792 | A | 04-05-2006 | US | 2006088105 | A1 | 27-04-2006 |
| WO 2006048329 | A | 11-05-2006 | NONE | | | |
| WO 2006053646 | A | 26-05-2006 | NONE | | | |
| WO 02066489 | A | 29-08-2002 | BR | 0207776 | A | 23-03-2004 |
| | | | CA | 2438910 | A1 | 29-08-2002 |
| | | | CN | 1492876 | A | 28-04-2004 |
| | | | DE | 10109898 | A1 | 05-09-2002 |
| | | | WO | 02066490 | A2 | 29-08-2002 |
| | | | EP | 1363933 | A2 | 26-11-2003 |
| | | | EP | 1363932 | A2 | 26-11-2003 |
| | | | JP | 2004521917 | T | 22-07-2004 |
| | | | JP | 2004525902 | T | 26-08-2004 |
| | | | US | 2004131666 | A1 | 08-07-2004 |
| | | | US | 2004120997 | A1 | 24-06-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02066012 A, Panzner
- WO 9821322 A
- DE 19753182
- EP 1392341 A
- WO 9932619 A
- WO 02055693 A

### Non-patent literature cited in the description


- **STRAUBINGER et al.** *FEBS Lett,* 1985, vol. 179 (1), 148-154
- **HAFEZ ; CULLIS.** *Biochim. Biophys. Acta,* 2000, vol. 1463, 107-114
- **HAFEZ et al.** *Biophys. J.,* 2000, vol. 79 (3), 1438-1446
- **LI ; SCHICK.** *Biophys. J,* 2001, vol. 80, 1703-1711
- **ISRAELACHVILI ; MITCHELL.** *Biochim. Biophys. Acta,* 1975, vol. 389, 13-19
- **ISRAELACHVILI et al.** *Biochim Biophys Acta,* 1977, vol. 470 (2), 185-201
- **ISRAELACHVILI et al.** *Q. Rev. Biophys,* 1980, vol. 13 (2), 121-200
- **CHO-CHUNG et al.** *Curr. Opin. Mol. Ther,* 1999